# EUROPEAN PATENT APPLICATION

(11) **EP 1 380 645 A2**
(43) Date of publication of application: **14.01.2004**
(21) Application number: 03077231.3
(22) Date of filing: 10.05.1990
(51) Int. Cl.: C12N 15/12, C07K 14/805, G01N 33/72, C12P 21/02

(54) **Production in bacteria ad yeast of hemoglobin and analogues thereof**

(30) Priority: 10.05.1989 US 349623; 30.06.1989 US 374161; 13.07.1989 US 379116
(62) Divisional of application: 95110064.3
(71) Applicant: Baxter Biotech Technology S.à.r.l., 2000 Neuchâtel (CH)
(72) Inventor: Hoffmann, Stephen J., Denver, Colorado 80207 (US); Looker, Douglas L., Aurora, Colorado 80015 (US); Rosendal, Mary S., Broomfield, Colorado 80020 (US); Stetler, Gary L., Denver, Colorado 80302 (US); Wagenbach, Michael, Boulder, Colorado 80302 (US)
(74) Representative: Bassett, Richard Simon

(57) **Abstract**

A method of producing a hemoglobin-like protein wherein an alpha globin-like polypeptide and a beta globin-like polypeptide are each expressed in transformed non-erythrocyte cells such as bacterial or yeast cells, the method comprising expressing the alpha and beta globin-like polypeptide in the same cell in such manner that the alpha and beta globin-like polypeptides are assembled and combined with heme so as to intracellularly produce a biologically functional hemoglobin-like protein in soluble, recoverable form.

## Description

This application is a continuation-in-part of Looker and Hoffman, U.S. Ser. No. 07/374,161, DI-ALPHA AND DI-BETA GLOBIN LIKE POLYPEPTIDES AND USES THEREFOR, filed June 30, 1989; Stetler and Wagenbach, U.S. Ser. No. 07/379,116, PRODUCTION OF HUMAN HEMOGLOBIN BY TRANSFORMED YEAST CELLS, filed July 13, 1989; and Hoffman, Looker, Rosendahl and Stetler, U.S. Ser. No. 07/349,623, POLYCISTRONIC CO-EXPRESSION OF THE ALPHA- AND BETA-GLOBINS AND IN VIVO ASSEMBLY OF BIOLOGICALLY ACTIVE, TETRAMERIC HEMOGLOBIN, filed May 10, 1989; all owned by Somatogenetics International, Inc.

### Cross-Reference to Related Applications

Hoffman and Nagai, U.S. Ser. No. 07/194,338, filed May 10, 1988, presently owned by Somatogenetics International, Inc., relates to the use of low oxygen affinity mutant hemoglobins as blood substitutes, and to the expression of alpha and beta globin in nonerythroid cells.

### Field of the Invention

The present invention relates to the intracellular assembly of a hemoglobin-like protein in biologically functional, substantially soluble form through co-expression of alpha- and beta-globin-like polypeptides in bacterial or yeast cells.

It further relates to the genetic cross-linking of the two alpha subunits of hemoglobin to form a novel polypeptide, di-alpha globin, which may be considered a partially assembled intermediate leading to a hemoglobin-like protein, and the use of this compound in the production of synthetic hemoglobins having an increased intravascular half-life as compared to stroma-free hemoglobins. It also relates to the analogous polypeptide di-beta globin.

### Information Disclosure Statement

It is not always practical to transfuse a patient with donated blood. In these situations, use of a red blood cell substitute is desirable. The product must effectively transport O₂, just as do red blood cells. ("Plasma expanders", such as dextran and albumin, do not transport oxygen.) The two types of substitutes that have been studied most extensively are hemoglobin solutions and fluorocarbon emulsions.

### A. Structure and Function of Hemoglobin

Hemoglobin (Hgb) is the oxygen-carrying component of blood. Hemoglobin circulates through the bloodstream inside small enucleate cells called erythrocytes (red blood cells). Hemoglobin is a protein constructed from four associated polypeptide chains, and bearing prosthetic groups known as hemes. The erythrocyte helps maintain hemoglobin in its reduced, functional form. The heme iron atom is susceptible to oxidation, but may be reduced again by one of two enzyme systems within the erythrocyte, the cytochrome b₅ and glutathione reduction systems.

About 92% of the normal adult human hemolysate is Hgb A (designated alpha2 beta2, because it comprises two alpha and two beta chains). The alpha chain consists of 141 amino acids. The iron atom of the heme (ferroprotoporphyrin IX) group is bound covalently to the imidazole of His 87 (the "proximal histidine"). The beta chain is 146 residues long and heme is bound to it at His 92. Apohemoglobin is the heme-free analogue of hemoglobin; it exists predominantly as the αβ-globin dimer.

Separated, heme-free, alpha-and beta-globins have been prepared from the heme-containing alpha and beta subunits of hemoglobin. The separated heme-free globin chains are folded very differently, even though the heme-containing subunits are highly similar in secondary structure and basic folding features. This shows that the binding of the prosthetic heme group to globin subunits has quite different effects on alpha and beta globin. Yip, et al., J. Biol. Chem., 247: 7237-44 (1972).

Native human hemoglobin has been fully reconstituted from separated heme-free alpha and beta globin and from hemin. Preferably, heme is first added to the alpha-globin subunit. The heme-bound alpha globin is then complexed to the heme-free beta subunit. Finally, heme is added to the half-filled globin dimer, and tetrameric hemoglobin is obtained. Yip, et al., PNAS (USA), 74: 64-68 (1977).

In cell-free systems prepared from unfractionated rabbit reticulocyte hemolysates, globin is actively synthesized for approximately five minutes, and then protein synthesis abruptly ceases. Prior addition of hemin prevents or delays the cessation of synthetic activity, as a result of the effect of hemin on an inhibitory protein known as "hemin-regulated inhibitor" (HRI). Hemin deficiency has a more severe effect on alpha chain synthesis than on beta chain synthesis as alpha globin mRNA is less efficient than beta-globin mRNA in initiating polypeptide chain synthesis. It has been speculated that alpha chains are released from their site of synthesis only in the presence of free beta chains, which immediately complex the released alpha chains to form αβ-globin dimers. These then combine with heme to form tetrameric hemoglobin. Winterhalter and Huehns, J. Biol. Chem., 239: 3699 (1964). It is certainly known that the addition of heme to αβ-globin dimers (apohemoglobin) leads to the rapid formation of hemoglobin.

The human alpha and beta globin genes reside on chromosomes 16 and 11, respectively. Bunn and Forget, supra at 172. Both genes have been cloned and sequenced, Liebhaber, et al., PNAS 77: 7054-58 (1980) (alpha-globin genomic DNA); Marotta, et al., J. Biol. Chem., 252: 5040-53 (1977) (beta globin cDNA); Lawn, et al., Cell, 21:647 (1980) (beta globin genomic DNA).

Hemoglobin exhibits cooperative binding of oxygen by the four subunits of the hemoglobin molecule (two alpha-globins and two beta-globins in the case of Hgb A), and this cooperativity greatly facilitates efficient oxygen transport. Cooperativity, achieved by the so-called heme-heme interaction, allows hemoglobin to vary its affinity for oxygen. Hemoglobin reversibly binds up to four moles of oxygen per mole of Hgb.

oxygen-carrying compounds are frequently compared by means of a device known as an oxygen dissociation curve. This curve is obtained when, for a given oxygen carrier, oxygen saturation or content is graphed against the partial pressure of oxygen. For Hgb, the percentage of saturation increases with partial pressure according to a sigmoid relationship. The P₅₀ is the partial pressure at which the oxygen-carrying solution is half saturated with oxygen. It is thus a measure of oxygen-binding affinity; the higher the P₅₀, the more loosely the oxygen is held.

When the oxygen dissociation curve of an oxygen-carrying solution is such that the P₅₀ is less than that for whole blood, it is said to be "left-shifted."

The oxygen affinity of hemoglobin is lowered by the presence of 2,3-diphosphoglycerate (2,3-DPG), chloride ions and hydrogen ions. Respiring tissue releases carbon dioxide into the blood and lowers its pH (i.e. increases the hydrogen ion concentration), thereby causing oxygen to dissociate from hemoglobin and allowing it to diffuse into individual cells.

The ability of hemoglobin to alter its oxygen affinity, increasing the efficiency of oxygen transport around the body, is dependent on the presence of the metabolite 2,3-DPG. Inside the erythrocyte 2,3-DPG is present at a concentration nearly as great as that of hemoglobin itself. In the absence of 2,3-DPG "conventional" hemoglobin binds oxygen very tightly and would release little oxygen to respiring tissue.

Aging erythrocytes release small amounts of free hemoglobin into the blood plasma where it is rapidly bound by the scavenging protein haptoglobin. The hemoglobin-haptoglobin complex is removed from the blood and degraded by the spleen and liver.

### B. Blood Substitutes, Generally

It is clear from the above considerations that free native hemoglobin A, injected directly into the bloodstream, would not support efficient oxygen transport about the body. The essential allosteric regulator 2,3-DPG is not present in sufficient concentration in the plasma to allow hemoglobin to release much oxygen at venous oxygen tension.

Nonetheless, solutions of conventional hemoglobin have been used as RBC substitutes. The classic method of preparing hemoglobin solutions employs outdated blood. The red cells are lysed and cellular debris is removed, leaving what is hopefully "stromal-free hemoglobin" (SFH).

Several basic problems have been observed with this approach. The solution must be freed of any toxic components of the red cell membrane without resorting to cumbersome and tedious procedures which would discourage large-scale production. DeVenuto, "Appraisal of Hemoglobin Solution as a Blood Substitute", Surgery, Gynecology and Obstetrics, 149: 417-436 (1979).

Second, as expected, such solutions are "left-shifted" (lower P₅₀) as compared to whole blood. Gould, et al., "The Development of Polymerized Pyridoxylated Hemoglobin Solution as a Red Cell Substitute", Ann. Emerg. Med. 15: 1416-1419 (Dec. 3, 1986).

Third, SFH has a half-life in the circulatory system of only about 2-4 hours. This is because oxy Hgb partially dissociates into a dimer (αβ) that is small enough to be filtered by the kidney.

Finally, SFH has a high colloid osmotic pressure (COD). Thus, administration of SFH in a dose that would have the same oxygen-carrying capacity as a unit of packed red blood cells is inadvisable, since the high osmotic pressure (60mm Hg) would cause a massive influx of water from the cells into the bloodstream, thus dehydrating the patient's tissues. This consideration limits the dose of SFH to that which provide a final concentration of about 6-8 gm Hgb/dl.

In an effort to restore the desired P₅₀, researchers added 2,3-DPG to the hemoglobin solution. Unfortunately, 2,3-DPG was rapidly eliminated from the circulation. Scientists then turned to other organic phosphates, particularly pyridoxal phosphate. Like 2,3-DPG, these compounds stabilized the "T state" of the Hgb by forming a salt bridge between the N-termini of the two beta chains. The pyridoxylated hemoglobin had a P₅₀ of 20-22 torr, as compared to 10 torr for SFH and 28 torr for whole blood. While this is an improvement over SFH, the pyridoxylated Hgb remains "high affinity" relative to whole blood.

### C. Chemical Crosslinking of Hemoglobin Subunits

The properties of hemoglobin have been altered by specifically chemically crosslinking the alpha chains between the Lys99 of alphal and the Lys99 of alpha2. Walder, U.S. 4,600,531 and 4,598,064; Snyder, et al., PNAS (USA) 84: 7280-84 (1987); Chaterjee, et al., J. Biol. Chem., 261: 9927-37 (1986). The P₅₀ was 29 mm Hg, and renal excretion was abrogated by the crosslinking, but the plasma half-life was increased just 2-3 fold.

This chemical crosslinking was accomplished by reacting bis(3,5-dibromosalicyl) fumarate with deoxyhemoglobin A in the presence of inositol hexaphosphate. This reaction has a low yield (10-20%). Moreover, purification is required to eliminate derivatives modified at other sites (there are 42 other lysine residues and the amino terminal amino groups of the four chains at which competing reactions could occur).

A further problem with the use of a "diaspirin" crosslinking agent is that it can participate in a side reaction yielding a carcinogenic halophenol.

In the hemoglobin analogue of the present invention, the N-terminal valine and C-terminal arginine of the alpha globins are connected by means of an amino acid or peptide linker, without resort of special coupling agents.

The beta chains have also been chemically crosslinked. Kavanaugh, et al., Biochemistry, 27: 1804-8( 1988). Kavanaugh notes that the beta N-termini are 16 Å apart in the T state and 20 Å apart in the R state. Not surprisingly, the introduction of a DIDS bridge between the N-termini of T state hemoglobin hindered the shift to the R state, thereby decreasing the O₂ affinity of the molecule. While the Kavanaugh analogue has desirable oxygen binding and renal clearance characteristics, it too is obtained in low yield.

### D. Gene Expression, Generally

Gene expression embraces the transcription of DNA into messenger RNA, and the translation of messenger RNA into protein. The process of transcription begins when an enzyme, DNA-directed RNA polymerase, binds to DNA. The binding site for this enzyme is often called the "promoter," and the binding of the enzyme to the promoter may be controlled by various repressors or inducers of transcription. The RNA polymerase slides along the DNA molecule, manufacturing a messenger RNA transcript. When it encounters a second regulatory element, the "terminator," the enzyme falls off, and the mRNA transcript is formed.

Messenger RNA is used by the ribosomes, the protein factories of the cell, as a template for the construction of the corresponding protein. The ribosomal binding site comprises the so-called Shine Delgarno (SD) sequence and a properly spaced initiation (start) codon. Beginning at a special RNA triplet known as the initiation codon, transfer RNAs bind to corresponding codons of the messenger. Each transfer RNA is two-handed; it binds to the messenger codon by means of a complementary anti-codon, while holding the corresponding amino acid in position to be linked into the growing polypeptide chain. The chain falls off when the ribosome encounters one of three special triplets known as "stop" codons. That part of the original gene which corresponds to the messenger sequence from the initiator codon to the last codon before the stop codon is known as the coding sequence. There is also a 5'-flanking sequence, which begins with the promoter, and a 3'-flanking sequence which ends with the terminator.

### E. Polycistronic Expression

It is possible for a single messenger RNA transcript to have one promoter, but two or more pairs of start and stop codons that define distinctly translatable sequences. Each such sequence is known as a "cistron," and the polypeptide corresponding to the cistrons are thus co-expressed under the control of the single promoter.

The majority of bacterial operons are polycistronic, that is, several different genes are transcribed as a single message from their operons. Examples include the lactose operon with three linked genes (lacZ, lacY and lacA) and the tryptophan operon with five associated genes (trpE, trpD, trpC, trpB, and trpA). In these operons, the synthesis of messenger RNA is initiated at the promoter and, within the transcript, coding regions are separated by intercistronic regions of various lengths. (An operon is a cluster of genes that is controlled as a single transcriptional genetic unit). Translational efficiency varies from cistron to cistron. Kastelein, et al., Gene, 23: 245-54 (1983).

When intercistronic regions are longer than the span of the ribosome (about 35 bases), dissociation at the stop codon of one cistron is followed by independent initiation at the next cistron. With shorter intercistronic regions, or with overlapping cistrons, the 30S subunit of a terminating ribosome may fail to dissociate from the polycistronic mRNA, being instantly attracted to the next translational initiation site. Lewin, Gene Expression, 143-148.

Unlike bacterial mRNAs, eukaroyotic mRNAs are generally monocistronic in nature. Lewin, Gene Expression, 157.

Synthetic polycistronic operons have been constructed and expressed in both prokaryotes and eukaryotes.

Lee, et al., Nucleic Acids Res., 12: 6797 (1984) describe a special case of a synthetic polycistronic operon in which all of the cistrons express the same polypeptide. This homopolycistronic structure was constructed to maximize the gene dosage effect.

Schoner, et al., PNAS, 83: 8506-10 (1986) translated a synthetic two-cistron mRNA in E. coli. The first cistron was a short, arbitrary AU-rich sequence, while the second cistron was a mammalian gene (bGH). It was found that "read through" translation occurred if the stop codon of the first cistron followed the SD element of the second cistron and lay close to the start codon of the second cistron. Schoner's purpose was to overcome his failure to express Met-bGH with its native codons at high levels, possibly as a result of inhibition of translation by local secondary structures. The first cistron was engineered to favor ribosome binding (by placing the SD sequence and the AUG initiation codon in an AU-rich region free of local secondary structure). See also Schoner, et al., Meth. Enzymol., 153: 401-416 (1987), which reveals that bGH overproduction by this technique was associated with the formation of protein granules.

Saito, et al., J. Biochem., 101: 1281-88 (1987) expressed a synthetic somatomedin C gene in E. coli using a two cistron system. They theorized that the instability of somatomedin C, a basic polypeptide, might be overcome by complexing it with an acidic polypeptide. Thus, they constructed a two-cistron system which could express both polypeptides. The termination codon of the first cistron overlapped the initiation codon of the second cistron. The transformants accumulated Somatomedin C at high levels. However, the somatomedin C was recovered in the form of insoluble pellets (see page 1282).

The ribosomes of mammalian cells are likewise capable of reinitiating translation at an initiation codon downstream from a termination codon. Thus, Boel, et al., FEBS Lett., 219:181 (1987) expressed a dicistronic transcription unit in mammalian (CHO) cells. This unit directed synthesis of both the precursor of human pancreatic polypeptide and of a selectable genetic marker (mouse DHFR).

CODON, WO88/0548 describes the production of dicistronic mRNA which encodes both a protein of interest (e.g., tissue plasminogen activator) and a selectable phenotype (e.g., neomycinresistance). The common promoter was, in each of the examples a derivative of the Harvey murine sarcoma virus, and the dicistronic mRNA was translated in suitable eukaryotic cells.

GENENTECH, EP Appl 117,058 discloses the expression in vertebrate host cells of a dicistronic expression vector wherein one cistron codes for the desired protein (e.g., HbsAg) and a second codes for a second protein (e.g., DHFR) whose synthesis is subject to environmental control (e.g., with methotrexate).

### F. Fused Genes and Proteins, Generally

Genes may be fused together by removing the stop codon of the first gene, and joining it in phase to the second gene. Parts of genes may also be fused, and spacer DNAs which maintain phase may be interposed between the fused sequences. The product of a fused gene is a single polypeptide, not a plurality of polypeptides as is expressed by a polycistronic operon. Different genes have been fused together for a variety of purposes. Thus, Gilbert, U.S. 4,338,397 inserted a rat preproinsulin gene behind a fragment of the E. coli penicillinase gene. His purpose was to direct E. coli transformants to secrete the expression product of the fused gene. Fused genes have also been prepared so that a non-antigenic polypeptide may be expressed already conjugated to an immunogenic carrier protein. The present invention, however, contemplates the joining of two copies of the same gene.

The use of linker DNA sequences to join two different DNA sequences is known. These linkers are used to provide restriction sites for DNA cleavage, or to encode peptides having a unique character that facilitates purification of the encoded fusion protein or a fragment thereof. See, e.g., Rutter, U.S. 4,769,326.

The lectin of Pisum sativum seeds is synthesized as a single 275-amino acid preproprotein consisting of a signal sequence followed first by the beta chain and then by the alpha chain. The signal sequence is removed in the endoplasmic reticulum, and in the protein bodies the resulting "prolectin" is cleaved into a 187-AA beta chain and a 58-AA alpha chain. (Further processing results in truncation at the carboxyl termini). While the pea seed isolate is thus a heterodimer, it was discovered that the uncleaved naturally-occurring "prolectin" also binds carbohydrates, and that this "prolectin" could be expressed in E. coli and recovered in functional form. Stubbs, et al., J. Biol. Chem., 26: 6141-44 (1986).

Toth, U.S. 4,774,180 teaches the expression of polyprotein. This polyprotein was made from a fused DNA sequence encoding both a first polypeptide which catalyzes the reaction of glycine with ATP to form glycyl-adenylate and a second polypeptide which reacts glycyl adenylate with tRNA^{GLY} to obtain the glycine-charged tRNA. These two polypeptides are the alpha and beta subunits of glycine tRNA synthetase which has an α₂β₂ quaternary structure. The two subunits, in the E. coli genome, are encoded by a single dicistronic gene. Toth linked the coding region of the alpha chain to the coding region of the beta chain by means of a linker encoding six amino acids. See also Toth and Schimmel, J. Biol. Chem., 261: 6643-46 (May 1986).

Ladner, U.S. 4,704,692 describes an expert system for finding linkers which may be used to convert two naturally aggregated but chemically separated polypeptide chains into a single polypeptide chain with a similar conformation after folding. This system relies on a database containing amino acid sequences for which 3-D structures are known. The database is examined for candidate amino acid sequences with a span similar in length to the interchain gap to be bridged. The direction and orientation of the candidate peptides in then checked. The algorithm assumes that these peptides will maintain the same length and orientation regardless of the flanking sequences.

Ladner, WO88/0660 presents a hypothetical approach to the preparation of "pseudodimeric" analogues of dimeric repressor proteins. In essence, an amino acid linker is introduced to convert the dimeric molecule into a single chain. According to Ladner, this linker may be designed directly by the method of the '692 patent; alternatively, the linker-encoding DNA is a random oligonucleotide and in vivo selection is used to find a pseudodimer whose linker permits the molecule to fold correctly and bind sequence-specifically to DNA.

Hallewell, et al., J. Biol. Chem., 264: 5260-68 (1989) prepared an analogue of CuZn superoxide dismutase. Each dismutase molecule is a dimer of two identical subunits; a copper ion and a zinc ion are liganded to the subunit. The dimer interaction in CuZn superoxide dismutase is so strong that the subunits have not been separated without inactivating the enzyme. The enzyme has considerable conformational similarity to immunoglobulins; Hallewell, et al., joined two human superoxide dismutase genes, either directly or with DNA encoding a 19-residue human immunologlobulin IgA1 hinge region and expressed the fused genes in a transformed host. In attempting to express the directly joined genes, recombination occurred to eliminate one of the tandem genes in some plasmid molecules. Hallewell, et al., postulated that the direct connection distorted the dimer, causing the exposure of hydrophobic areas which then had a toxic effect. This would have provided selection pressure favoring gene deletion. No recombination was detected with the IgA1 linker construction.

Unfortunately, it cannot be assumed that a pseudodimeric fusion protein containing a peptide linker will fold properly so to be a functional equivalent of its parental heterodimer.

### G. Expression of Soluble Proteins

Efforts to produce heterologous proteins in transformed cells sometimes result in the precipitation of some or all of the protein as insoluble inclusion bodies, also known as refractile bodies. See, e.g., Paul, et al., Eur. J. Cell Biol., 31:171-174 (1983) (human proinsulin/E. coli trpE fusion protein); Denefle, et al., Gene, 56:61-70 (1987) (angogenin); Langley, et al., Eur. J. Biochem., 163:313-321 (1987) (bovine growth hormone); Petrov, et al., Biology of the Cell, 61:1-4 (1987) (calcitonin); Richardson, et al., Biochim. Biophys. Acta, 950:385-94 (1988) (ricin B chain); Davis, et al., Biochemistry, 26:1322-26 (1987) (tumor necrosis factor); Lee, et al., Biochim. Biophys. Res. Commun., 151:598-607 (1988) (gamma interferon); Meng, et al., J. Chromatogr., 443:183-92 (1988) (Somatomedin C); Tsuji, et al., Biochemistry, 26:3129-34 (1987) (interleukin-2). The term "refractile" refers to the ability to observe these bodies by phase contrast microscopy. Frequently, this insoluble protein retains only a fraction of the expected biological activity, possibly due to incorrect folding. It has been suggested that inclusion bodies are formed when molecules of partially folded proteins interact with each other faster than they can fold into their native, active conformation. Kruger, et al., Biopharm, 40 (March 1989); Haase-Pettingell and King, J. Biol. Chem., 263:4977-83 (1988). "Factors contributing to the formation of inclusion bodies in recombinant bacteria remain obscure and it is not easy to predict the physical state of the product of a newly expressed eukaryotic gene in E. coli." Petrov, supra.

While the formation of these inclusion bodies results in enrichment of the recombinant protein, and is therefore sometimes desirable, it also necessitates solubilization of the aggregates and regeneration of the protein's biological activity. Petrov, supra at 4, comments, "sometimes these obstacles seem to be the most critical point of the recombinant technology."

Attempts have been made to solubilize and renature these proteins. Wetzel, U.S. 4,599,197; Builder, U.S. 4,620,948; Olson, U.S. 4,511,503; Jones, U.S. 4,512,922. However, such efforts can be laborious and uncertain of success. See Giantini and Shatkin, Gene, 56:153-160 (1987). As stated by Weir and Sparks, Biochem. J., 245: 85-91 (1987), "proteins vary considerably in their optimal conditions for renaturation; various factors such as pH, salt concentration and type, rate of removal of denaturant, concentration of the target protein and of contaminants may strongly affect the recovery of authentic protein." These complications are avoided if the protein of interest is expressed in soluble form.

Gatenby, et al., Eur. J. Biochem., 168: 227-31 (1987) has discussed difficulties in the preparation of the higher plant enzyme ribulose-bisphosphate carboxylase. This enzyme has the subunit structure L₈S₈, where L is a large subunit and S is a small subunit. In nature, a binding protein apparently maintains L in soluble form prior to assembly with S. Attempts to assemble an active higher plant RuBPCase in E. coli have been frustrated by the formation of an insoluble, inactive aggregate of L.

### H. Bacterial Expression of Human Alpha and beta Globins

Nagai and Thorgerson (Nature, 309: 810-812, 1984) expressed in E. coli a hybrid protein consisting of the 31 amino-terminal residues of the lambda cII protein, an Ile-Glu-Gly-Arg linker, and the complete human beta globin chain. They cleaved the hybrid immediately after the linker with blood coagulation factor Xa, thus liberating the beta-globin chain. Later, Nagai, et al., P.N.A.S. (U.S.A.), 82:7252-55 (1985) took the recombinant DNA-derived human beta globin, naturally derived human alpha globin, and a source of heme and succeeded in producing active human hemoglobin. Because the alpha globin was derived from erythrocytes, the final product may contain undesirable erythrocyte membrane constituents.

More recently, an efficient bacterial expression system for human alpha globin was reported. GB 8711614, filed May 16, 1987; see copending Ser. No. 07/194,338 and WO 88/09179. This led to the production of wholly synthetic human hemoglobin by separate expression of the insoluble globin subunits in separate bacterial cell lines, and in situ refolding of the chains in the presence of oxidized heme cofactor to obtain tetrameric hemoglobin. This procedure is laborious and low in yield. It requires the use of denaturing solvents (urea and guanidine), and chemical reduction of ferric ion to the ferrous state (see example). One object of the present invention is to overcome these disadvantages.

While human alpha and beta globins may be expressed separately in E. coli, Walder, Proceedings, Biotech USA 1988 (San Franciso, Nov. 14-16, 1988) warns at page 360, "isolated alpha and beta [globin] chains are unstable and tend to precipitate." If human alpha and beta globin are not produced in soluble form, they must be solubilized with denaturing agents and then refolded to restore activity. Moreover, when a wild-type alpha globin gene is expressed in E. coli, alpha globin accumulates only slowly. It is not certain whether this is due to inefficient translation or to the action of host proteases, but WO 88/09179 teaches that this problem may be overcome by fusing a short section of the beta globin gene to the alpha globin gene, so that a hybrid protein is produced. This hybrid protein must then be cleaved, e.g., with a protease, to release the globin. If the protease is not completely selective (perhaps because of contamination by other proteases), the desired cleavage product may not be the only one obtained. In any event, that product must be separated from other E. coli polypeptides, and any contaminants associated with the protease.

Sperm whale myoglobin has been expressed in E. coli, demonstrating that bacteria can incorporate prosthetic heme groups into a protein expressed from a cloned eukaryotic gene. Springer and Sligar, PNAS (USA) 84: 8961-65 (1987). Walder says, "it remains to be seen if hemoglobin can be similarly made if both the alpha and beta chains are expressed within the same cell." While there is a high degree of tertiary structure similarity between myoglobin (a single chain protein) and the individual alpha and beta globin subunits, hemoglobin is a heterotetrameric protein, the primary globin sequences have no more than a 27% homology and myoglobin is now known to enjoy significantly higher stability than either alpha or beta globin. Thus, it could not be predicted that co-expression of alpha- and beta-globin in the same cell would result in intracellular assembly of a functional hemoglobin, which requires proper folding of the alpha and beta chains and incorporation of heme.

### I. Human Gene Expression in Yeast, Generally

A number of human proteins have been expressed in transformed yeast cells, especially Saccharomyces cerevisiae, either cytoplasmically or by secretion into the culture medium. King, et al., Biochem. Soc. Transac., 16:1083-1086 (1988). But success is not guaranteed. Thim, et al., FEBS Lett., 212:307-312 (1987) experienced difficulty in obtaining properly crosslinked insulin from yeast cells in which the intact proinsulin-encoding gene had been inserted. They overcame this problem by constructing a modified proinsulin gene which encoded the B and A chains linked by a hexapeptide spacer. The product of this gene was cleaved and the two chains were properly folded and crosslinked by the cells.

Richardson, et al., Biochim. Biophys. Acta, 950:385-94 (1988) expressed the B chain of the heterodimeric protein ricin in E. coli. They reported that it was hard to obtain high levels of secretion of a yeast alpha factor leader/ricin B chain fusion protein. No attempt was made to co-express and assemble the ricin A and B chains.

Murakami, et al., DNA, 6:189-97 (1987) reported production of a heme-containing fused enzyme in transformed yeast cells.

Horwitz, et al., PNAS (USA), 85:8678-82 (Nov. 1988) described the construction of yeast strains which secrete functional mouse variable region/human IgG1 constant region chimeric antibodies into the culture medium. They characterize their paper the first report of the secretion of a foreign multimeric or heterodimeric protein in yeast. But see also Carlson, Mol. Cell. Biol., 8:2638-46 (June 1988), showing transcription and translation of heavy and light-chain cDNAs into polypeptides which associate and bind antigen.

Beggs, et al., Nature, 283:835 (1980) attempted to express a chromosomal rabbit beta globin gene in S. cerevisiae. However, these yeast cells were unable to properly splice the intron-containing globin mRNA transcript.

No admission is made that any reference cited herein is prior art. The description of the work and the citation of publication date are based solely on the published information and the applicants reserve the right to question the accuracy of that information.

### SUMMARY OF THE INVENTION

It is the object of this invention to overcome the aforementioned deficiencies of the prior art. For example, Applicants have achieved the first complete expression and assembly of tetrameric hemoglobin in cells which do not produce hemoglobin in nature. Prior work has related to the separate expression of alpha and beta globin and their extracellular combination with heme to form hemoglobin.

A central feature of the present invention is the intracellular assembly of alpha- and beta-globin-like polypeptides and intracellular incorporation of heme to form a biologically functional hemoglobin-like protein. This intracellular assembly is achieved by expressing the alpha-and beta-globin-like polypeptides in the same cell so that fold together and incorporate heme. An important characteristic of this invention is a substantial reduction the formation of insoluble globin inaggregates, in particular of beta globin, as compared to what is observed when globins are separately expressed in E. coli or S. cerevisiae. Co-expression may be achieved from genes on two separate but compatible plasmids in the same cell, or from two different operons on the same plasmid, or from a single polycistronic operon.

In one embodiment, the alpha- and beta-globin-like polypeptides are co-expressed in bacterial cells. The corresponding genes may be included in the same synthetic operon (i.e., driven by one promoter), or placed in separate operons with separate promoters (which may be the same or different). Preferably, expression of the alpha- and beta-globin is enhanced by placing a "ribosomal loader" sequence as hereafter described before each globin gene. This is particularly advantageous in the case of alpha globin which is more difficult to produce in-quantity.

In another embodiment, the alpha- and beta-globin-like polypeptides are co-expressed in yeast cells. Improvements in both the yield of the alpha globin and the solubility of beta globin are obtained.

A further aspect of the invention is the production of novel intermediates, di-alpha globin and di-beta globin (and mutants thereof), which can be expressed in a cell and assembled with each other or with beta- or alpha-globin-like polypeptides, respectively, into a hemoglobin-like protein. While intracellular assembly is not strictly required, di-alpha and di-beta globin may be considered specially adapted to intracellular assembly of a functional hemoglobin since expression of, e.g., a di-alpha globin is analogous in some respects to intracellular assembly of two alpha globin subunits, differing from assembly as previously discussed in that the association is accomplished by expression of a covalent peptide linker rather than by noncovalent interaction of the subunits. Di-alpha and Di-beta-globin-like polypeptides may be expressed in, preferably, bacterial cells or in yeast cells.

These facets of the invention are now discussed in greater detail.

### Yeast Expression of Hemoglobin-Like Proteins

Applicant have discovered that it is possible to produce human hemoglobin (or mutants thereof) in yeast, especially Saccharomyces cerevisiae. The use of a yeast expression system obviates the need to separate the hemoglobin from bacterial endotoxins. We have also found that alpha and beta globins with the correct N-terminal amino acid may be obtained without first expressing the globin as a part of selectively cleavable fusion protein. We believe that this is because the yeast enzyme methionyl aminopeptidase is capable of removing the N-terminal methionine from Met-alpha-globin and Met-beta-globin to expose the desired N-terminal amino acid (Valine). Production of altered oxygen affinity mutants as discussed in WO88/09179 is of special interest. Such mutants may be produced by site-specific mutagenesis of globin genes followed by cloning and expression in yeast.

In a preferred embodiment, expression is controlled by a "gal-gap49" hybrid promoter as hereafter defined.

### Co-Expression of Alpha and Beta Globin Genes in Yeast Cells

In a preferred embodiment, the alpha and beta globin genes are both expressed within the same yeast cell. Expression of the beta globin gene alone results in the production of beta globin as a largely insoluble, difficult-to-extract protein comprising less than 2% of the total cell protein. Expression of the alpha globin gene alone results in production of alpha globin at very low levels (under 0.5% of the total cell protein). In neither case is heme incorporated. When, however, the alpha and beta globin genes are co-expressed, the transformed yeast cells fold the alpha and beta globin chains together and incorporate heme groups to obtain functional recombinant human hemoglobin in soluble form, accumulating to about 10% of the total cell protein, without any change in the promoters operably linked to the genes.

The alpha and beta globin genes may, in turn, be carried on different plasmids or on the same plasmid within the host cell.

### Polycistronic Co-Expression of the Alpha and Beta Globin Genes in Bacterial Cells.

Applicants have translationally coupled alpha and beta globin genes to a small "ribosomal loader" gene encoding a small consumable peptide that will lead the ribosome directly into the ATG of the desired alpha and beta globin message and thus enhance translational efficiency. The have also placed the alpha and beta globin genes in the same operon so they are transcribed into a single polycistronic mRNA transcript. The globins are then translated as separate polypeptide chains which subsequently are folded together and joined with intracellular heme by transformed cells to form the hemoglobin tetramer. Applicant's method overcomes the problem associated with separate purification of precipitated alpha and beta globins.

The polycistronic expression and assembly of a heterooligomeric human protein in soluble, active form in a heterologous host has not been previously reported. It is especially noteworthy that this was a mammalian protein expressed in a prokaryotic (bacterial) host. It should further be considered that this protein incorporates prosthetic groups, which add a further complication to the goal of proper post-translational processing.

In one embodiment, Met-FX-alpha globin and Met-FX-beta globin are co-expressed, where FX denotes a leader peptide which a recognition site for Factor Xa cleavage activity. FX-alpha globin and FX-beta globin assemble to form a mutant hemoglobin with reversible oxygen binding activity, albeit higher in affinity for oxygen than native hemoglobin. Alternatively, the FX leader, or other fused leader, may be cleaved to obtain a duplicate of native Hgb.

In another embodiment, Met-alpha globin and Met-beta globin are co-expressed. This eliminates the need for a cleavage step.

In a third embodiment, des-val-alpha globin and des-val beta globin are co-expressed. Native alpha and beta globin both begin with valine. The valine may, however, be replaced with methionine, which is of similar hydrophobicity.

In further embodiments, one or more codons of the native genes are altered so that a alpha and/or beta globin-related protein characterized by one or more amino acid differences (insertions, deletions or substitutions) from the native species is produced.

### Di-Alpha and Di-Beta Globins

A new protein, di-alpha globin, has been prepared, which consists of two alpha globin amino acid sequences covalently connected by peptide bonds, preferably through an intermediate linker of one or more amino acids. Surprisingly, these "genetically crosslinked" alpha globin chains were capable of appropriately folding and combining with beta globin and heme to produce functional hemoglobin analogue. The term "genetically crosslinked" refers to the method of production. Two copies of the globin gene are fused together, preferably with a spacer DNA encoding the amino acid linker, so the construct directly encodes the desired di-alpha globin. The term "analogue" is used because in native hemoglobin, the alpha1 and alpha2 subunits are noncovalently bound. The analogous preparation of di-beta globin is also envisioned.

The preparation of "genetically crosslinked" hemoglobins avoids the disadvantages of chemical crosslinking. The latter is inefficient and often requires deoxygenation of the hemoglobin solution and the presence of another molecule (e.g., inositol hexaphosphate or 2,3-DPG) to prevent competing reactions.

In a preferred embodiment, the di-alpha globin and/or the beta globin contain mutations which reduce the oxygen-binding affinity of the hemoglobin analogue in solution so as to approach the oxygen-binding characteristics of whole blood.

The di-alpha hemoglobin advantageously exhibits a substantially longer half-life in the circulatory system than does conventional (des-val) recombinant hemoglobin. Preferably, in humans, the half-life exceeds 9 hours at a dose of at least 1 gm/kgm body weight. This would be expected to correspond to a half-life of about 3 hours in rats given a comparable dose.

The di-alpha and di-beta globins can be expressed in both bacteria and yeast.

### BRIEF DESCRIPTION OF THE DRAWINGS

- Figure 1:: Flowchart for construction of plasmids for expression of FX-alpha globin (pDL II-62m), FX-beta globin (pDL II-10a), and FX-hemoglobin (pDL II-66a) are schematized.

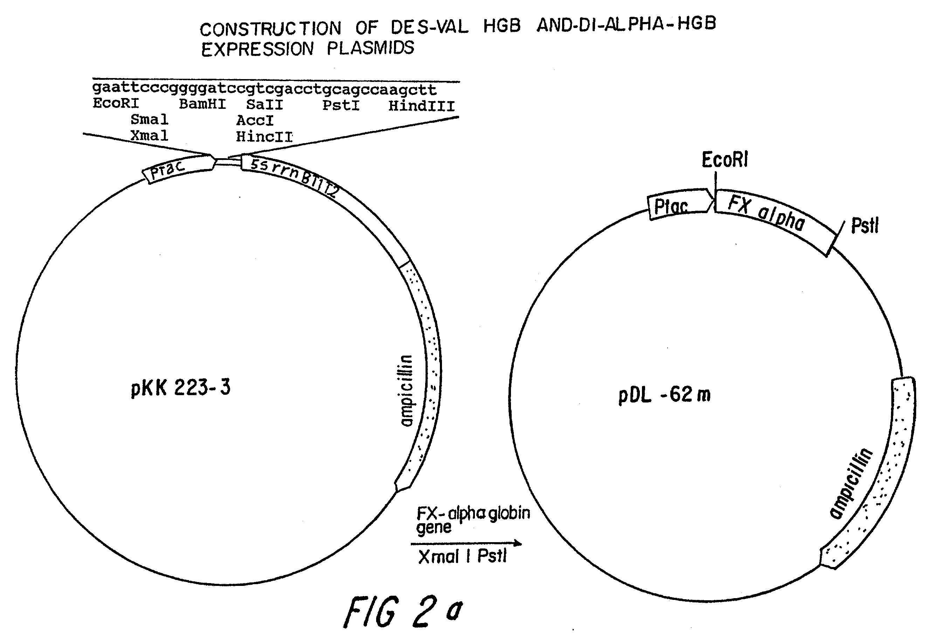

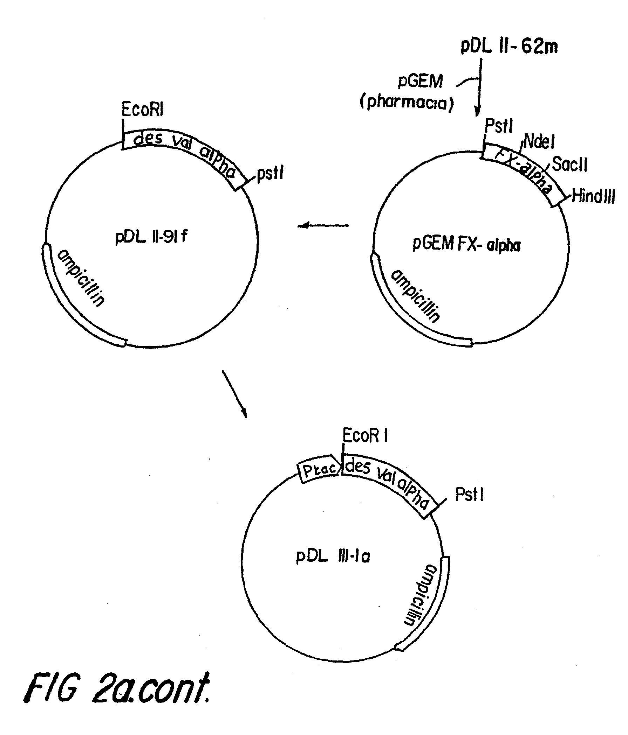

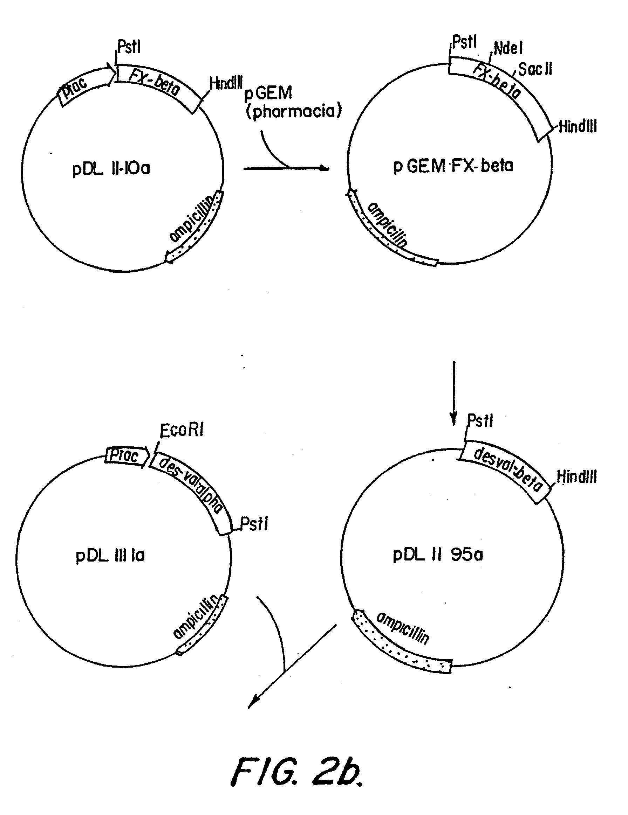

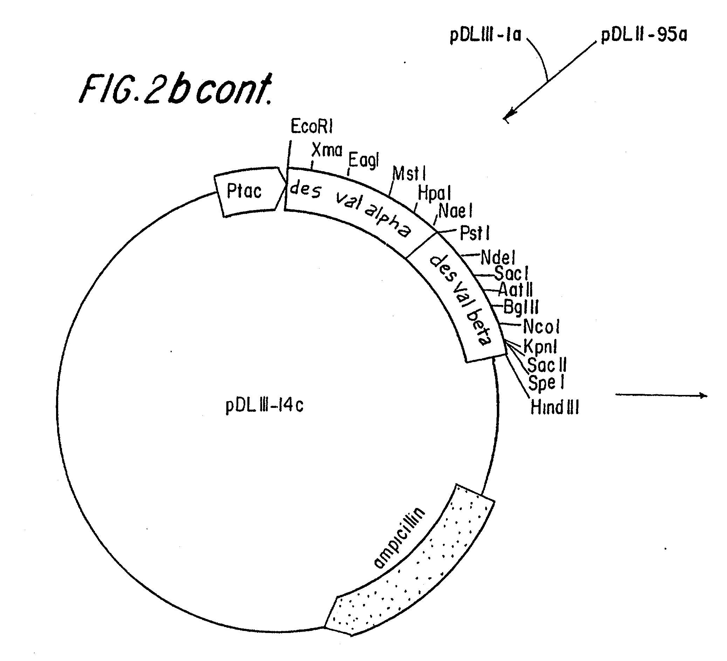

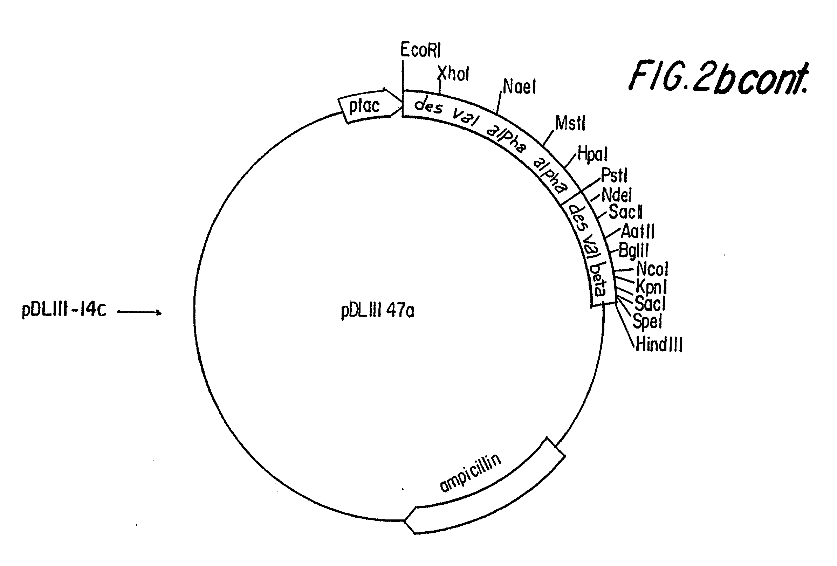

- Figure 2:: Flowcharts for construction of plasmid pDL III-1a (2a) bearing dicistronic Des-Val-Alpha globin gene under control of Tac promoter, and polycistronic di-alpha/beta co-expression plasmid pDL III-47a (2b).

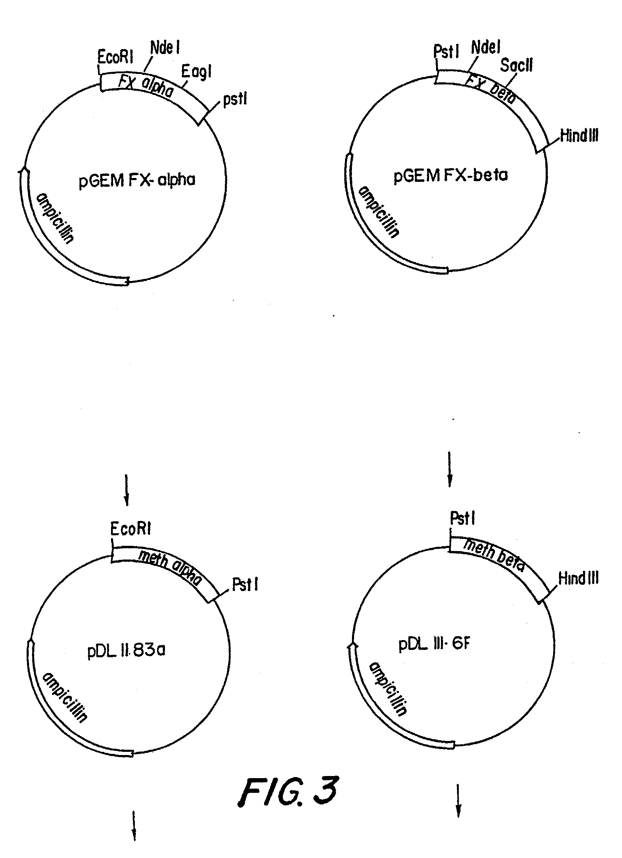

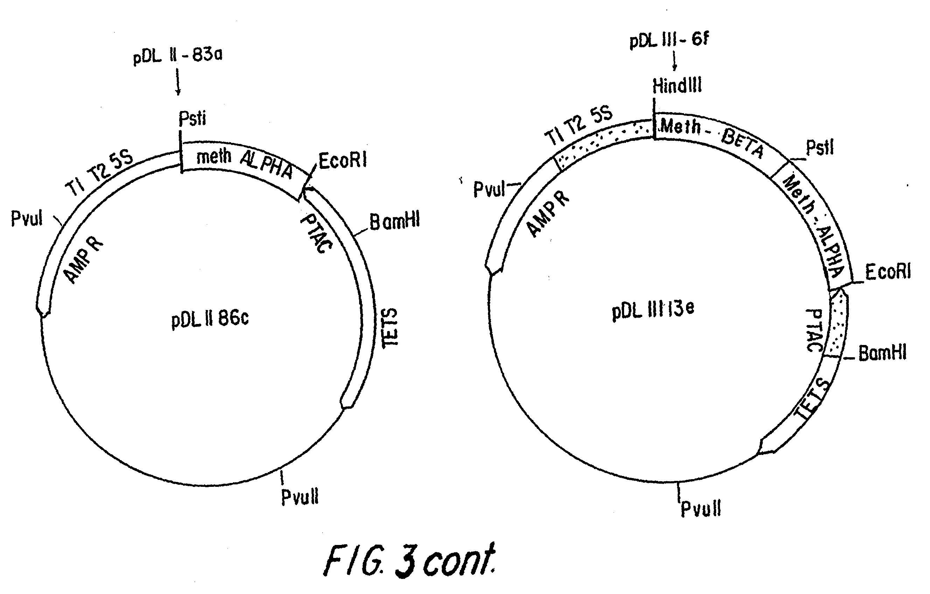

- Figure 3:: Flowchart for construction of plasmid for co-expression of Met-alpha and Met-beta globins, pDL III-13e.
- Figure 4:: Oligonucleotides for construction of synthetic FX-alpha and FX-beta globin genes. The top strand is shown 5' to 3' and the bottom strand as 3' to 5'. Areas of overlap between complementary synthetic oligonucleotides are shown as areas where both strands are shown in the same case letters. The PstI site that joins FX-alpha and FX-beta occurs at the overlap of SJH I-35a and SJH I-36b.
- Figure 5:: Synthetic gene for expression of Met-FX-alpha and Met-FX-beta globin. Region A contains the alpha globin gene and region B the beta globin gene. The location of the Factor X sequence and the two Shine-Delgarno sequences (SD#1 and SD#2) in both regions is indicated. Selected restriction sites are also found. The translated amino acid sequences for the ribosomal loader and Met-FX-alpha/and beta-globin are given.
- Figure 6:: Elution profile and absorbance spectrum for FX-hemoglobin.
- Figure 7:: Oligonucleotides for construction of mutant hemoglobins (differing in amino acid sequence from conventional hemoglobin).
- Figure 8:: Oligonucleotides for construction of plasmids which do not encode the Factor Xa substrate recognition site.
- Figure 10:: Oxygen Binding of Des-Fx Hgb
- Figure 11:: Plasmids pDL III-14c and pDL III-38b

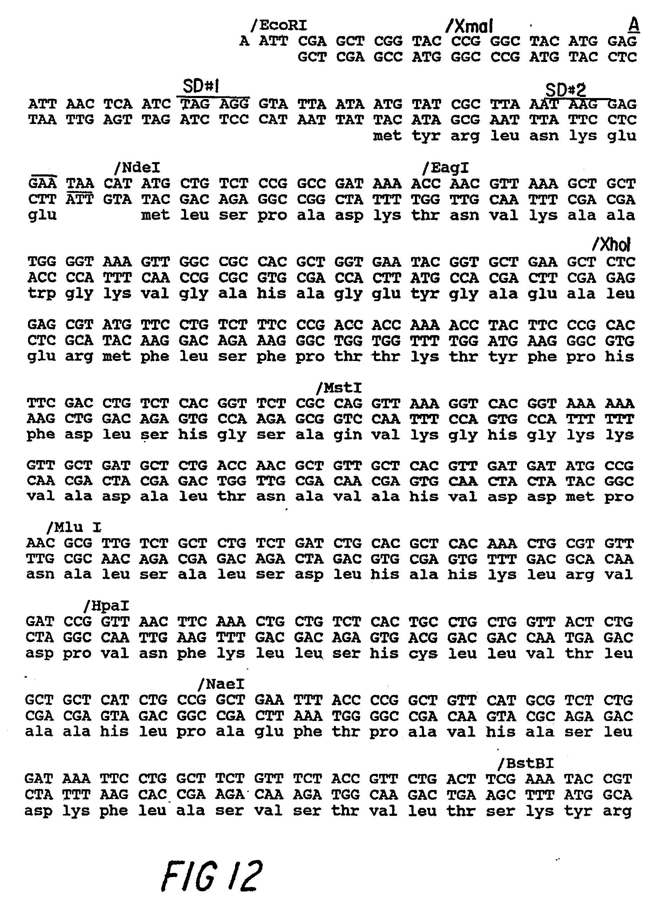

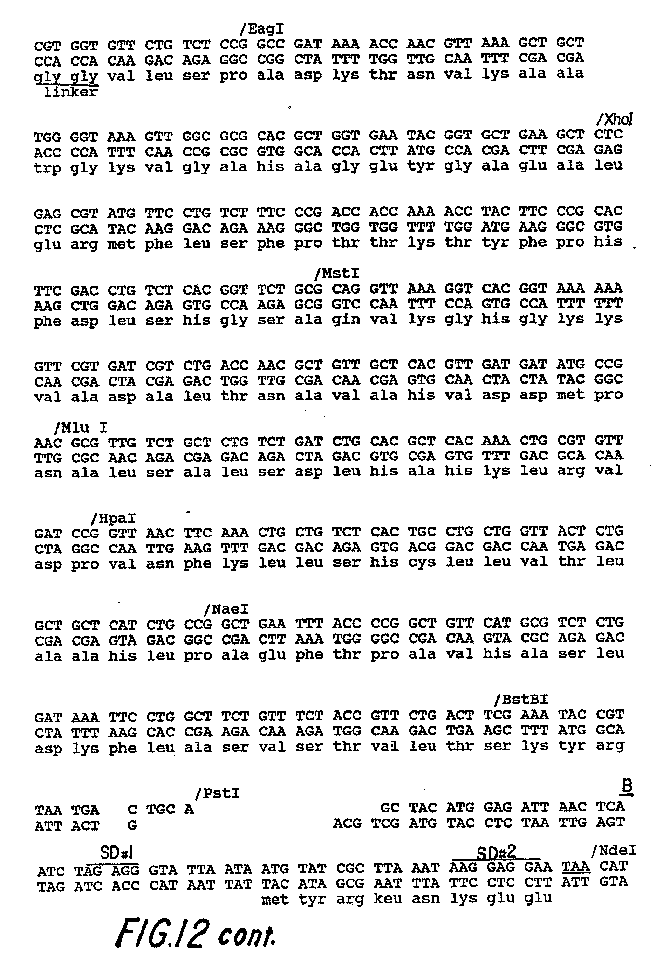

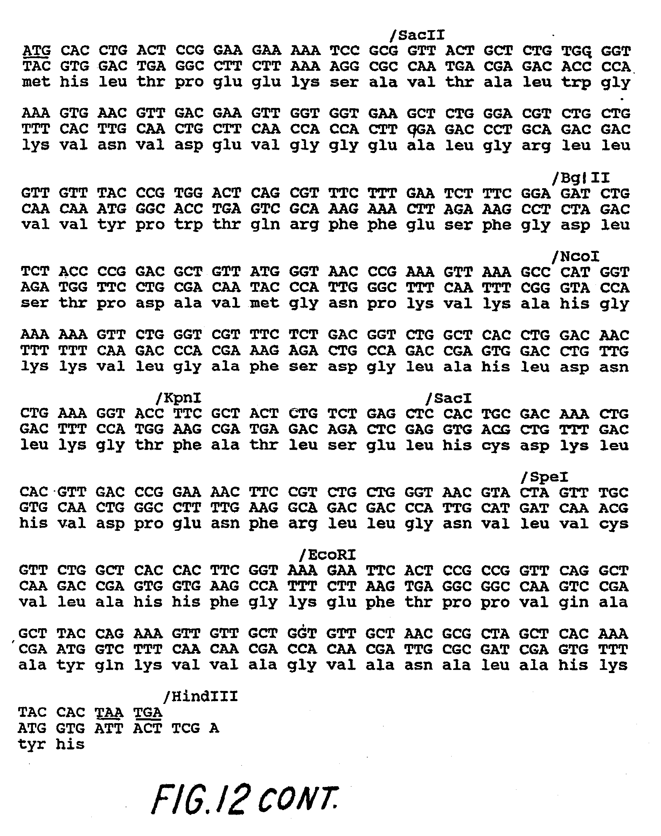

- Figure 12: Shows the sequence of a preferred synthetic gene for expression of (des-Val)-alpha-(GlyGly)-alpha and des-Val beta globin. A shows the region (EcoRI to PstI) containing Shine-Delgarno ribosomal binding sites (SD#1 and SD#2), the sequence expressing the octapeptide (Met...Glu) which serves as a cotranslational coupler, and the sequence encoding the two nearly identical alpha globin-like polypeptides and the interposed Gly-Gly linker. The first alpha globin sequence begins "Met-Leu", that is, it contains an artifactual methionine, omits the valine which is the normal first residue of mature alpha globin, and continues with the second residue, leucine. The second alpha globin sequence begins "Val-Leu", immediately after the underlined "Gly-Gly" linker. Start and stop codons are underlined. B shows the analogous region (PstI to HindIII) containing the coding sequence for des-Val beta globin. A and B are connected at the PstI site to form a single polycistronic operon.
- Figure 13:: Shows the structure of the final expression vector pDL III-47a. "PTac" is the Tac promoter, and "ampicillin" is the ampicillin resistance gene. Figure 13a shows an XbaI-BamHI fragment of PDL III-47a.
- Figure 14:: Plasmid PSGE0.0E4
- Figure 15:: Plasmid pSGE1.1E4
- Figure 16:: Plasmid pDL IV-67a
- Figure 17:: Plasmid pJR VI-54a
- Figure 18:: Plasmid pDL di-alpha/beta/beta
- Figure 19:: Flowchart showing the construction of various expression vectors featuring lambda P_{L} regulation of various polycistronic globin operons.
- Figure 20:: Nucleotide Sequence of GAL-GAP promoter, with restriction sites indicated. The region from SphI to EcoRV contains a synthetic GAL₁₋₁₀ regulatory region (M. Johnston and R. Davis. 1984. Molecular and Cellular Biology, 4:1440-1448). The UAS is in the region numbered 29-63 on this Figure. The region from EcoRV to the XbaI site contains the consensus GAP491 transcriptional start region, with the approximate start of transcription being at 395. (L. McAlister and M.J. Holland. 1983. J. Biol. Chem., 260:15019-15027; J. Holland, et al. 1983. J. Biol. Chem., 258:5291-5299.)
- Figure 21:: Flowcharts showing construction of beta-globin expression cassette (21a and 21b).
- Figure 22:: Flowcharts showing construction of beta-globin expression vector pGS4988 (22a) and alpha-globin expression vector pGS4688 (22b).
- Figure 23:: Flowcharts (23a and 23b) showing construction of an alpha-globin expression cassette and of vectors pGS289 and pGS389 for co-expression of alpha- and beta globin from the same plasmid. Note that alpha and beta globin are expressed from separate promoters.
- Figure 24:: Absorbtion spectra for yeast-produced recombinant and native human hemoglobin.
- Figure 25:: Flowchart (25a) showing construction of di-alpha/beta hemoglobin yeast expression vector and map of plasmid pGS3089 (25b).
- Figure 26:: Map of plasmid pGS3089RGV desβ.

### DETAILED DESCRIPTION OF THE PREFERRED EMBODIMENTS

The structure of conventional hemoglobin is well known. We herewith incorporate by reference the entire text of Bunn and Forget, eds., Hemoglobin: Molecular, Genetic and Clinical Aspects (W.B. Saunders Co., Philadelphia, PA: 1986) and of Fermi and Perutz "Hemoglobin and Myoglobin," in Phillips and Richards, Atlas of Molecular Structures in Biology (Clarendon Press: 1981).

The primary structure of a polypeptide is defined by its amino acid sequence and by identification of any modifications of the side chains of the individual amino acids.

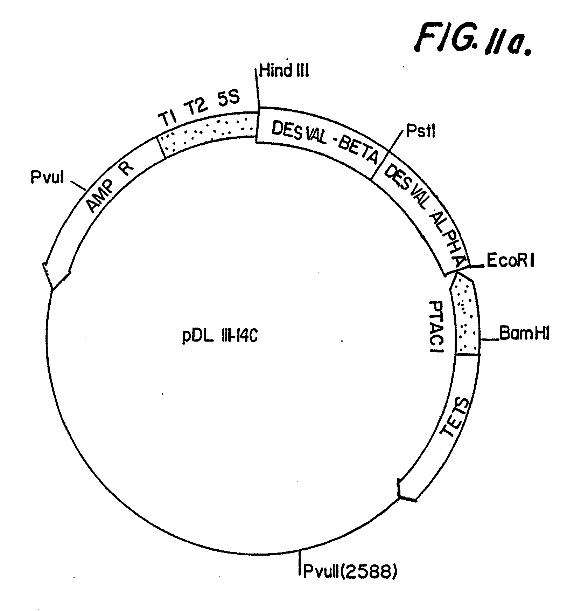

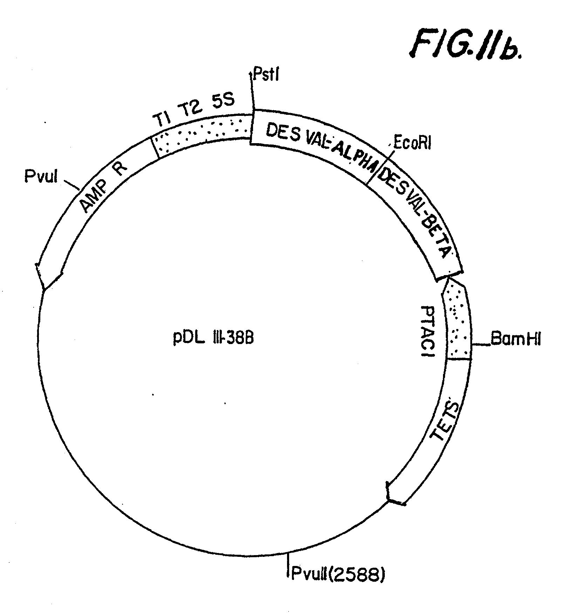

About 92% of the normal adult human hemolysate is Hgb A (designated alpha2 beta2, because it comprises two alpha and two beta chains). The alpha chain consists of 141 amino acids (See Figure 11). The iron atom of the heme (ferroprotoporphyrin IX) group is bound covalently to the imidazole of his 87 (the "proximal histidine"). The beta chain is 146 residues long (see Figure 12) and heme is bound to it at his 92.

Other recognized hemoglobin species are Hgb A₂ (α₂ α₂ δ₂), Hgb A₁ₐ Hgb A_{1b}, and Hgb A_{1c}, as well as the rare species Hgb F (α₂ gamma₂) Hgb Gower-1 (Zeta₂ epsilon₂), Hgb Gower-2 (alpha₂ epsilon₂), Hgb Portland (Zeta₂ gamma₂), and Hgb H (beta₄) and Hgb Bart (gamma₄). They are distinguished from Hgb A by a different selection of polypeptide chains.

Segments of polypeptide chains may be stabilized by folding into one of two common conformations, the alpha helix and the beta pleated sheet. In its native state, about 75% of the hemoglobin molecule is alpha-helical. Alpha-helical segments are separated by segments wherein the chain is less constrained. It is conventional to identify the alpha-helical segments of each chain by letters, e.g., the proximal histidine of the alpha chain is F8 (residue 8 of helix F). The nonhelical segments are identified by letter pairs, indicating which helical segments they connect. Thus, nonhelical segment BC lies between helix B and helix C. In comparing two variants of a particular hemoglobin chain, it may be enlightening to attempt to align the helical segments when seeking to find structural homologies. For the amino acid sequence and helical residue notation for conventional human hemoglobin Aₒ alpha and beta chains, see Bunn and Forget, supra, and Table 1 herein.

The tertiary structure of the hemoglobin molecule refers to the steric relationships of amino acid residues that are far apart in the linear sequence, while quaternary structure refers to the way in which the subunits (chains) are packed together. The tertiary and quaternary structure of the hemoglobin molecule have been discerned by X-ray diffraction analysis of hemoglobin crystals, which allows one to calculate the three-dimensional positions of the very atoms of the molecule.

In its unoxygenated ("deoxy", or "T" for "tense") form, the subunits of hemoglobin (alpha1, alpha2, beta1, and beta2) form a tetrahedron having a twofold axis of symmetry. The axis runs down a water-filled "central cavity". The subunits interact with one another by means of Van der Waals forces, hydrogen bonds and by ionic interactions (or "salt bridges"). The alpha1beta1 and alpha2beta2 interfaces remain relatively fixed during oxygenation. In contrast, there is considerable flux at the alpha1beta2 (and alpha2 beta1) interface. In its oxygenated ("oxy", or "R" for "relaxed" form), the intersubunit distances are increased.

The tertiary and quaternary structures of native oxyhemoglobin and deoxyhemoglobin are sufficiently well known that almost all of the nonhydrogen atoms can be positioned with an accuracy of 0.5 A° or better. For human deoxyhemoglobin, see Fermi, et al., J. Mol. Biol., 175: 159 (1984), and for human oxyhemoglobin, see Shaanan, J. Mol. Biol., 171: 31 (1983), both incorporated by reference.

While analyses of hemoglobin structure tend to focus on the alpha-beta interfaces, it is known that the distance between the amino terminus of one alpha subunit and the carboxyl terminus of the other is about 5.6 A° in the deoxy configuration and 3.3 A° in the oxy configuration.

For the purpose of the appended claims, a hemoglobin-like protein is an oxygen binding protein with a plurality of heme prosthetic groups and a P₅₀ of at least about 6 torr (when measured as for Table 1 of Ser. No. 07/194,338), and composed of a plurality of polypeptides each of which is a human alpha (or di-alpha) globin-like or human beta (or di-beta) globin-like polypeptide. A human alpha globin-like polypeptide is native human alpha globin or a mutant thereof differing from the native sequence by one or more substitutions, deletions or insertions, while remaining at least 75% homologous with human alpha globin, and still capable of incorporating heme and associating with beta globin. A beta globin-like subunit is analogously defined. Subunits of animal hemoglobins or mutants thereof which are sufficiently homologous with human alpha or beta globin are embraced by the term "human alpha or beta globin-like polypeptide." For example, the subunits of bovine hemoglobin are within the scope of these terms. The alpha- and beta-globin-like polypeptides may be referred to collectively as "globins".

For the purpose of the appended claims, a di-alpha globin-like polypeptide is one which consists essentially of two alpha-globin-like polypeptide sequences connected by peptide bonds between the normal C-terminus of the first alpha-globin-like polypeptide and the normal N-terminus of the second alpha-globin-like polypeptide. These two sequences may be directly connected, or connected through a peptide linker of one or more amino acids. Alpha globin chains crosslinked at the N- and C-terminals other than by peptide bonds (e.g., by DIDS) are excluded. The di-alpha globin-like polypeptide must be capable of folding together with beta globin and incorporating heme to form functional hemoglobin-like protein. The di-beta globin-like polypeptide is analogously defined. The di-alpha and di-beta globin-like polypeptides may collectively be referred to as "pseudodimeric globin-like polypeptides".

A "Met FX alpha globin" is an alpha globin-like polypeptide comprising an N-terminal methionine, a oligopeptide which acts as a recognition site for Factor Xa (e.g., Ile-Glu-Gly-Arg), and an alpha globin-like sequence (e.g., Val-His-Leu-Thr-Pro...) which may correspond to wild-type alpha globin or to a mutant thereof as taught herein. The term "Met FX alpha globin" is sometimes abbreviated as "FX alpha globin". "FX beta globin" is an analogously defined beta globin-like polypeptide.

"Met-alpha globin" is an alpha globin-like polypeptide with an extra N-terminal methionine. The second amino acid is valine, which is the first amino acid of mature wild-type alpha globin. Met-beta globin is analogously defined. A "Des-FX alpha globin" gene (or "dFX alpha globin") is a Met-alpha globin gene obtained by excising the FX codons from a Met-FX alpha globin gene. Note that "Met-Hgb" is used to refer to methionyl Hgb formed from methionyl-alpha globin and methionyl-beta globin.

"Des-Val-alpha globin" (or "dVal alpha globin") is an alpha globin-like polypeptide wherein methionine is substituted for the valine which begins the sequence of mature wild-type alpha globin. Des-Val-beta globin is analogously defined. Des-Val-alpba/alpha globin (di-Des-Val-alpha globin) is a "di-alpha globin" in which a "Des-Val-alpha" sequence is linked via an appropriate peptidyl linker to an alpha globin-like sequence which begins with Val.

The alpha and beta globin-like chains need not correspond exactly in sequence to the alpha and beta globins of "conventional" hemoglobin. Rather, mutations may be introduced to alter the oxygen affinity or stability of the hemoglobin, or the ease of expression and assembly of the individual chains. By way of example and not limitation, several mutant hemoglobins have been prepared by the method of this invention. Guidance as to further mutations is provided by the copending application of Hoffman and Nagai, Ser. No. 07/194,338, incorporated by reference herein.

The DNA sequences encoding the individual alpha (or di-alpha) and beta (or di-beta) globin chains may be of genomic, cDNA and synthetic origin, or a combination thereof. Since the genomic globin genes contains introns, genomic DNA must either be expressed in a host which can properly splice the premessenger RNA or modified by excising the introns. Use of an at least partially synthetic gene is preferable for several reasons. First, the codons encoding the desired amino acids may be selected with a view to providing unique or nearly unique restriction sites at convenient points in the sequence, thus facilitating rapid alteration of the sequence by cassette mutagenesis. Second, the codon selection may be made to optimize expression in a selected host. For codon preferences in E. coli, see Konigsberg, et al., PNAS, 80:687-91 (1983). For codon preferences in yeast, see the next section. Finally, secondary structures formed by the messenger RNA transcript may interfere with transcription or translation. If so, these secondary structures may be eliminated by altering the codon selections.

Of course, if a linker is used to genetically crosslink subunits, the linker will normally be encoded by a synthetic DNA. While the di-alpha globin and the beta globin may be expressed separately and then combined with each other and heme in vitro, they are preferably placed on one plasmid.

The present invention is not limited to the use of any particular host cell, vector, or promoter. However, the preferred host cells are bacterial (especially, E. coli) and yeast (especially S. cerevisiae) cells. The promoter selected must be functional in the desired host cells. It preferably is an inducible promoter which, upon induction, provides a high rate of transcription. A preferred bacterial promoter is the Tac promoter, a trp/lac hybrid described fully in DeBoer, U.S. 4,551,433 and commercially available from Pharmacia-LKB. Other promoters which might be used include the temperature sensitive lambda P_{L} and P_{R} promoters, as well as the lac, trp, trc, pIN (lipoprotein promoter and lac operator hybrid), gal and heat shock promoters. The promoter used need not be identical to any naturally-occurring promoter. Guidance for the design of promoters is provided by studies of promoter structure such as that of Harley and Reynolds, Nucleic Acids Res., 15:2343-61 (1987) and papers cited therein. The location of the promoter relative to the first structural gene may be optimized. See Roberts, et al., PNAS (USA), 76:760-4 (1979). The use of a single promoter is favored. Suitable yeast expression systems are described in detail elsewhere in this specification.

The vector used must be one having an origin of replication which is functional in the host cell. It desirably also has unique restriction sites for insertion of the globin genes and the desired regulatory elements and a conventional selectable marker. A vector may be modified to introduce or eliminate restriction sites to make it more suitable for futher manipulations.

The alpha and beta globin chains may be expressed either directly or as part of fusion proteins. When expressed as fusion proteins, the latter may include a site at which they may be cleaved to release the alpha and beta globin free of extraneous polypeptide. If so, a site sensitive to the enzyme Factor Xa may be provided, as taught in Nagai and Thorgenson, EP Appl 161,937, incorporated by references herein. Alternatively, the alpha and beta fusion proteins may be synthesized, folded and heme incorporated to yield a hemoglobin analogue.

The direct expression of the alpha and beta globin subunits is desirable. Factor Xa is a blood derivative. Preparations of Factor Xa may therefore contain undesirable blood-associated substances or etiologic agents. In any event, the hemoglobin must be separated from the Factor Xa.

Nagai and Thorgerson, EP Appl 161,937, incorporated by reference herein, teach the construction of fused genes in which DNA coding for a polypeptide of interest is immediately preceded by DNA encoding a cleavage site for Factor Xa, a serine protease. Certain of the peptide sequences to be ,cleaved by Factor Xa are quoted below (wherein the cleavage site is denoted by an "="):

Ile-Glu-Aly-Arg=Val-His-Leu-Thr  CII FxB-globin

Ile-Glu-Gly-Arg=Thr-Ala-Thr-Ser  Hu prothrombin

Ile-Glu-Gly-Arg=Thr-Ser-Glu-Asp  Bo prothrombin

Ile-Asp-Gly-Arg=Ile-Val-Glu-Gly  Hu prothrombin

Ile-Glu-Gly-Arg=Ile-Val-Glu-Gly  Bo prothrombin

Ala-Glu-Gly-Arg=Asp-Asp-Leu-Tyr  Hu antithrombin III

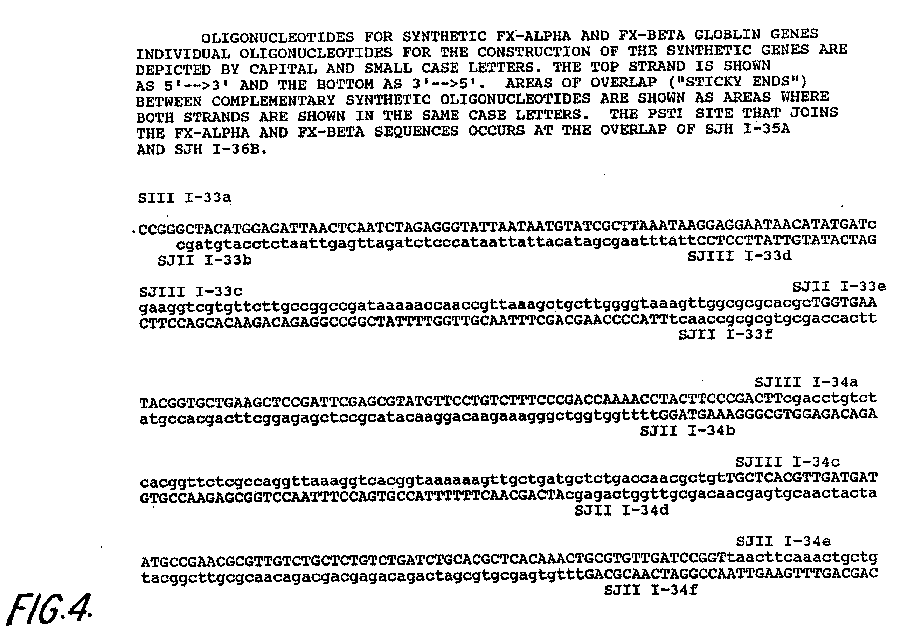

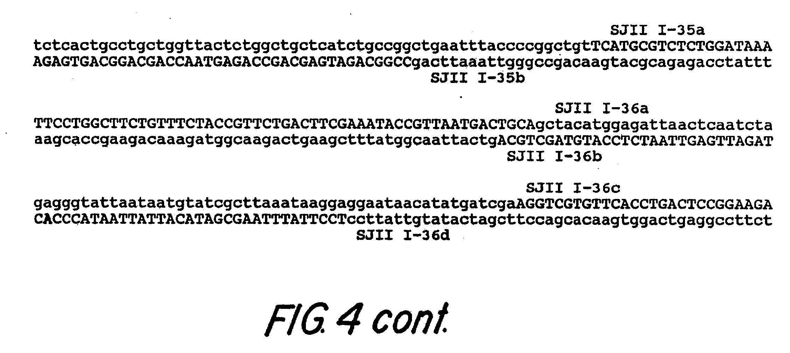

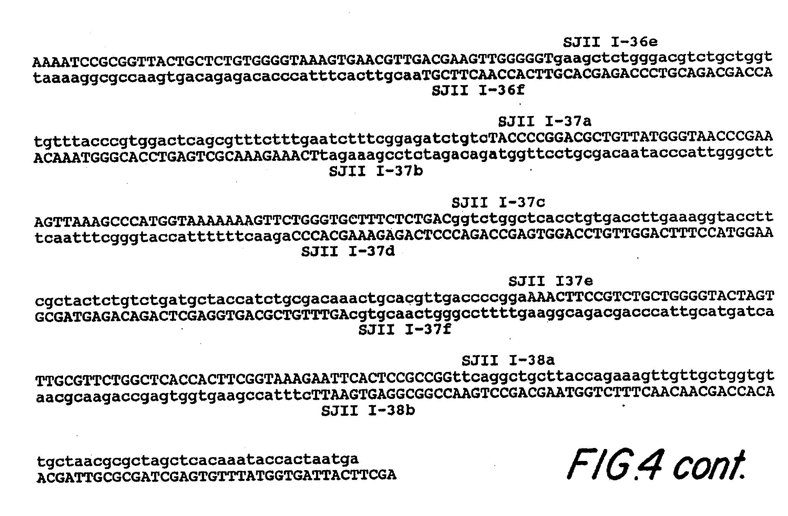

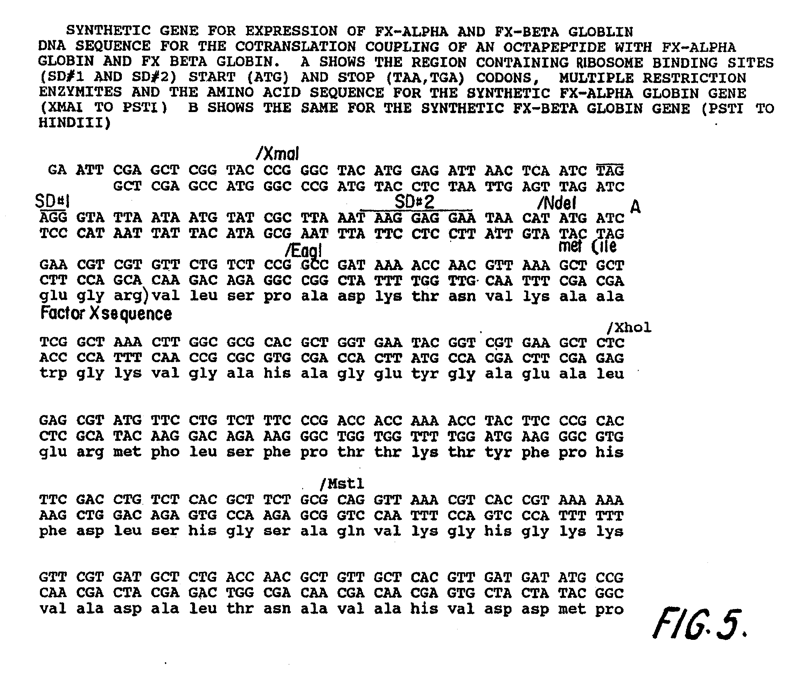

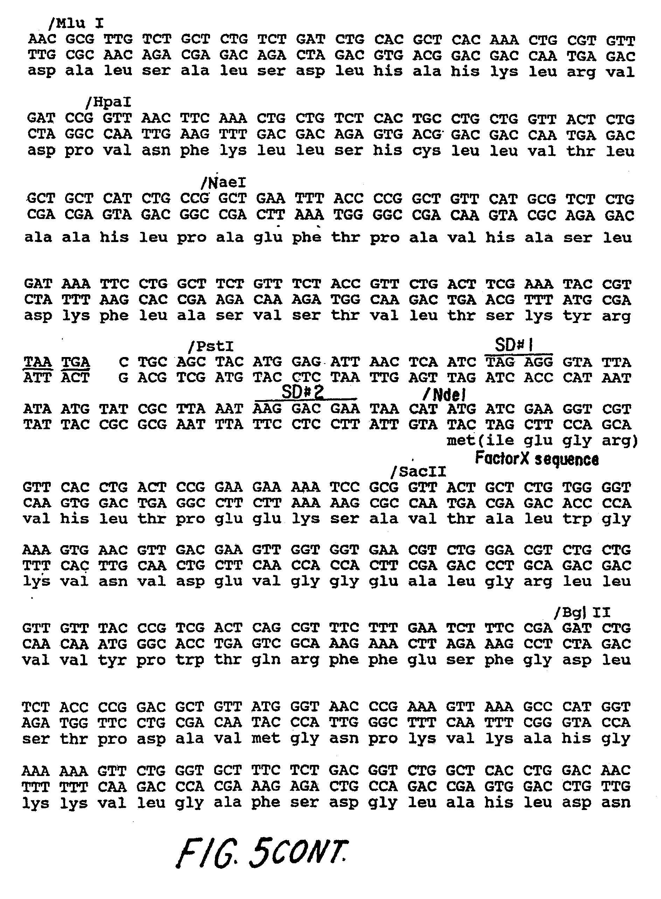

In the above list, "CIIFXβ-globin" refers to a hybrid fusion protein comprises the 31 amino-terminal residues of the lambdaCII protein, the Factor Xa recognition sequence "Ile-Glu-Gly-Arg," and the complete amino acid sequence of human beta globin (which begins "Val-His-Leu-Thr-..."). It will be evident from study of Figure 4 of the present invention that FX-alpha and FX-beta globins of Example 1 correspond to the native globin preceded by "Met-Ile-Glu-Gly-Arg."

In bacterial mRNA, the site at which the ribosome binds to the messenger is a polypurine stretch which lies 4-7 bases upstream of the start (AUG) codon. The consensus sequence of this stretch is 5'...AGGAGG...3', and is frequently referred to as the Shine-Dalgarno sequence. Shine and Dalgarno, Nature, 254: 34 (1975). The exact distance between the SD sequence and the translational start codon, and the base sequence of this "spacer" region, affect the efficiency of translation and may be optimized empirically. Shepard, et al., DNA 1: 125 (1985); DeBoer, et al., DNA 2: 231 (1983); Hui, et al., EMBO J., 3: 623 (1984).

In addition, the SD sequence may itself be modified to alter expression. Hui and DeBoer, PNAS (USA), 84:4762-66 (1987). Comparative studies of ribosomal binding sites, such as the study of Scherer, et al., Nucleic Acids Res., 8:3895-3907 (1907), may provide guidance as to suitable base changes. If the hemoglobin is to be expressed in a host other than E. coli, a ribosomal-binding site preferred by that host should be provided. Zaghbil and Doi, J. Bacteriol., 168:1033-35 (1986).

Any host may be used which recognizes the selected promoter and ribosomal binding site and which has the capability of synthesizing and incorporating heme. Bacterial and yeast hosts are preferred.

The intracellularly assembled hemoglobin may be recovered from the producing cells and purified by any art-recognized technique.

### Polycistronic Co-Expression of Alpha and Beta Globins and Their Assembly Into Hemoglobin

In one embodiment, expression of the alpha and beta globin genes is driven by a single promoter, and the genes are arranged so that a polycistronic messenger RNA transcript is transcribed, from which the separate alpha and beta globin polypeptides are subsequently translated. However, the present invention includes the co-expression of the alpha and beta globin genes from separate promoters, i.e., the host transcribes separate alpha and beta globin mRNAs.

The use of a single promoter is favored on theoretical grounds. Ideally, alpha and beta globin are expressed in stoichiometrically equal amounts. While use of a single promoter does not guarantee equality, it eliminates one unbalancing influence --differences in transcription owing to differences in promoter strength and accessibility. If differences in promoter strength were minimized by use of two identical promoters on the same plasmid, plasmid stability would be reduced as there would be a propensity toward recombination of the homologous regions. We note, however, that in preliminary experiments we have co-expressed alpha and beta globins from separate promoters.

Another justification for using a single promoter is to minimize the number of repressor binding sites.

Preferably, the alpha and beta globin genes are arranged so that the ribosome will translate the alpha globin cistron first. The rationale is that there is some basis for believing that alpha globin affects the folding of beta globin. Nonetheless, the position of the genes may be switched so that beta globin is synthesized first, as is shown in Example 6.

The stability of the polycistronic mRNA transcript, the efficacy of its translation into alpha and beta globin, and the folding of the globin chains into tetrameric hemoglobin may be modified by varying the length and base sequence of the intercistronic regions (the region lying between the stop codon of one cistron and the start codon of the next cistron), the phasing of a second cistron relative to a first cistron, and the position and sequence of the ribosomal binding site for the one cistron relative to the preceding cistron.

In a preferred embodiment, the alpha and beta globin genes are each preceded by a short "introductory" cistron or "ribosomal loader" which facilities the subsequent translation of the globin cistron. In Figure 4, region A contains two cistrons and a Shine-Delgarno sequence preceeding each cistron. The first Shine-Delgarno sequence (SD#1) is bound by the ribosome, which then translates the first cistron, a short cistron encoding an octapeptide. (This cistron is referred to as an "introductory cistron or ribosomal loader.) The second cistron is a globin gene, in this case, an FX alpha-globin gene. The Shine-Delgarno sequence (SD#2) for facilitating translation of the second cistron actually lies within the first cistron. For this reason, the two are said to be "translationally coupled". Region B is identical in structure, except that the second cistron encodes FX-beta globin. Between regions A and B is a 43-base intercistronic region. The introductory cistrons of regions A and B correspond to the first cistron of the two-cistron expression system denoted pCZ144 in Schoner, et al., Meth. Enzymol., 153: 401-16 (1987). The present invention is not, however, limited to the particular "starter" cistron taught by Schoner, et al.; other introductory cistrons that allow for restart of high level translation of a following cistron may be used.

Guidance as to the design of intercistronic sequences and as to the location of SD sequences may be obtained by comparing the translational efficiency of spontaneous or controlled mutants of the same polycistronic operon, as exemplified by Schoner, et al., PNAS, 83: 8506-10 (1980). It is also possible to look for consensus features in the intercistronic regions of different operons. McCarthy, et al., EMBO J., 4: 519-26 (1985) have identified a translation-enhancing intercistronic sequence in the E. coli atp operon.

The present invention is intended to reduce or avoid the localization of the hemoglobin or its component polypeptides into inclusion bodies. Consequently, a further feature of the invention is that the functional hemoglobin is substantially found (preferably over 80%) in the soluble fraction of the cell. It appears that with this invention, over 90% of the functional hemoglobin can be so directed when alpha₂ beta₂ hemoglobin is assembled from alpha- and beta-globin chains co-expressed from a tetracistronic operon as described herein. With di-alpha, beta₂ hemoglobin, nearly 100% is soluble when expression is induced at 25°C and less at higher induction temperatures. These percentages reflect the percent of all di-alpha and beta chains found in the soluble fraction of the cell and not actual recovery of protein from the cell.

### Expression in Yeast

In another embodiment the present invention relates to the production of hemoglobin-like molecules in yeast. Our preferred host for expression of recombinant human hemoglobin in yeast is Saccharomyces cerevisiae. However, other fungi or yeast may be used for the purpose, such as strains of Aspergillus or Pichia. For yeast to be a suitable host it must be capable of being transformed with recombinant vectors, either replicating or integrating types. This allows the insertion of the desired DNA sequence for the gene of interest. It must also be capable of high density cell growth, in appropriate volume to provide sufficient cell mass to isolate the desired gene product from the desired reaction vessels, where ideally the growth would be easily controlled by several parameters including nutrient formulation, agitation and oxygen transfer and temperature. It is also desirable to be able to induce the expression of protein synthesis with the manipulation of the media, temperature, or by the addition or consumption of certain chemicals. Finally, to be a suitable host, the yeast must be capable of producing recombinant proteins, preferably in excess of 1% of the total cell protein. This allows more facile isolation of the desired recombinant protein.

With reference to S. cerevisiae, haploid strains of potential use include:

| | |
|---|---|
| BJY3501 | Matα pep4::HIS3 prb1-Δ 1.6R his3 200 ura3-52 can1 GAL, |
| GSY112 | As above but Leu2::HISG |
| GSY112 cir° | As above but cured of 2µ plasmid |
| BJY3505 | Mata pep4::HIS3 prb1-Δ 1.6R HIS3 lys2-208 trp1-101 ura3-52 gal2 can1 |
| GSY113 | As above but leu2::HISG |
| RSY330 | Matα pep4-3 prb1-1122 hist7 ura3-52 trp1-289 can1 gal1 |
| BJY2168 | Mata prc1-407 prb1-1122 pep4-3 leu2 trp1 ura3-52 |
| BJY1991 | Matα prb1-1122 pep4-3 leu2 trp1 ura3-52 |
| RSY334 | Matα regl-501 pep4-3 prb1-1122 ura3-52 leu2-3, 112 gal1 |
| RSY214 | Matα pep4-3 prb1 ura3-52 |

To date, strains such as GSY112 GSY113 and RSY334 have been the best hemoglobin producers. Strains such as RSY334 that carry the reg1-501 mutation may be particularly important as they uncouple glucose repression from galactose induction, allowing one to induce with lower levels of galactose in the presence of glucose. Because this strain carries the gall mutation it cannot metabolize galactose, so the galactose concentration remains constant and continues to function as a inducer.

Diploid strains formed from crosses of any of the above compatible strains or similar compatible strains may also be useful as they tend to have faster growth rates than haploid strains.

For example, the following diploid strains, which co-express alpha (or di-alpha) and beta globins, are described in the Examples:
BJY350 [pGS4988] x RSY330 [pGS4688]
BJY3505 [pGS4988] x BJY 1991 [pGS4688]

Other matings may likewise be used in practicing the present invention.

The use of protease-deficient strains may also be advantageous.

Yeast expression systems can be divided into two main categories: (1) Systems designed to secrete protein and (2) system designed for the cytoplasmic expression of proteins. The advantages of secretion systems are:
(1) The protein is often easier to purify from culture medium than from total cell extracts.
(2) If the proteins has essential disulfide bonds, they are more likely to form if the protein passes through the secretory pathway. This is thought to be partly due to the presence of protein disulfide isomerase in the endoplasmic reticulum and to a less reducing environment than in the cytoplasm.
(3) Secretion can also be advantageous if the first amino acid (after the initiating methionine) creates a dipeptide sequence that is poorly processed by methionyl aminopeptidase. The addiction of a secretory signal sequence may allow processing by signal peptidase during secretion resulting in a protein with the authentic amino acid at the amino terminus of the protein.

The disadvantages of a secretion system are:
(1) Generally, the expression level are much lower than those that can be obtained by cytoplasmic expression. This seems to be, in part, due to a rate limiting step in secretion.
(2) Not all proteins are secretable.
(3) Often, particularly with S. cerevisiae, misprocessed forms of the protein accumulate and these can be difficult to purify away from correctly processed forms.

The most commonly used secretory expression system used in yeast is the yeast α-factor secretory signal sequences and the α-factor promoter. See A.J. Brake. Yeast Genetic Engineering Eds. P. Barr, A. Brake, and P. Valenzuela. Butterworth Publishing, Boston 1989, for a review. The invertase signal sequence coupled to a variety of promoters has also been used to express heterologous proteins in yeast. See G. Stetler et al., Biotechnology, 7:55-60 (1989), R. Smith et al., Science, 229:1219-1229 (1985).

The advantages of cytoplasmic expression are:
(1) Expression levels can be quite high with reports of proteins expressed at >20% of the total cell protein, usually as a soluble protein.
(2) Some proteins cannot be efficiently secreted.
(3) Proteins that contain glycosylation sites, if secreted from yeast, will be glycosylated with the pattern of sugars unique to yeast. Because these highly hydrophilic, post-translational modifications lie on the surface of proteins they are likely to confer antigenicity on the protein. If the protein is functional without the carbohydrate side chains it may be advantageous to produce the protein without them rather than with the yeast-specific modification (see for ex. Travis et al. 1985. J Biol Chem 260:4384-4389).
(4) Some proteins have other specific modifications that may occur only in the cytoplasm, for example aminoterminal acetylation, modification with lipids, perhaps heme incorporation and so forth.

At present, cytoplasmic expression is preferred since the yeast cells fold together the globin chains and incorporate heme to produce hemoglobin in vivo. However, it is possible to separately express and secrete the alpha and beta globin chains and assemble hemoglobin in vitro.

The globin genes must be placed under the control of a suitable promoter. The commonly used yeast promoters generally fall into two broad categories: regulated and constitutive. Constitutive promoters that are in wide use include GAP, PGK (phosphoglycerate kinase) and the α-factor promoter. Regulated promoters have also been used and these include the yeast metallothionein promoter (regulated by copper), the Gal1-10 promoter, GAL7 promoter (regulated by galactose and glucose) the ADHII promoter (regulated by ethanol and glucose) the PH05 promoter (phosphate regulation) and several hybrid promoters such as PHO5-GAP, GAL-PGK, ADHII-GAP, and GAL-CYC1.

The use of regulated promoters may be important for plasmid stability. Often expression of recombinant proteins, at high levels, inhibits the growth of the host organism. This disadvantage can result in the accumulation of cells in the population with few copies of the plasmid present, a decrease in growth rate and a drop in overall expression levels. By maintaining the gene in a repressed state and inducing late in the fermentation, high growth rates can be maintained and selection against high plasmid copy numbers can be avoided.

It is somewhat difficult to obtain accurate data on the relative strength of yeast promoters because the only true measure of this would be based on kinetics of mRNA synthesis. Most of the data that exists measures only the final protein concentration that has been obtained. Because there can be great differences in protein stability, it is necessary to compare the same protein and mRNA with multiple promoters on identical vectors. A study that approaches this was done by Verbakel et al. (Gene, 61:207-215 (1987)). They compared the GAPDH, CYC1 GAL7, PHO5, and PGK promoters and measured the expression of a β-galactosidase/poliovirus VP2 protein fusion. CYC1 resulted in an expression level of 0.14% of the total cell protein (TCP); GAPDH, 0.22% TCP; PHO5, 0.26% TCP; PGK, 0.9% TCP; and GAL7, 0.96% TCP.

Using our GALGAP hybrid promoter, we have obtained expression levels of hemoglobin that represents ≥15% of the total cell protein. This most probably represents an increased promoter strength along with a highly stable protein product and perhaps mRNA with an extended half life.

The use of a GAL-GAP hybrid promoter is preferred. Both elements (the GAL_{UAS} and the GAP transcriptional initiation site) are well understood. Studies on the mechanisms of transcriptional regulation of the GAL regulon have been fairly extensive. The galactose regulon includes five genes that encode enzymes required for the utilization of galactose. Four of these genes (GAL1, GAL7, GAL10, and GAL2) are expressed only in the presence of galactose. Galactose induction does not occur in the presence of glucose unless the yeast strain bears a mutation in the REG1 gene. The GAL1, 7, 10 and 2 genes are regulated by at least two other genes, GAL80 and GAL4. The GAL4 gene is a transcriptional activator protein that activates mRNA synthesis from the GAL1, 7, 10 and 2 upstream activator sequences (UAS_{GAL}). Although GAL4 is constitutively expressed, it is functionally silent in the absence of galactose. Repression of GAL4 activity, in the absence of galactose is maintained by the product of the GAL80 gene. The GAL80 protein apparently interacts physically with GAL4 to prevent transcriptional activation. Presumably galactose or a galactose derivative prevents this interaction to allow GAL4 mediated induction.

Haploid strains of S.cerevisiae have three different genes encoding the enzyme glyceraldehyde-3-phosphate dehydrogenase (GAP). These genes have been designated TDH1, TDH2 and TDH3 and each is present as a single copy per haploid genome. The TDH3 gene produces approximately 60% of the cell's GAP enzyme and TDH1 and 2 produce about 12% and 28%, respectively (McAllister, L and M.J. Holland, 1985. J. Biol Chem, 260: 15019-15027). Holland's group (Holland et al. 1981. J. Biol Chem, 256:1385-1395; and Holland et al. 1983. J Biol Chem 258:5291-5299) has cloned and characterized the three GAP genes of S.cerevisiae. The clones have been designated pGAP11, pGAP63, and pGAP491. pGAP491 corresponds to the TDH3 gene and is therefore, the most highly expressed. This promoter has been used to express a wide variety of proteins in yeast including:

| Protein | REF |
|---|---|
| α Interferon | (1) |
| Hepatitis B antigen | (1) |
| Thaumatin | (2) |
| Hepatitis B antigen | (3) |
| HIV III reverse transcriptase | (4) |
| human SOC | (5) |
| α1 antiprotease | (6) |
| (1) Bitter, G. and K.M. Eagan. 1984. Gene, 32.263-274. | |
| (2) Edens, L. et al. 1984. Cell, 37:629-633. | |
| (3) Kitano, K. et al. 1987. Biotechnology 5:281-283. | |
| (4) Hallewell, R.A. et al. 1987. Biotechnology 5:363-366. | |
| (5) Barr, P.J. et al. 1987. Biotechnology 5:486-489. | |
| (6) Travis, J. et al. 1985. J Biol Chem 260:4384-4389. | |

This promoter is commonly used as a 600-850bp fragment and is essentially un-regulated. In its long form this is a very powerful promoter. The form we are using consists of only ^{~}200bp 5' of the translational initiation site. This form, with no added enhancer sequences is substantially less active than the longer form of the promoter (Edens, L. et al. Cell, 37:629 (1984)). Our addition of the GAL enhancer region confers both regulation and high levels of expression. With only the GAP491 promoter, alpha and beta globin were produced at a level of less than 0.2% total cell protein; with the GAL-GAP491 hybrid promoter, expression jumped to 7-10% total cell protein.

Several other hybrid promoters are of particular interest:
GAL-SIGMA
   A strong galactose-regulated promoter with the sigma transcriptional start site.
SIGMA-GAP
   A strong peptide hormone-regulated promoter with the GAP491 transcriptional start site.
GAL-EF III
   A strong galactose-regulated promoter with the elongation factor III transcriptional start site.
SIGMA-EF III
   A strong peptide hormone-regulated promoter with the elongation factor III transcriptional start site.

One could easily conceive of other promoter systems that would also work. This would include, but not be limited to, a variety of constitutive promoters. For example, the yeast mating factorα (MFα) promoter or the mating factor a promoter MF(a), the phosphoglycerate kinase promoter (PGK), hexokinase1, hexokinase2, glucokinase, pyruvate kinase, triose phosphate isomerase, phosphoglycerate isomerase, phosphoglycerate mutase, phosphofructose kinase or aldolase promoters may all be used. In short, any well expressed yeast promoter may work for expression of hemoglobin in yeast. A wide variety of naturally occurring, regulated promoters could also be used, for example: GAL1-10, GAL7, PHO5, ADHII have all been used to produce heterologous proteins in yeast. A variety of synthetic or semi-synthetic yeast promoters could also be employed such as GAL-PGK, GAL-MFα-1, GAL-MFa1, GAL-SIGMA. ADHII regulatory sequences could also be coupled to strong transcriptional initiation sites derived from a variety of promoters. The PHO5 regulatory sequence or the sigma element regulatory sequences could also be used to construct powerful hybrid promoters. In addition to yeast promoters, it is conceivable that one could use a powerful prokaryotic promoter like the T7 promoter. In this case, one could place the T7 polymerase under the control of a tightly regulated yeast promoter. Induction of the phage polymerase in yeast cells bearing hemoglobin genes under T7 promoter regulation would allow transcription of the genes by this very efficient phage polymerase.

Because most of the yeast regulatory sequences described above serve as targets for proteins that are positive regulators of transcription, it is conceivable that these proteins may limit transcription in situations where the target sequence is present in many copies. Such a situation may obtain with vectors such as pC1B, pCIT, pC1U or pC1N which may be present in excess of 200 copies per cell. over-expression of the positive regulator (for example GAL4) may result in enhanced expression. It is possible to construct a strain in which the GAL4 gene is altered to remove its promoter and the promoter replaced with the GAL7 or GAL1-10 promoters, both of which are transcribed more efficiently than the GAL4 promoter. In this situation, the positive transcriptional activator protein GAL4 would be expressed at elevated level at the time hemoglobin expression was induced.

The consensus sequence for higher eukaryotic ribosome binding sites has been defined by Kozack (Cell, 44:283-292 (1986)) to be: G^{A A} _{G} CCAUGG. Deviations from this sequences, particularly at the -3 position (A or G), have a large effect on translation of a particular mRNA. Virtually all highly expressed mammalian genes use this sequence. Highly expressed yeast mRNAs, on the other hand, differ from this sequence and instead use the sequence AAAAAUGU (Cigan and Donahue, Gene, 59:1-18 (1987)). The ribosome binding site that we use for expression of the α and β-globins corresponds to the higher eukaryotic ribosome binding site. It is within the contemplation of this invention to systematically alter this RBS to test the effects of changes that make it more closely resemble the RBS of yeast. It should be pointed out, however, that alterations at the -2, -1 and +3 positions, in general, have been found to only slightly affect translational efficiency in yeast and in mammals.

Intracellular expression of genes in S. cerevisiae is primarily affected by the strength of the promoter associated with the gene, the plasmid copy number (for plasmid-borne genes), the transcription terminator, the host strain, and the codon preference pattern of the gene. When secretion of the gene product is desired, the secretion leader sequence becomes significant. It should be noted that with multicopy plasmids, secretion efficiency may be reduced by strong promoter constructions. Ernst, DNA 5:483-491 (1986).

A variety of extrachromosomally replicating vectors (plasmids) are available for transforming yeast cells. The most useful multicopy extrachromosomal yeast vectors are shuttle vectors that use a full length 2µ-circle combined with an E. coli plasmid. These vectors carry genes that allows one to maintain the plasmid in appropriate yeast mutants and antibiotic resistance markers that allow selection in E. coli. Use of the full-length 2µ-circle, in contrast to vectors containing only a partial 2µ sequence, generally results in much higher plasmid stability, particularly in yeast strains that have been cured of endogenous 2µ plasmid. The pC series of vectors described herein are vectors of this type.

Strains could also be constructed in such a way that the GALGAP hemoglobin expression cassettes were integrated into chromosomes by using yeast integrating vectors. Although the copy number of the hemoglobin genes would be lower than for plasmid vectors, they would be quite stable and perhaps not require selection to be maintained in the host cell. Yeast integrating vectors include Yip5 (Struhl, et al, PNAS, 76:1035-39, 1989), Yip1 (Id.), and pGT6 (Tchumper and Carbon, Gene, 10:157-166, 1980). For information on these and other yeast vectors, see Pouwels, et al., Cloning Vector, VI-I, et seq. (Elsevier, 1985).

The alpha and beta globin genes may be introduced by separate plasmids, or both upon the same plasmid. The advantage of a single plasmid system over a double plasmid system is theoretical. It is generally thought that there is an upper limit to the total number of plasmid copies per cell. If it is 1000, for example, the two plasmid system could have only 500 copies of α-chain plasmid and 500 of the β-chain plasmid. A single plasmid of 1000 copies per cell would bear 1000 copies of each α- and β-chain gene. The number of copies may be irrelevant, however, if other factors are limiting. In fact, several groups favor using strains that contain genes integrated into various chromosomal loci. Such strains very stably maintain the foreign gene and do not require special media to maintain selection for the plasmid.

Highly expressed yeast genes show a very high codon bias. The genes encoding glyceraldehyde-3-phosphate dehydrogenase and ADH-I, for example, show a 90% bias for a set of 25 codons. Highly expressed yeast genes (>1% of the total mRNA) have yeast codon bias indices of >.90. Moderately expressed genes (0.1-.05% of the total mRNA) have bias indices of 0.6-0.8, and genes expressed at low levels (>0.05% of the total cell protein) have a codon bias of 0.10-0.50 (Bennetzen and Hall, J. Biol. Chem., 257:3026-3031 (1982)). The calculated value for the codons of the human α-globin cDNA is 0.23. A similar value can be calculated for the β-globin cDNA. Because there is a very high correlation between the most commonly used codons, it is possible that hemoglobin expression from the human cDNA in yeast may be limited by the availability of the appropriate tRNA molecules. If this is so, a complete synthesis of the gene using the most highly favored yeast codons could improve the expression levels. It is quite possible that the greatest negative effect of adverse codon use would be if there was an abundance of codons used in the cDNA that are represented by low abundance tRNAs. In such a case, high level expression of hemoglobin could completely drain that pool of tRNA molecules, reducing translation not only of hemoglobin but of yeast proteins that happen to use that codon as well. In the case of the α-globin human cDNA, the most commonly used leucine codon is CTG (14 of 21), this codon is never used in highly expressed yeast genes (Guthrie and Abelson, The Molecular Biology of the Yeast Saccharomyces, Eds. Stratern, Jones and Broach, 1982. Cold Spring Harbor, NY). The low codon bias index and the presence of rare yeast codons in the globin cDNAs have been sufficient incentive for us to synthesize a modified form of the alpha- and beta-globin genes using the preferred yeast codons.

### "Di-AlPha" and Di-Beta" Hemoglobins

The present invention further contemplates in some embodiments the combination of (a) one molecule of a di-alpha globin-like polypeptide with two molecules of a beta globin-like polypeptide to form a "di-alpha" hemoglobin-like protein; (b) two molecules of an alpha-globin-like polypeptide with one molecule of a di-beta globin-like polypeptide to form a "di-beta" hemoglobin-like protein; or (c) one molecule of a di-alpha globin-like polypeptide with one molecule of a di-beta globin-like polypeptide to form a "di-alpha/di-beta" hemoglobin-like protein.

It should further be noted that the delta, gamma and epsilon chains have considerable homology with the beta chain and that the zeta chain has considerable homology with the alpha chain. Di-delta, di-gamma, di-epsilon and di-zeta polypeptides are therefore within the compass of the invention and may be used in the preparation of novel hemoglobins of types other than Hgb A1.

Preferably the di-alpha linker (if one is used) consists of 1 to 3 amino acids which may be the same or different. A Mono-Gly linker is especially preferred. In designing such a linker, it is important to recognize that it is desirable to use one or more amino acids that will flexibly connect the two subunits, transforming them into domains of a single di-alpha globin polypeptide.

The preparation of "di-beta" mutants is also contemplated. The distance between the N-terminus of one beta subunit and the c-terminus of the other is 18.4 Å in the deoxy configuration and 5.2 Å in the oxy form. Preferably, the di-beta linker consists of 4 to 9 amino acids which may be the same or different.

The linker should be unlikely to form a secondary structure, such as an alpha helix or a beta-sheet. Certain amino acids have a greater tendency to participate in such structures. See Chou and Fasman, Biochemistry, 13:222-245 (1974), incorporated by reference. The amino acids are ranked in order of decreasing participation below. The preferred linker amino acids are boldfaced. Glycine is the most suitable amino acid for this purpose. The most preferred di-alpha linkers are Gly or Gly-Gly.

| Alpha Helix Formers | Beta Sheet Formers |
|---|---|
| Glu (1.53) | Met (1.67) |
| Ala (1.45) | Val (1.65) |
| Leu (1.34) Hα | Ile (1.60) Hβ |
| His (1.24) | Cys (1.30) |
| Met (1.20) | Tyr (1.29) |
| Gln (1.17) | Phe (1.28) |
| Val (1.14) | Gln (1.23) |
| Trp (1.14) | Leu (1.22) |
| Phe (1.12) hα | Thr (1.20) |
| Lys (1.07) | Trp (1.19) hβ |
| Ile (1.00) | Ala (0.97) Iβ |
| Asp (0.98) | Arg (0.90) |
| Thr (0.82) | Gly (0.81) |
| Arg (0.79) | Asp (0.80) iβ |
| Ser (0.79) | Lys (0.74) |
| Cys (0.77) iα | Ser (0.72) |
| Asn (0.73) | His (0.71) |
| Tyr (0.61) bα | Asn (0.65) |
| Pro (0.59) | Pro (0.62) bβ |
| Gly (0.53) Bα | Glu (0.26) Bβ |

(The letter symbols are Hα, strong α former; hα, α former; Iα; weak α former; iα, α indifferent; bα, α breaker; and Bα strong α breaker. The β symbols are analogous. Trp is bβ if near the C-terminal of a β-sheet region.)

The alpha helix of a polypeptide chain comprises an average of 3.6 residues per turn. In globular proteins, the average length is about 17Å, corresponding to 11 residues or 3 helix turns. In alpha and beta globin, the helices range in length from 7 to 21 amino acids (A.A.). The beta pleated sheet comprises 2.3 residues per turn; the average length is about 20Å or 6 residues.

Chou and Fasman define an alpha helix nucleus as a hexapeptide containing four helix forming residues and not more than one helix breaker, and a beta sheet nucleus as a pentapeptide containing three beta sheet forming residues and not more than one sheet breaker.

The amino acid sequence in the vicinity of the di-alpha linker is as follows:

| residue # | 138 | 139 | 140 | 141 | 1 | 2 | 3 | 4 |
|---|---|---|---|---|---|---|---|---|
| AA | Ser | Lys | Tyr | Arg -(XXX)ₙ | Val | Leu | Ser | Pro |
| Helix Not | H21 | HC1 | HC2 | HC3 | NA1 | NA2 | A1 | A2 |
| Helix Pot | 079 | 107 | 061 | 079 | 114 | 134 | 079 | 059 |
| Sheet Pot | 072 | 074 | 129 | 090 | 165 | 122 | 072 | 062 |
| (Note: Helix- and sheet forming potentials have been multiplied by 100 for typographical reasons.) | | | | | | | | |

The di-alpha linker is preferably only 1-3 amino acids. Thus, it can form an alpha helix only in conjunction with the linker "termini". A one or two residue linker, even if composed of amino acids with strong secondary structure propensities, would be unlikely to assume an alpha helix or beta sheet configuration in view of the disruptive effect of, e.g., Arg 141 or Ser 3. If the linker is 3 residues long, it would be preferable that no more than one residue be a strong alpha helix former, unless the linker also included a strong alpha helix breaker.

The amino acid sequence in the vicinity of the di-beta linker may impose more stringent constraints.

| 143 | 144 | 145 | 146 | | 1 | 2 | 3 | 4 |
|---|---|---|---|---|---|---|---|---|
| His | Lys | Tyr | His | - (XXX) - | Val | His | Leu | Thr |
| H21 | HC1 | HC2 | HC3 | | NA1 | NA2 | NA3 | A1 |
| 124 | 107 | 061 | 124 | | 114 | 124 | 134 | 082 |
| 071 | 074 | 129 | 071 | | 165 | 071 | 122 | 120 |

The di-beta linker is longer (preferably 4-9 A.A.) and therefore more susceptible to secondary structure formation. It is desirable that the amino acid adjacent to Val-1 be an alpha helix breaker in view of alpha-helix propensities of Val-His-Leu. More generally, it is desirable that the linker not contain (or cooperate with the proximately linked amino acids to form) an alpha helix nucleus or beta sheet nucleus.

Amino acids with a high propensity toward alpha helix formation may be used in the linker if accompanied by "helix breaking" amino acids. Similarly, Beta sheet formation may be prevented by "sheet disrupting" amino acids.

Of course, prediction of secondary structure using Chou and Fasman's approach has its limitations and the ultimate test of the acceptability of a linker is whether or not the di-alpha or di-beta hemoglobin has the desired affinity for oxygen. In particular, a poly-alanine linker, despite its supposed propensity to alpha-helix formation, may well be of value since the alanine group is compact and therefore the linker should be quite flexible if secondary structure does not form.

In an especially preferred embodiment, di-alpha and beta globin genes are combined into a single polycistronic operon. The use of a polycistronic operon is not, however, necessary to practice the present invention, and the alpha (or di-alpha) and beta (or di-beta) globin genes may be expressed from separate promoters which may be the same or different.

While the preferred "genetically crosslinked hemoglobin" of the present invention is one comprising a di-alpha and/or di-beta globin, other globin chains may be genetically crosslinked and used in the production of hemoglobins of species other than Hgb A1 (α₂β₂).

The appended claims are hereby incorporated by reference as a further enumeration of the preferred embodiments. All cited references are incorporated by reference to the extent necessary to enable the practice of the invention as now or hereafter claimed.

By the means suggested in this specification, we have had the following achievements:
(1) Expression of Met-FX-alpha globin in E. coli from a dicistronic operon comprising a introductory cistron and a Met-FX-alpha globin gene, both transcribed from a Tac promoter. (Example 2)
(2) Expression of Met-FX beta globin in E. coli by similar means. (Example 2)
(3) Co-expression of Met-FX-alpha globin and Met-FX-beta globin in E. coli from a tetracistronic operon comprising an introductory cistron, an FX-alpha globin gene, an intercistronic sequence, a second introductory cistron, and an FX-beta globin gene, all controlled by a single Tac promoter. The alpha and beta globins were intracellularly assembled into functional FX hemoglobin. The FX Hgb was enzymatically converted to Hgb. (Example 3)
(4) Co-expression as in (3) above of mutants of FX-hemoglobin in which the beta globin subunits possessed the Beth Israel, Cheverly, Providence/MSR, Kansas or beta⁶⁷ Val → Ile mutations. (Example 4)
(5) Co-expression as in (3) above of Met-alpha globin and Met-beta globin and intracellular assembly into Met-Hgb (a.k.a. Des-FX-Hgb). (Example 5)
(6) Co-expression as in (3) above of Des-Val-alpha globin and Des-Val-beta globin. (Example 6)
(7) Co-expression as in (6) above, but with the Des-Val-beta globin gene preceeding the Des-Val-alpha globin gene within the operon. (Example 6)
(8) Co-expression as in (3) above of (Des-Val)-alpha-(Gly-Gly)-alpha globin and beta-globin and intracellular assembly into a di-alpha Hgb. (Example 8)
(9) Co-expression as in (8) above of (Des-Val-alpha-(GlyGly)-alpha globin and a mutant (Nagai, Arg-Nagai, or Kansas) of beta globin and intracellular assembly into a mutant di-alpha Hgb. (Example 9)
(10) Co-expresion as in (3) above of Des-Val-alpha globin and a Des-Val beta-globin with the Presbyterian mutation and intracellular assembly into a mutant Des-Val Hgb. (Example 11).
(11) Co-expression as in (8) above of a (Des-Val)-alpha-(GlyGly)-alpha globin and of a betal globin with the Presbyterian mutation. (Example 11).
(12) Expression of di-alpha globin and beta-globin from separate promoters on the same plasmid (Example 12).
(13) Devised Hypothetical Protocol for Co-expression of di-alpha globin and two copies of beta globin from same operon (Example 13).
(14) Devised Hypothetical Protocol for Co-produciton of Di-beta Hgb (Example 14).
(15) Devised Hypothetical Protocol for co-expression of alpha globin and beta-globin under control of separate promoters on the same plasmid (Example 16).
(16) Devised Hypothetical Protocol for co-expression of alpha and beta-globin under control of different promoters on different plasmids (Example 17).
(17) Co-expression of α and β globin in S. cerevisiae (Example 19).
(18) Co-expression of di-α globin and β-globin in S. cerevisiae. (Example 23).
(19) Construction of Des-Val-alpha globin and Des-Val-beta globin in E. coli under lambda P_{L} control.
(20) Co-Expression of di-alpha globin and Des-Val-beta globin in E. coli under lambda P_{L} control.
(21) Co-Expression of alpha and beta globin from separate plasmids in diploid strains of S. cerevisiae.
(22) Co-Expression of di-alpha globin and beta globin Presbyterian mutant in S. cerevisiae.
(23) Preparation of vectors for expression of di-alpha globins with -Gly- or -Pro- linkers.
(24) Evaluation of different strains and induction temperatures for expression of di-alpha Hgb in E. coli.
(25) Co-Expression of alpha and mutant beta globin in S. cerevisiae, assembling to form low-affinity Hgb mutants.

An unexpected and surprising change in oxygen binding characteristics of hemoglobin was observed upon replacement of the N-terminal valine with methionine. As illustrated in Example 11, hemoglobin Aₒ purified from blood has a P₅₀ value of 4.03 with N=2.7. DesFX-hgb produced in E. coli, a hemoglobin identical to Aₒ except for the addition of a methionine at the N-termini of the alpha and beta chains, has essentially the same P₅₀ and N values. (Example 7). Thus, the addition of a methionine, without altering the adjacent valine residue, has little or no effect on oxygen binding. On the other hand, a two-fold higher P₅₀ value, 7.04, was observed for desVal-hgb produced in E. coli, a hemoglobin in which the normal N-terminal valine of each chain was replaced with methionine. Cooperativity, as measured by N, was the same, however, for this molecule as for the others. A similar comparison was made for two hemoglobins each containing a crosslinked alpha chain and each containing the Presbyterian mutation, one produced in E. coli (Example 11) and one in yeast. The E. coli hemoglobin was constructed with a Des-Val alpha chain, i.e., the N-terminus had the normal valine replaced with methionine. Oxygen binding was characterized by P₅₀=33 and N=2.4 (Example 11). The corresponding yeast coding region begins with an additional methionine codon in front of the normal valine codon. Because this initial methionine is removed post translationally in vivo, the purified hemoglobin has a normal N-terminal valine. For this molecule, P₅₀=23 to 25 and N=2.5. Thus, in two quite different cases, the replacement of an N-terminal valine with an N-terminal methionine increased the P₅₀ value. Under physiological conditions, it is expected that the crosslinked Presbyterian hemoglobin produced in E. coli will deliver 20-30% more oxygen than the similar hemoglobin, with its altered N-terminus, produced in yeast.

A very large number of different plasmids are referred to in the Examples which follow. In order to highlight the relationships among these plasmids, a Table of vectors has been compiled (See Table 100).

### Example 1: Construction of FX-alpha Globin (pDLII-62m) and BetaGlobin (pDLII-10a) Expression Vectors

### Materials and Methods

Unless otherwise stated all electroelutions, phenol/chloroform extractions, ethanol (EtOH) precipitations, alkaline-SDS plasmid purifications, agarose electrophoresis and DNA manipulations were carried out essentially as described by Maniatis et al. ("Molecular Cloning" Cold Spring Harbor, New York, 1982).

The following abbreviations and definitions are used: ethylenediaminetetraacetic acid (EDTA); sodium dodecylsulfate (SDS); polyacrylamide gel electrophoresis (PAGE); dimethylsulfoxide (DMSO); dithiothreitol (DTT); isopropyl-beta-D-thiogalactopyranoside (IPTG); 2xYT medium (16g bacto tryptone, 10g Bacto yeast extract, 5g NaCl per liter water); SDS-PAGE loading buffer (0.125M Tris-HCl, pH6.8, 20% v/v glycerol, 2% SDS, 2% 2-mercaptoethanol, 0.01% bromphenol blue); phosphate buffered TB medium (24g yeast extract, 12g tryptone, 4 mL glycerol, 17mL 1M KH₂PO₄, 72 mL 1M K₂ HPO₄ per liter water); Tris-EDTA buffer (TE: 10mM Tris, pH8.0, 1mM EDTA); Tris-borate, EDTA buffer (TBE: 0.089 M Tris, 0.089 M boric acid, 0.002 M EDTA); Tris-acetate EDTA buffer (TAE: 0.04 M Tris, 0.04 M acetic acid, 0.001 M EDTA).

Protein electrophoresis was performed by the method of Laemmli, U.K. (Nature 1970, 227, 680-685) on 15% SDS-polyacrylamide gels.

E. coli JM109 cells were made competent as follows: Two hundred milliliters of 2xYT medium was inoculated with 2mL of an E. coli JM109 overnight culture. The 200mL culture was then incubated with shaking at 37°C for 1 hour. Cell were harvested by centrifugation at 6000 rpm at 4°C for 4 min in a Beckman JS13.1 swinging bucket rotor (Beckman Instruments, Inc., Palo Alto, CA). The cells were resuspended in 50mL total of a buffer containing 45mM MnCl2, 60mM CaCl₂, 40mM KOAc, pH 6.2, 15% sucrose (w/v) 1.3% RbCl (w/v), and 7.5% (v/v) glycerol. Following centrifugation as above, the cells were resuspended in 20mL of the same buffer and incubated at 0°C for 30 minutes. cells were dispensed in one milliliter aliquots and stored at -80°C until used.

Unless otherwise stated, all restriction enzyme digests were performed under conditions suggested by the manufacturer. DNA concentrations were determined by Beer's law using measured absorbance at 260 nm (A₂₆₀) against a water reference standard. For synthetic oligonucleotides the following extinctions were used: E²⁶⁰ = 0.05mL/ug/cm and for double stranded DNA E²⁶⁰ = 0.02 mL/ug/cm.

### Globin Gene Synthesis

Each globin gene was constructed from 14 separate oligonucleotides ranging in length from 50-85 base pairs. The FX-alpha globin gene was synthesized from oligonucleotides SJH I-33a-f, SJH I-34a-f and SJH I-35a,b; FX-beta globin gene was synthesized from SJH I-36a-f, SJH I-37a-f and SJH I-38a,b. Each globin gene is preceeded by a short loader gene as previously described. Oligonucleotides were synthesized on a Biosearch 8600 instrument using beta-cyanoethylphosphoramidite chemistry on 1,000 angstrom CPG columns (0.2 mmole) (Beaucage, S.L. and Caruthers, M.H. Tet. Lett. 1981, 22, 1859-1862). The sequence of these oligonucleotides is given in Figure 4.

Unless otherwise stated, the oligonucleotides were cleaved from the columns using the following protocol: Approximately 0.5mL of fresh, concentrated NH₄ OH was drawn into the column with a 1mL syringe. The NH₄ OH was allowed to react for 20min then expressed into a glass vial (approximately 4mL capacity). This process was repeated 2 times, the vial was filled to greater than 75% capacity with NH₄ OH, and heated at 55°C, overnight. The samples were lyophilized, resuspended in 0.1mL H₂O and the concentration estimated by measuring A₂₆₀.

Two hundred micrograms of the individual oligonucleotides were purified by urea polyacrylamide gel electrophoresis. To do this, an equal volume of 2x loading buffer (90% formamide (v/v), 0.5xTBE, 0.05% (w/v) bromophenol blue, 0.05% (w/v) xylene cyanol) was added to the oligonucleotide. The sample was heated at 95°C for 10 min. and applied to a 10% acrylamide gel containing 7M urea and 1XTBE. Electrophoresis was at 800 volts for approximately 2 hrs. The full length oligonucleotide was visualized under ultraviolet light. That region of the gel was then excised and incubated in 3mL of 100mM Tris, pH 7.8, 500mM NaCl, 5mM EDTA buffer at 60°C, overnight.

The oligonucleotide solution was further purified by reverse phase chromatography as follows: A C18 Sep-Pak cartridge (Waters Associates) was washed with 10mL 100% methanol followed by 10mL of H₂O. The oligonucleotide solution was applied to the column, washed with 20mL H₂O and eluted with 3x1mL aliquots of 50mM triethylammonium acetate, pH 7.3/methanol (1:1). The purified oligonucleotide was lyophilized, washed with 100% ethanol, dried, and resuspended in 0.1mL H₂O. The concentration was determined by A₂₆₀.

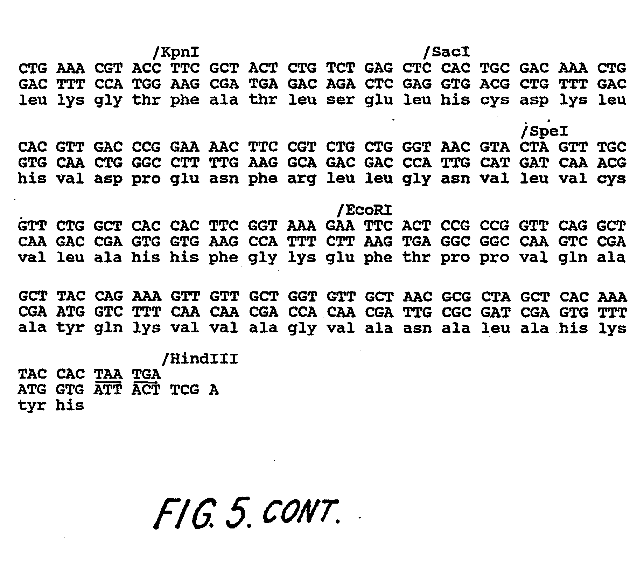

The synthetic FX-beta gene sequence (included in Figure 5) was constructed as follows: 100pmole of the following oligo nucleotides were kinased in 3 separate reactions. Reaction 1 contained oligonucleotides SJH I-36b, c, d, e, and f. Reaction 2 contained SJH I-37a, b, c, and e. Reaction 3 contained SJH I-37d, f, and SJH I-38a. After combining the appropriate oligonucleotides, the solutions were lyophilized to dryness and resuspended in 16uL of H₂O. Two uL of 10x kinase buffer (0.5M Tris-HCl, pH7.4, 0.1M MgCl₂), 0.5 uL of 100mM DTT, and 1uL of 1.0mM ATP were then added. The reaction was initiated by addition of 1uL (2U) of T4 polynucleotide kinase (IBI, Inc., New Haven, CT). After incubation at 37°C for 1 hour, the reactions were heated to 95°C for 10 minutes to inactivate the kinase. The three reactions were combined and 100 pmoles of oligonucleotides SJH I-36a and SJH I-38b were added. After addition of 10uL of 100mM Tris, pH 7.8, 100mM MgCl₂, the oligonucleotides were allowed to anneal by incubating at 65°C for 30 min, 37°C for 30min, and 15°C for 1 hour. Annealed oligonucleotides were ligated by addition of ATP (1mM, final) and DTT (10 mM final) and 4uL (20U) T4 DNA ligase (IBI, Inc., New Haven, CT) and incubation at 15°C for 1 hour. Aliquots of this ligation mixture were then cloned directly into M13mp19 (see below).

Oligonucleotides for the construction of FX-alpha globin were similarly purified, kinased, annealed, and ligated. Before ligation into M13mp19, the full length FX-alpha globin gene was purified by electrophoresis through 0.8% agarose in 1XTAE buffer and electroeluted into dialysis tubing using 0.5X TBE as the electroelution buffer by the method of Maniatis et al. Eluted DNA was phenol extracted, EtOH precipitated, and resuspended in 20uL TE buffer. Aliquots were used for cloning into M13mp19.

### Phage Vectors

For cloning, a 2, 5 or 10 fold molar excess of the individual FX-alpha and FX-beta globin gene sequences were combined with 200ng of double cut, gel purified M13mp19-RF (New England BioLabs, Inc., Beverly, MD) and ligated overnight at 15°C in 50uL ligation buffer (IBI, Inc., New Haven, CT) containing 2U of T4 ligase. FX-Alpha globin was cloned into the Xmal/PstI sites of M13mp19. FX-Beta globin was cloned into the PstI/HindIII sites of M13mp19.

E.coli JM109 was transformed with the M13mp19 ligation mixture containing the FX-alpha or FX-beta globin gene sequences using the following transformation protocol: Nine microliters of DMSO was added to 0.25mL of competent E. coli JM109 and the cells were incubated on ice for 10min. Aliquots of the FX-alpha or FX-beta globin ligation reactions were added and incubated on ice for 40 minutes and at 42°C for 3 min. One hundred microliters of a JM109 overnight culture was added to each transformation mix followed by 60µL of a solution containing 50µL of 2% (w/v) 5-bromo-4-chloro-3-indolyl galactopyranoside in dimethylformamide and 10µL of 100mM isopropylthiogalactoside. Molten B-top agar (10g Bacto tryptone, 8g NaCl, 6g agar per liter), 2.5mL was added and the mixture poured onto a B-bottom agar plate (10g Bacto tryptone, 8g NaCl, 12g of agar per liter). Following incubation overnight at 37°C colorless plaques (i.e., clones containing inserted DNA) were removed from the plates using sterile transfer pipettes and inoculated into 1mL of a JM109 overnight culture diluted 1:100 in 2xYT media.

Bacteriophage clones were grown at 37°C for 6 to 8 hrs. and centrifuged in a microcentrifuge for 5 minutes. The cell pellets were processed for M13mp19 RF using the alkaline-SDS method and resuspended in 20µL of TE buffer containing 20µg/mL DNAase free RNAase (Sigma, St. Louis, MO.). Aliquots (1-3µL) of the RF preparations were digested with 10-20 units each of the appropriate restriction enzymes (see above) and analyzed on 0.8% agarose electrophoresis gels (see above).

M13mp19 clones containing the correct size inserts were grown in larger quantities to obtain single stranded phage DNA for sequencing. Thirty-five milliliters of 2xYT medium was inoculated with 0.3mL of a JM109 overnight culture. After growth for 1 hour at 37°C, the 35mL culture was inoculated with 200µL of the appropriate phage-containing supernatant. The culture was incubated for 6 hours at 37°C and the culture supernatant collected by centrifugation at 9000 rpm for 10 min in a JS13.1 rotor. The phage were precipitated from 31mL of supernatant by addition of 5mL 4M NaCl and 4mL 40% (w/w) polyethylene glycol 6000 and recentrifuged as above. The phage pellet was resuspended in 0.4mL of TE, extracted with phenol/chloroform/isoamyl alcohol (50:49:1 (v/v)) three times, chloroform/isoamyl alcohol (49:1 (v/v)) one time, and ethanol precipitated. The DNA pellet was resuspended in 20µL TE and quantitated spectrophotometrically by A₂₆₀. One microgram of phage DNA was used per set of sequencing reactions.

Sequencing was by the dideoxy method of Sanger (Sanger, F.S. et al. Proc. Nat. Acad. Sci., USA 1977, 74, 5463-5467) with M13 -20 and M13 -40 universal primers (New England BioLabs, Beverly, MA) and gene specific primers (for FX-alpha globin the following primers were used: alpha-1 5'-CGTATGTTCCTGTCTTT-3'; alpha-2 5'-ACAAACTGCGTGTTGAT-3'; for FX-beta globin the following primers were used: beta-1 5'-GCTGGTTGTTTACCCGT-3'; beta-2 5'-ACCCGGAAAACTTCCGTC-3').

### Expression Vectors pDL II-62m and PDL II-10a

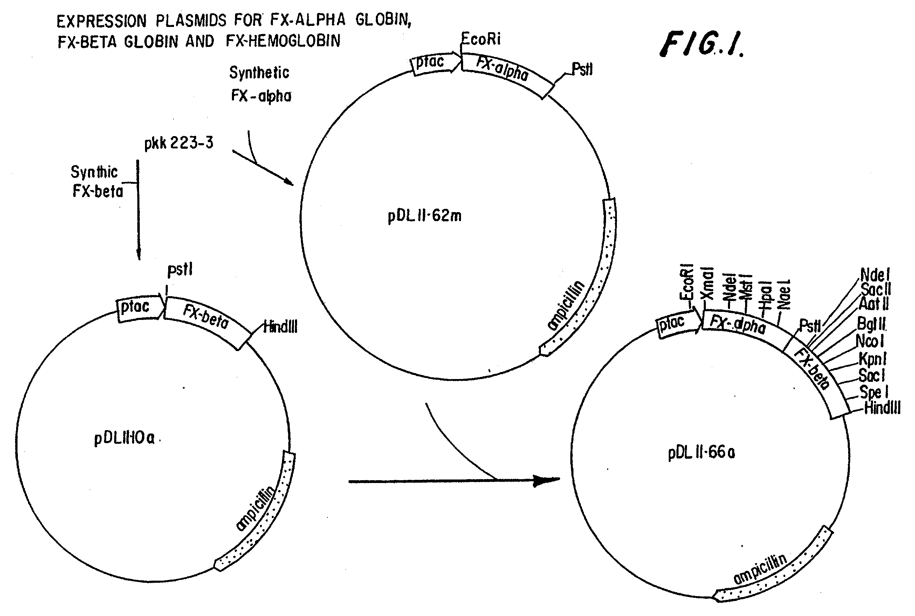

The appropriate sequences were excised from the M13mp19 vector and cloned into the pKK-223-3 (Pharmacia/LKB, Piscataway, NJ)) expression vector under control of the Tac promoter (Brosius, J, and Holy, A. Proc. Nat. Acad. Sci., USA 1984, 81, 6929-6933). A DNA sequence encoding alpha globin was removed by cutting with EcoRI and PstI. The globin containing fragment was gel purified and ligated into EcoRI and PstI double cut, gel purified pKK-223-3 using methods described above. E.coli JM109 cells were transformed with the ligation reaction containing the desired FX-alpha globin sequence (pDL II-62m) (Figure 1) and selected for by growth on 2xYT media containing ampicillin (100 µg/mL). Individual clones were screened for the presence of the desired insert (yielding pDL II-62m) by alkaline-SDS purification of plasmids and restriction analysis with the enzymes EcoRI and PstI using the methods described above. Cloning of the FX-beta globin sequence into the pKK-223-3 expression vector, yielding pDL II-10a (Figure 1) was done analogously except that restriction analysis and cloning was done with PstI and HindIII.

### Example 2: Separate Expression of Synthetic FX-Alpha and FX-Beta Globin

To assess the expression of the individual FX-globin gene products, E.coli JM109 clones transformed with either pDL II-62m or pDL II-10a were inoculated into 2mL of TB media containing ampicillin (100µg/mL). The inoculum was grown at 37°C for 3-4 hours, then divided into two 1mL aliquots. One of the aliquots was induced by the addition of IPTG (1mM, final) and grown for an additional 3-4 hours. The cells were collected by centrifugation, resuspended in 0.5mL SDS-PAGE loading buffer and heated at 85°C for 10 minutes. The total cell protein mixture was electrophoresed on 15% SDS-polyacrylamide gels using authentic hemoglobin as a molecular weight standard.

### Example 3: Polycistronic Coexpression of FX-alpha and FX-beta Globin Gene Products from the same Operon (PDLII-66a) And Conversion of FX-hemoglobin to Hemoglobin

To achieve coexpression of FX-alpha and FX-beta globins from a single polycistronic operon, the FX-beta globin sequence from pDL II-10a was excised with HindIII and PstI, gel purified, and ligated into PstI/HindIII cut and gel purified pDL II-62m. Ligation and transformation conditions were identical to those described above. Note that each globin cistron was preceeded by an "introduction" cistron as previously described, so that the entire Tac promoter driven operon had four cistrons. Clones were individually examined for the presence of both FX-alpha and FX-beta globin genes by digestion of plasmids with EcoRI and separation of fragments by electrophoresis through 0.8% agarose. Plasmids containing both genes (pDL II-66a, Figure 1) produced a fragment of approximately 1.0kb following EcoRI digestion.

Clones containing pDL II-66a were grown in 2mL of TB media containing ampicillin (100µg/mL) for 4 hours at 37°. The culture was divided into two 1mL aliquots, one of which was induced with 1mM IPTG. Incubation was continued for 4 hours. Total cell protein extracts for both the uninduced and induced clones were examined by SDS-PAGE electrophoresis to confirm the coexpression of both FX-alpha and FX-beta globin.

To determine if the coexpression of both gene products resulted in the formation of tetrameric FX-alpha globin₂ FX-beta globin₂ protein, the following experiments were performed. Two liters of TB medium containing ampicillin (100µg/ml) was inoculated with 20mL of an overnight culture of an FX-alpha/FX-beta expressing E. coli clone and grown to an optical density at 600nm (OD₆₀₀) of 2.1 at 37°C. The culture was induced with IPTG (2.5mM final concentration) and grown to an OD₆₀₀ of 3.5.

The cells (40gm) were collected by centrifugation at 10,000xg and suspended in 80mL of lysis buffer (50mM Tris-HCl, pH 8.0, 25% sucrose, 1mM EDTA). Ten milliliters of lysozyme solution (18mg/ml in lysis buffer) was added and the mixture incubated on ice for 30 min. MgCl₂, MnCl₂ and DNAse I (Sigma, St. Louis, MO) were added to final concentrations of 10mM, 1mM and 10µg/mL, respectively. The cells were incubated at room temperature for 1 hour and an equal volume of a solution containing 1% one percent deoxycholic acid, 1% Nonidet P-40, 20mM Tris-HCl pH 7.5, 2mM EDTA was added to the lysate.

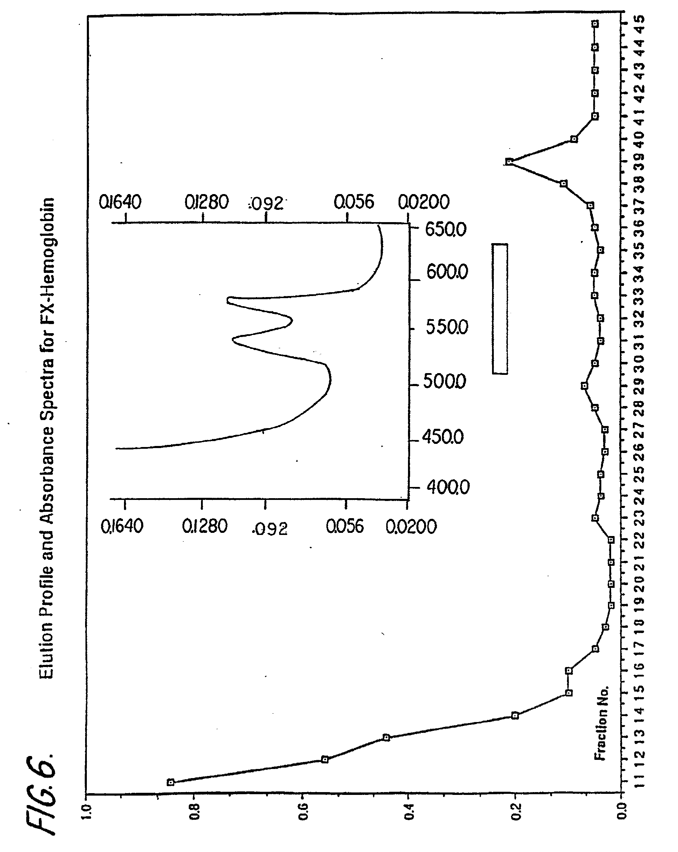

Particulate material was removed by centrifugation at 10,000 x g for 10 min. The supernatant (-200mL) was bubbled with carbon monoxide for 5 min and dialyzed overnight against 4 liters of 10mM NaPO₄ buffer, pH 6.0. The cell-free extract was clarified by centrifugation at 10,000xg for 10 min. To the supernatant was added 20g DE-52 (Whatman, U.K.). The pH of the suspension was adjusted to 7.0 and the ion exchange resin was removed by centrifugation. The pH of the supernatant was readjusted to 6.0 and the supernant was loaded onto a CM-cellulose column (2.5 x 15cm) equilibrated in 10mM NaPO₄, pH 6.0 at 4°C. The column was washed with two bed volumes of 10mM NaPO₄, pH 6.0 followed by a linear gradient of 10mM NaPO₄, pH 6.9 to 20mM NaPO₄, pH 9.0 (400mL total volume). Fractions 36 to 42 contained a red solution and were combined; an aliquot of this solution was scanned from 650nm to 400nm revealing a spectrum identical to that for carboxyhemoglobin (Figure 6). An aliquot of the same peak was analyzed by SDS-PAGE electrophoresis with hemoglobin as molecular weight standard and was found to contain two protein bands of approximate MW 15,500 and 16,200. As expected, these bands migrated at a slightly slower rate than authentic hemoglobin (alpha MW=15,100; beta MW=15,850) presumably due to the five amino acid extension of the Factor X recognition sequence. On this basis the material was designated FX-hemoglobin.

FX-Hemoglobin (2.0mg) was digested at room temperature for 2 hours in 3mL of 20mM HEPES, pH 7.4, 0.1 M NaCl, 10mM CaCl₂ containing 2mg of trypsin (Sigma, St. Louis, MO., 10,000 units/mg). SDS-/PAGE electrophoresis confirmed the conversion of FX-hemoglobin to material that comigrates with native hemoglobin.

### Example 3A: Distribution of FX-Globin Products from E.coli Expression

One hundred milliliter cultures of E. coli clones expressing Fx-alpha globin (plasmid pDL II-62m), Fx-beta globin (plasmid pDL II-10a) and Fx-hemoglobin (plasmid pDL II-66a) were started with 1 ml inocula of overnight cultures. After growth for 4 hours, protein expression was induced by addition of IPTG to 1 mM final concentration. Incubation was continued for an additional 3 hours. The cells were collected by centrifugation, weighed, and lysed as described above except that DNAase treatment was done for 30 minutes on ice. The samples were centrifuged at 5000xg, 10 min and the supernatants (representing the soluble protein fraction) brought to 2 ml final volume with H₂O and frozen at -80°C. The pellets (representing the insoluble protein or inclusion body fraction) were washed twice in 5 mL 0.5% Triton X-100, 1 mM EDTA, resuspended in 2 ml H₂O and frozen at -80°C.

Analysis of protein distribution was accomplished by SDS-PAGE and Western blotting. The primary antibody was rabbit anti-human hemoglobin IgG. The western blotting protocol was according to the manufacturer's recommendations (Proto Blot Western Blot AP system, Promega Corp., Madison, WI). Samples of soluble and insoluble protein representing 60 µg of wet cell weight were analyzed from the FX-alpha and FX-beta expressing clones. Due to the greater level of expression in the FX-hemoglobin clone, material representing only 15 µg of wet cell weight was analyzed.

As seen in Table 11 the distribution of the proteins varied. Fx-alpha globin was detectable only in the soluble fraction while FX-beta globin partitioned between the insoluble and soluble fractions of the cel.. FX-Hgb, which stabilizes each separate subunit, was found only in the soluble fraction, and at a concentration at least 2.6 times that of the individually expressed subunits. These results indicate that FX-beta globin is totally soluble only when allowed to assemble with FX-alpha globin.

### Example 4: Construction And Expression of Mutant FX-Alpha/FX-Beta Tetrameric Hemoglobin Expression Vector

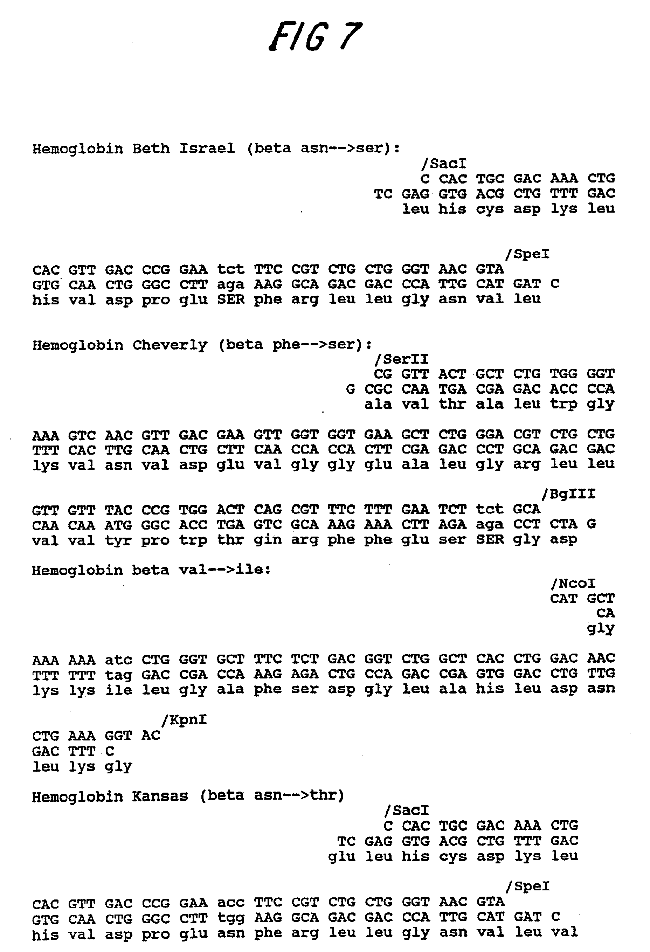

Hemoglobin Beth Israel: pDL II-10a was digested with restriction enzymes SacI and SpeI, gel purified, and isolated by electro-elution. Oligonucleotides incorporating the appropriate codon change for Hemoglobin Beth Israel (beta¹⁰² asn-->ser) (Figure 7) were synthesized as previously described above to bridge the SacI to SpeI restriction sites. Purification and quantitation of the individual oligonucleotides was as previously described. The complementary oligonucleotides were annealed by heating to 95°C for 10 min. followed by slow cooling to room temperature over a 2 hour period. An aliquot of the annealed mixture was then combined with the SacI/SpeI digested, gel purified pDL II-10a plasmid at molar ratios similar to those used for the initial FX-alpha and FX-beta cloning. T₄ DNA ligase (2U) was added and the mixture incubated for 1 hour at room temperature. E.coli JM109 was transformed with this ligation mixture as previously described. Individual clones were isolated, and plasmids purified and sequenced using primer beta-1 (vida supra). Plasmid sequencing was done with a Sequenase kit (United States Biochemical Corp., Cleveland, OH) following the protocol supplied by the manufacturer. The appropriate mutated beta globin sequence was then excised with HindIII and PstI, gel purified, and cloned into pDL II-62m as described above.

Other hemoglobin mutants: The synthetic genes encoding Hemoglobin Cheverly (beta⁴⁵ phe-->ser) Hemoglobin Providence/MSR (beta⁸² lys-->asp) and Hemoglobin beta⁶⁷ val-->ile and Hemoglobin Kansas (beta ¹⁰² asn-->thr) were prepared similarly except with synthetic oligonucleotides spanning the SacII-->BglII, SalI --> SpeI, NcoI-->KpnI and SacI -->SpeI restriction sites respectively (Figure 7). Synthesis of the mutant oligonucleotides, restriction enzyme digestion, gel purification, and ligation conditions were identical to those used for Hemoglobin Beth Israel. All mutations were first cloned into plasmid pDL II-10a, appropriate clones were sequenced, and the mutated beta globin gene was subcloned into PstI and HindIII digested pDL II-66a. Plasmid sequencing was accomplished as described previously. E. coli cells were transformed, cultured, and induced as previously described. FX-hemoglobin mutants were purified by the method of Example 3. Oxygen binding of purified hemoglobin mutants is shown in Table 9.

### Example 5: Production of Synthetic Met-Alpha/Met-Beta Hemoglobin

### Construction of Des-FX Alpha and Des-FX Beta Globin Genes

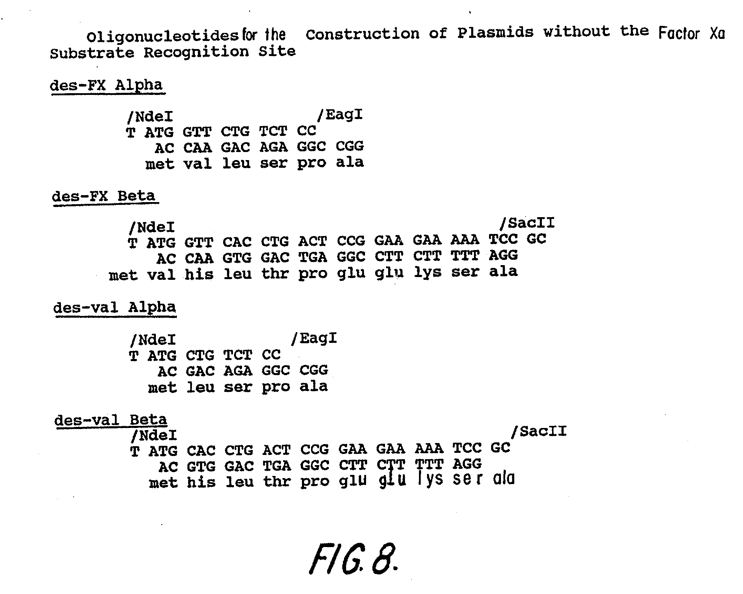

We established that E.coli could produce tetrameric fusion-hemoglobin. Elimination of the DNA sequences coding for the Factor Xₐ substrate recognition site on the N-terminal end of each peptide should result in production of synthetic (FX free; "des-FX") hemoglobin containing the N-terminal methionine as its only extra residue. pDL II-62m was digested with EcoRI and PstI to excise the sequence containing the FX-alpha globin gene. The FX-alpha globin gene was then gel purified. pGEM-1 (Promega Corp.) was linearized with EcoRI and PstI, gel purified, and the FX-alpha globin gene ligated into the plasmid as described previously. Clones (FX-alpha pGEM) containing the FX-alpha globin gene in pGEM-1 were identified by digestion of purified plasmids with EcoRI and PstI followed by agarose gel electrophoretic analysis. FX-Alpha pGEM was digested with NdeI and EagI to remove the Factor Xₐ coding sequence (Figure 5). Oligonucleotides containing the DNA sequence encoding native alpha globin were synthesized with ends compatible to NdeI and EagI (Figure 8) restriction sites. After synthesis, the oligoncleotides were purified, annealed, and ligated as described above. The sequence of pGEM-desFX-alpha (pDL II-83a) was confirmed by dideoxy-sequencing of the plasmid using the T₇ promoter primer (Promega Corp., Madison, WI). A clone containing the correct sequence was then digested with EcoRI and PstI. The des-FX alpha globin gene was gel purified, cloned into EcoRI/PstI digested, gel purified pKK-223-3 to generate pDL II-86c. E.coli strain JM109 was transformed with the ligation mixtures and expression of des-FX alpha globin by individual clones was determined by analysis of total cell protein extracts of induced culture inocula (see above). Des-FX alpha globin, in contrast to FX-alpha globin, co-migrates with authentic alpha globin on SDS-PAGE.

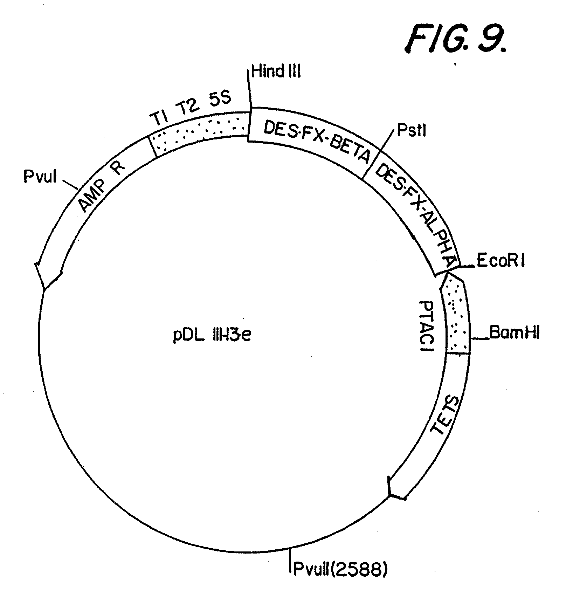

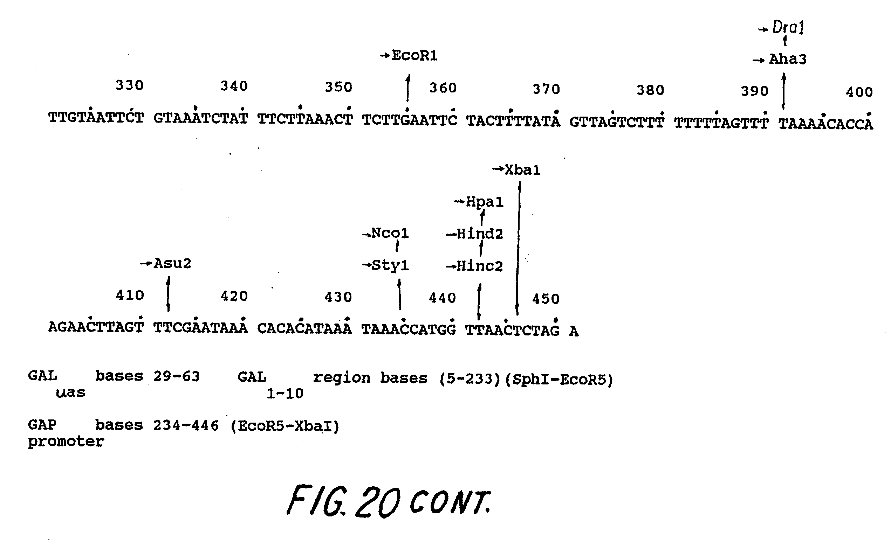

The des-FX beta globin sequence was prepared in an analogous fashion using the FX-beta globin gene excised from pDL II-10a with PstI and HindIII. This gel purified beta globin sequence was then ligated into pGEM-1 that had been digested with the same two enzymes. A pGEM-1 plasmid containing the FX-beta globin gene was digested with NdeI and SacII, gel purified, and used for construction of the des-FX beta globin gene. The oligonucleotides conferring the desired sequence (Figure 20) were synthesized, purified, annealed, and ligated into NdeI, SacII cut pGEM FX-beta to form pGEM-des-FX beta (pDL III-6f) as described above. After confirming that the sequence was correct the des-FX beta gene was removed with PstI and HindIII and gel purified. Des-FX beta was then ligated into the des-FX alpha-containing plasmid pDL II-86c using the PstI/HindIII sites. Clones containing both des-FX alpha and beta globin genes (pDL III-13e) (Figure 9) were confirmed by EcoRI digestion of purified plasmids (see above), and screened for expression by comparison of IPTG induced and non-induced cultures. Des-FX hemoglobin co-migrates with native hemoglobin on SDS-PAGE.

### Characterization of Met-Hgb (Des-FX Hgb)

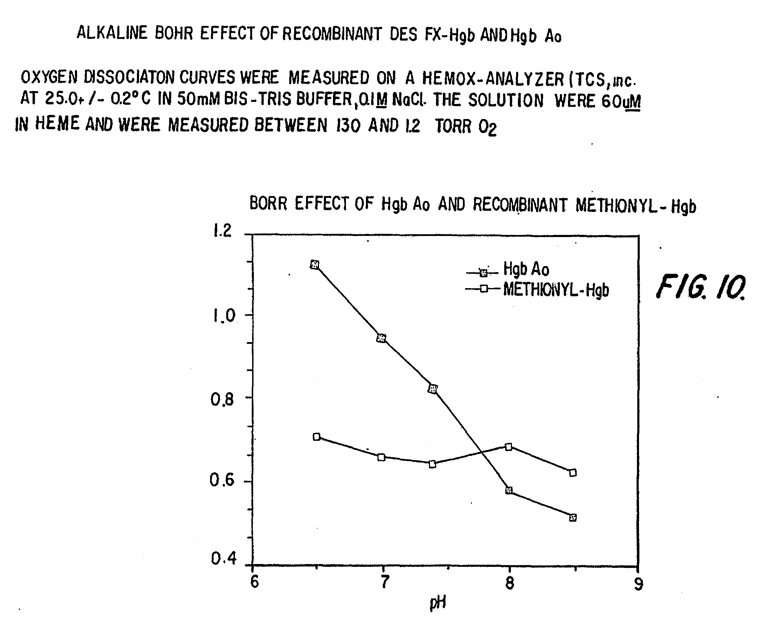

Recombinant methionyl-Hgb has different reactivities than Hgb Ao in the presence of chloride and phosphate ions (Table 10) and with changes in hydrogen ion concentration (Figure 10). The reason for this is thought to be the additional amino acid, methionine, on the N-termini of both globins. The N-terminal amino group of the alpha chain is important for the change in P₅₀ by phosphate ion. Displacement of the N-terminal amino group in space or changing its electronic state by the addition of the methionine can alter these effects.

The increase in P₅₀ seen in the presence of inositol hexaphosphate ion is irrelevant for any Hgb found in solution because the concentration of monophosphate ion found in plasma is not enough to significantly increase the P₅₀. There is no inositol hexaphosphate ion found in plasma. The increase in P₅₀ needed for Hgb in solution to effectively off-load oxygen can be best attained by incorporating mutations to make Hgb lower in affinity for oxygen.

The magnitude of change in P₅₀ with respect to chloride ion is not important physiologically. The effect, which is interesting in terms of its biochemical mechanism, does not add a significant amount of oxygen off-loading capacity to Hgb in solution. Some animal Hgb have extremely small chloride effects. Again, the chloride effect is thought to be transmitted by the alpha globin N-terminal amino group; with methionyl-Hgb that effect could be changed because of the stearic change or because the pKa of the methionine amino group is different than that of valine.

The P₅₀ of Hgb is normally most dramatically changed by hydrogen ion concentration. The so-called "Bohr effect" is also thought to, in part, involve the N-terminal amino group of the alpha globin. Numerous mutant human Hgb molecules have been shown to have altered Bohr effect changes without any physiological deficiency. There is an advantage in having a limited Bohr effect, as well as phosphate and chloride effects, in a Hgb molecule to be used in solution for a number of different medical and biochemical applications. In terms of the Bohr effect, there are several applications for methionyl-Hgb where it could be used in the more alkaline pH range, e.g., tissue culture, organ perfusion. Figure 10 depicts a preliminary experiment which indicated that the P₅₀ is actually greater for methionyl-Hgb than for Hgb Ao at greater than pH 7.8. It will be noted from Figure 10 that the slope of the P₅₀ to pH plot for Met-Hgb is shallower than that for Hgb Aₒ, i.e., the Bohr effect is smaller. Subsequent experiments suggest that the difference in Bohr effect between Met-Hgb and Hgb Aₒ is smaller than that shown in Figure 10.

The main advantage for using a Hgb molecule with fixed changes in P₅₀ relative to pH, chloride and phosphate, is that the practitioner will know the oxygen off-loading capacity of the formulation without regard for the specific conditions of its use.

### Example 6: Synthesis of Synthetic Hemoglobin (Des-Val Hgb) of Native Size

### Construction of Des-val-Alpha (pDL II-91f) and Des-Val-Beta (pDL III-1a) Globin Genes

DNA sequences encoding the globin genes in which the N-terminal valine codon in each gene is replaced by an ATG (methionine) codon were constructed in a manner analogous to the des-FX clones except that the oligonucleotides inserted (Figure 8) were ones encoding the amino acid sequences "met-leu..." and "met-his..." for alpha and beta globin genes, respectively. Following confirmation of correct sequence for both des-val alpha (pDL II-91f) and des-val beta (pDL II-95a) genes in pGEM-1, the des-val alpha globin gene was cloned into the EcoRI/PstI cut pKK-223 (see above) to create pDL III-1a. The des-val beta globin gene from pDL II-95a was then cloned into pDL III-1a using the PstI/HindIII restriction sites.

More specifically, the des-val alpha transfer vector was prepared from plasmid pDL II-62m as follows. Plasmid pDL II-62m was digested with EcoRI and PstI to excise the fragment containing the FX alpha globin gene. The FX alpha globin gene was then gel purified. The plasmid pGEM-1 (Promega Corp.) was linearized with EcoRI and PstI, gel purified, and the FX alpha globin gene ligated into the plasmid as described previously. It was necessary to subclone into pGEM-1 because redundant restriction sites in pKK 223-3 prohibited the removal of the FX coding sequence directly from the individual FX-alpha and FX-beta globin genes. Clones containing the FX alpha globin gene in pGEM-1 were identified by digestion of purified plasmids with EcoRI and PstI followed by agarose gel electrophoretic analysis. FX alpha pGEM-1 was digested with NdeI and EagI to remove the FX alpha coding sequence and oligonucleotides containing DNA sequences coding for native alpha globin in which the N-terminal valine is replaced by a methionine were synthesized with ends compatible to NdeI and EagI restriction sites. After synthesis, oligonucleotides were purified, annealed and ligated as described above. The sequence of pGEM des-val alpha (plasmid pDL II-91f) was confirmed by dideodoxy sequencing of the plasmid using the T₇ primer (Promega Corp., Madison, Wisconsin). A clone containing the correct sequence for des-val alpha (pDL II-91f) was then digested with EcoRI and PstI. The des-val alpha globin gene was gel purified, and ligated into EcoRI/PstI digested, gel purified, pKK-223-3 to generate plasmid pDL III-1a.

The des-val beta globin transfer vector was prepared in an analogous fashion using the FX beta globin gene of plasmid pDL II-10a. FX-beta was excised from pDL II-10a with PstI and HindI. This gel purified beta globin sequence was then ligated into pGEM-1 that had been digested with the same two enzymes. A pGEM clone containing the FX beta globin gene was digested with NdeI and SacII, gel purified, and used for construction of the des-val beta globin gene. The oligonucleotides encoding the desired sequence for des-val beta were synthesized, purified, annealed, and ligated into NdeI, SacII cut, pGEM FX-beta to form pGEM des-val beta (plasmid pDL II-95a). After confirming that the sequence was correct for des-val beta globin, the gene was removed with PstI and HindIII and gel purified.

### Preparation of pDL III-14c end III-38b (des-Val-alpha/des Val beta polycistronic Gene Clones)

Plasmid PDL III-1a containing the Des-Val alpha globin gene was digested with PstI and HindIII and gel purified. The Des-Val beta globin gene was removed from pDL II-95a using the same method. Following ligation and transformation, individual clones containing the Des-Val alpha/des-val beta globin coexpressing plasmid pDL III-14c were analyzed for Des-Val hgb production by IPTG induction and SDS-PAGE.

Plasmid pDL III-38b which contains the Des-Val beta globin gene 5' to the Des-Val alpha globin gene was then constructed and analyzed.

Plasmid pDL III-1a containing the des-val alpha globin gene was linearized with the restriction enzyme SmaI. The plasmid was then treated with bacterial alkaline phosphatase to remove the 5'-phosphate groups, phenol extracted, ethanol precipitated, and resuspended in TE buffer as above. A pGEM-1 clone containing the des-val beta gene was digested with HindIII, phenol extracted, ethanol precipitated and resuspended in a ligation mixture containing a 50:1 molar ratio of a HindIII-SmaI linker to plasmid. This ligation mixture was then digested with SmaI and the beta globin fragment now containing SmaI restriction sites on both the 5'- and 3'- ends was gel purified and added to a ligation reaction containing the linearized form of plasmid pDL III-1a, above, thus obtaining plasmid pDL III-38b. The orientation of the alpha and beta globin genes in pDL III-14c and pDL III-38b was confirmed by restriction analysis.

Cells of E. coli strain JM109 were transformed with pDL III-14c or pDL III-38b and grown in 2xYT media containing ampicillin. Colonies were induced with IPTG as above. Individual clones were analyzed for their ability to produce des-val alpha and des-val beta globin polypeptides by SDS-PAGE and Western blotting. There was no appreciable difference between expression of immunoreactive des-val alpha or des-val beta globins from the alpha-->beta orientation (pDL III-14c) or the beta-->alpha orientation (pDL III-38b).

### Example 7: Analysis and Comparison of the Functional Characteristics of Recombinant Des-FX and Des-Val Hemoglobins

JM109 cells expressing either des-FX hemoglobin (dFX-hgb) or des-Val hemoglobin (dV-hgb) were grown to an OD600 of 15 in a 10 liter fermenter and then induced by the addition of 300µm IPTG. Induction period was for 6 hrs. Cells were harvested by centrifugation and frozen at -80°C until processed.

For purification of hgb approximately 200g of cells were resuspended in 350mL of 50mM sodium phosphate (NaPi) buffer, pH 7.0 containing 200 units of aprotinin/mL and 20µg/ml DNAase 1. Cells were then lysed by the addition of 1mg/mL of lysozyme and 4 passages through a Dynomill. Cellular debris was removed by centrifugation at 10,000 rpm in a Beckman JA14 rotor at 4°C for 40 min. The supernatant was added to 200mL of a hemoglobin-binding resin equilibrated with 10mM NaPi, pH 7.0. The pH was adjusted to 7.0 with 10M NaOH or concentrated phosphoric acid. The resin was then loaded into a 5x30 cm chromatography column and allowed to settled. The column was then washed with 2.5 column volumes of 10mM NaPi, pH 7.0 containing 100 units/mL aprotinin. Hgb was eluted from the column in 20mM Tris-HCl, pH 7.5 containing 100 units/mL aprotinin. This partially purified hgb was then 0.2 micron filtered and loaded onto a 1.6x10 cm Mono-Q anion exchange column equilibrated with 20mM Tris-HCl, pH 8.0. Hgb was eluted using a linear gradient of 0 to 0.4M NaCl in 20 mM Tris-HCl, pH 8.0. The material was then loaded onto a 1.6x10 cm Mono-S cation exchange column. Hemoglobin was eluted with a linear gradient of 10mM NaPi, pH 7.0 to 10mM NaPi, pH 8.5, 160mM NaCl. The major peak of hgb was collected, concentrated to approximately 100mg/mL. and used for analysis.

Functionality of the recombinant hgb was evaluated using a Hemox analyzer at 25°C in 50mM HEPES, pH 7.4 containing 0.1M Cl⁻ . The following oxygen binding data were obtained:

| SAMPLE | P₅₀ | N |
|---|---|---|
| Ao | 4.03 | 2.7 |
| dFX-hgb | 3.43 | 2.6 |
| dV-hgb | 7.04 | 2.8 |

Of significance in these data are the following:
1) The addition of an extra amino acid, methionine, on the N-termini of alpha and beta globins (dFX-hgb) appears to reduce slightly the P₅₀ of the molecule but has little effect upon cooperativity (N).
2) Replacement of the N-terminal valines of alpha and beta globins with methionine (dV-hgb) increases the P₅₀ of the molecule but has little effect upon the cooperativity (N).

### Example 8: Polycistronic Co-expression of Des-Val-Alpha/Alpha (Di-Alpha) and Des-Val Beta Globin

The overall synthetic plan for the preparation of a plasmid (pDL III-47a) which co-expresses di-alpha globin and beta globin is given below. The starting materials are the commercially available transfer vectors M13mp19-RF, pKK 223-3 and pGEM-1, and the synthetic oligonucleotides described in the examples.

For convenience, our manipulations began with plasmids, pDL II-62m and pDL II-10a. Plasmid pDL II-62m was obtained by cloning the "FX alpha globin" gene into pKK 223-3 downstream of the Tac promoter. Plasmid pDL II-10a was prepared by an analogous insertion of the "FX beta-globin" gene.

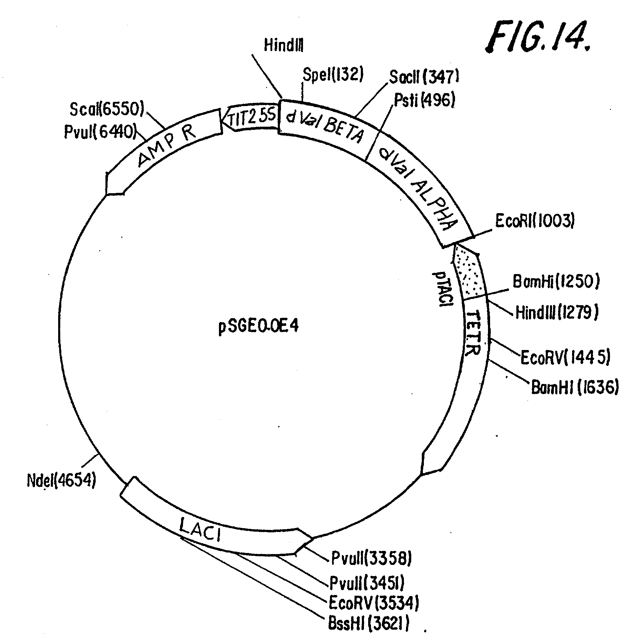

As set forth in greater detail in Figure 14, the "FX alpha globin" operon encodes two cistrons, the first expressing an octapeptide "loader", and the second, alpha globin preceded by Met-Ile-Glu-Gly-Arg. The latter four amino acids constitute a recognition site for Factor X cleavage activity. The "FX beta globin" operon is similarly constructed.

Since the Factor X recognition site was not needed here, the genetic material was manipulated to excise the "FX" codons. (This could have been avoided by synthesizing the desired di-alpha globin and des-val-beta globin genes directly rather than using the FX-alpha and FX-beta genes of pDL II-62m and pDLII-10a.) The FX-alpha globin gene cassette was excised and cloned into pGEM-1 to obtain pGEM FX-alpha. Similarly, the FX-beta gene of pDL II-10a was transferred to pGEM-1 to obtain pGEM FX-beta.

The recognition site (FX)-encoding sequence could now be removed from pGEM FX-alpha and pGEM FX-beta to obtain pDL II-91f and pDL II-95a, respectively. The des-val alpha globin gene of pDL II-91f was recloned into pKK 223-3 to generate pDL III-1a, the gene being operably linked to the Tac promoter of pKK-223-3. The des-val beta globin gene of pDL II-95a was purified and inserted downstream of the des-val alpha globin gene of pDL III-la to form a single transcriptional unit which would encode a polycistronic alpha globin/beta globin mRNA, see pDL III-14c. Finally, a synthetic oligonucleotide comprising the desired di-alpha linker encoding sequence and another copy of the alpha globin gene was inserted into pDL III-14c to create pDL III-47a, wherein a Tac promoter controls transcription of a di-alpha globin gene and a des-val beta globin gene.

### Preparation of pDL III-47a (di alpha/beta globin clone)

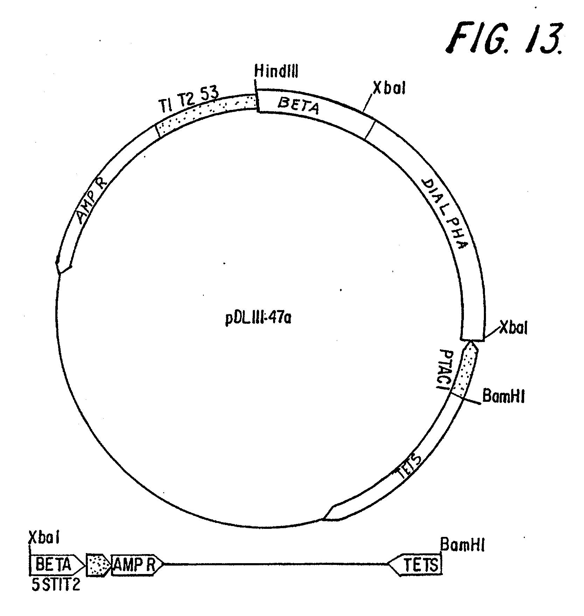

The EagI and PstI restriction fragment containing most of the alpha globin gene from the plasmid pDL II-91f was gel purified and ligated to a synthetic DNA linker containing the sequence from the BstBI site of the alpha globin gene to its carboxyl terminus, the glycine linker, and the amino terminus of alpha globin to the EagI site (Figure 12). After digesting this ligation mixture with Pst I, the resulting fragment was cloned into BstBI/PstI-cut pDL III-14C to create plasmid pDL III-47a (Figure 13).

### Expression of Di-alpha/Beta Hemoglobin

Individual E.coli clones were analyzed by Western blotting for production of dimeric alpha globin protein in combination with monomeric beta globin. Appropriate plasmid construction was confirmed by digestion with EcoRI and 0.8% agarose gel electrophoresis. The EcoRI fragment present in the di-alpha constructs is approximately 1450 bp.

Expression of genetically crosslinked hemoglobin was accomplished using the IPTG induction protocol and S-sepharose purification of recombinant hemoglobin. E.coli cells (400 ml) were grown to an OD₆₀₀ of 3.0 and induced with 1mM IPTG. The cells were allowed to continue to grow for another 4 hours and then harvested by centrifugation. The cell pellet was resuspended in the 10mM sodium phosphate, pH 6.0 containing 1mM benzamidine, 1mM EDTA and 0.1% Triton-X100. The cell suspension was then sonicated, centrifuged at 15,000xg for 15 minutes, and the supernatant loaded on to an S-Sepharose column equilibrated with 10mM sodium phosphate pH 6.0. After the sample was loaded on the column, the column was washed with 10 bed volumes of 10mM sodium phosphate pH 6.8. The dialpha hemoglobin was eluted from the column with 10mM sodium phosphate, pH 7.4, 30mM NaCl. Confirmation of hemoglobin production was accomplished by visible light spectroscopy, SDS-PAGE, and Western blot analysis of purified material.

### Example 9: Preparation of Di-AlPha Hemoglobin Low Affinity Mutants

In order to reduce the oxygen affinity of recombinant di-alpha hemoglobin, several mutations were introduced into the beta globin polypeptides using synthetic oligonucleotides. The restriction sites used to incorporate these mutants are shown in Table 3. For insertion of the Nagai (beta Val 67->Ile) and Arg-Nagai (also beta Lys 82-> Arg) mutations, the des-Val-beta plasmid pDL II-95a was digested with the restriction enzymes NcoI and KpnI and gel purified. Oligonucelotides spanning these two restriction sites and containing the appropriate codon changes were synthesized, purified, annealed and ligated into the gel purified plasmid. Following confirmation of correct sequence, the mutant des-Val-beta globin gene was excised with PstI, HindIII, gel purified, and cloned into plasmid pDL III-47a. The beta globin gene containing the Kansas mutation (beta Asn 102=>Thr) was similarly constructed using SacI and SpeI restriction sites. Mutated codons for all of these beta globin mutations are shown in lower case letters in Table 3.

### Example 10: Characterization of Di-Alpha Hemoglobins

### Oxygen Binding

Oxygen binding measurements were made at 37°C in a Hemox Analyzer (Southampton, PA). The solutions were 50mM Bis-Tris, pH 7.4, 0.1M NaCl and 60uM heme equivalents of di-alpha hemoglobin. The solutions were measured between 120 and 1.5 torr oxygen pressure. P₅₀ values are given in Table 4.

### In Vivo Half Life

Di-Alpha Hgb (wild type) containing a gly-gly linker between alpha₁ and alpha₂ was prepared as described previously. The protein was formulated in 20mM NaPO₄, pH 7.4 at a concentration of 95 mg/ml. Di-alpha Hgb was infused into male Sprague-Dawley rats 388 - 426 gm) at a dose of 875 mg/kg through a central venous catheter over 20-30 sec. Samples of blood were drawn at 2, 30, 80, 90, 120, 150, 180, 210, and 240 min. into heparinized vials. The blood was centrifuged to remove red blood cells and the plasma hemoglobin was assayed by absorbance at 540 nm. The percent hemoglobin remaining versus time was determined by comparison to the 2 min time point which was assumed to be a homogeneously mixed sample. The same experiment was repeated with non-crosslinked des-val Hgb at a concentration of 100 mg/ml. The data were averaged for each sample and plotted as percent Hgb remaining against time after infusion. The measured half-lives were 205 min and 104 min respectively for di-alpha Hgb and des-val Hgb, respectively.

### Example 11: Co-expression of Wild Type Des-Val Alpha Globin and Di-Alpha Globin with Des-Val Beta Globin Containing the Presbyterian Mutation

Plasmid pSGE0.0-E4 is shown in Figure 14 and contains the following modifications as compared to other expression vectors derived from plasmid pKK223-3.
1) The plasmid now contains a functional tetracycline resistance gene.
2) The lacI gene which encodes for the lac repressor protein has been incorporated into the plasmid. The lac repressor protein represses the TAC promoter until induction with IPTG. The repressor gene was inserted into the plasmid to permit transformation of E. coli cell lines which do not have endogenous lac repressor genes.

The desVal beta globin gene containing the Presbyterian mutation was constructed by insertion of a complementary pair of synthetic oligonucleotides into the Sacl to Spel restriction of sites of pSGE0. 0-E4.

The following oligonucleotides (Pres-A and Pres-B) were used to construct the Presbyterian mutation in dVal-beta globin.

Pres-A   5'   CCACTGCGACAAACTGCACGTTGACCCGG (Continued below)

Pres-B    3'TCGAGGTGACGCTGTTTGACGTGCAACTGGGCC (Continued below)
            SacI

Pres-A    AAAACTTCCGTCTGCTGGGTaaaGTA    3'

Pres-B    TTTTGAAGGCAGACGACCCAtttCATGATC 5'
                                   SpeI

Following digestion with the two restriction enzymes the plasmid was gel purified to remove the wild type encoding DNA fragment. The annealed oligonucleotides were then ligated into the plasmid. Following transformation of JM109 cells individual colonies were selected and analyzed for production of alpha and beta globins by IPTG induction and SDS-PAGE. Dideoxynucleotide sequencing was used to confirm the presence of the Presbyterian mutation.

The mutant hemoglobin was produced, purified, and analyzed as described in Example 7 above. The following results were obtained:

| SAMPLE | P₅₀ | N |
|---|---|---|
| Ao | 4.03 | 2.7 |
| dV-hgb | 7.04 | 2.8 |
| dV-hgbPres | 33.0 | 2.5 |

If should be noted that the Presbyterian mutation, which results in the change of beta asparagine 108 to lysine, decreases the affinity of the molecule for oxygen by 10 fold but does not affect the cooperativity of the molecule.

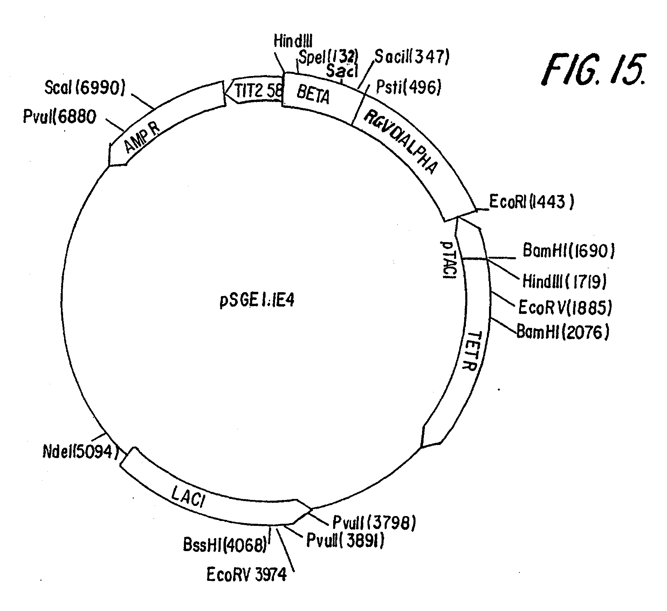

The Presbyterian mutation has also been co- expressed with di-alpha globin containing a single glycine linker utilizing plasmid pSGE1.1-E4 (Figure 15). The result shown below indicate that the joining of the carboxy terminus of alpha 1 to the amino terminus of alpha 2 has no effect on oxygen binding and cooperativity.

| SAMPLE | P₅₀ | N |
|---|---|---|
| dV-hgbPres | 33.0 | 2.5 |
| dialpha/Pres | 33.0 | 2.4 |

### Example 12: Construction of a Two Promoter System for the Co-expression of Di-Alpha Globin and Beta Globin

In this example, a di-alpha globin gene is operably linked to one promoter and a beta globin gene to a second promoter, but both genes reside on the same vector. Compare Examples 16 and 17, infra.

Oligonucleotides (see below) encoding the sequence of the complementary strands of the TAC promoter (syn pTAC) and appropriate restriction enzyme sites were synthesized, gel purified, and annealed. Notice that the sequence complementary to the Xbal restriction enzyme site was designed to eliminate this restriction site when ligated into an authentic Xbal site. This was done to facilitate future manipulation of syn pTAC.

Sequence of syn pTAC

BamHI PstI
5'GATCCTGCAGAGCTGTTGACAATTAATCATCGGCTCGTATAATGTGTGG (Cont'd)
3' GACGTCTCGACAACTGTTAATTAGTAGCCGAGCATATTACACACC (Cont'd)

XbaI COMP
AATTGTGAGCGGATAACAATTTCACAC 3'
TTAACACTCGCCTATTGTTAAAGTGTGGATC 5'

Plasmid pDL III-47a (Figure 13) was digested with the restriction enzymes BamHl and Xbal, and the plasmid, now containing as an insert only part of the beta globin gene from the Xbal site to the HindIII site (Figure 13a), was gel purified.

Syn pTAC was then ligated into the plasmid to create plasmid pDL IV-64a and JM109 cells were transformed. Individual transformants were isolated and analyzed by IPTG induction and SDS-PAGE to confirm the presence of a functional TAC promoter.

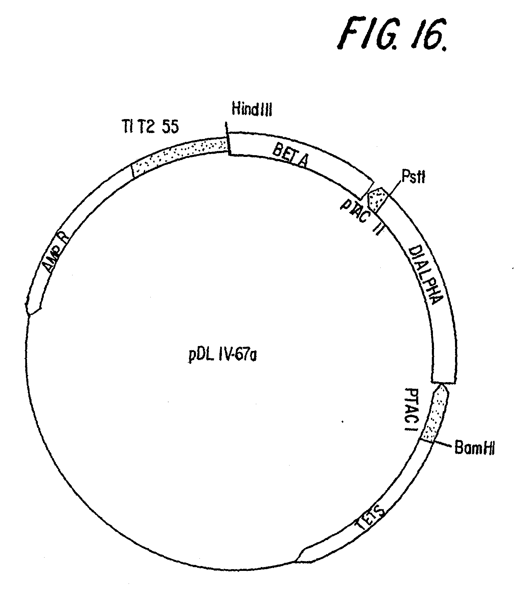

Plasmid pDL III-47a was digested with the restriction enzymes Pstl and HindIII and gel purified to remove the beta globin coding sequences. Plasmid pDL IV-64a was digested with these same enzymes and the fragment encoding syn pTAC/beta gel purified. Ligation of the syn pTAC/beta fragment into the Pstl/HindIII digested pDL III-47a created plasmid pDL IV-67a (Figure 16). Individual transformants were screened for production of di-alpha and beta by IPTG induction and SDS-PAGE.

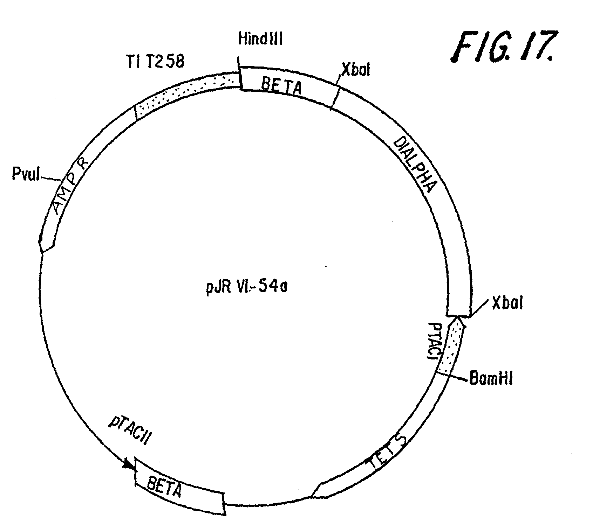

The dVal-beta globin gene under the control of syn pTAC was also ligated into another location in pDL III-47a. Syn pTAC/beta was removed from pDL IV-62a by digestion with Hind III and Pstl. The restriction site overhangs were filled with T4 polymerase and blunt end ligated into the Pvu II site of PDL III-47a to create plasmid pJR VI-54a (Figure 17). IPTG induction and SDS-PAGE analysis were as previously described.

Results of induction experiments as evaluated by SDS-PAGE indicated that control of expression of di-alpha and beta by separate promoters gave little increase in the expression of either protein. Similarly, insertion of a second beta globin gene under regulation of a separate promoter had little effect upon production of the proteins.

### Example 13: Hypothetical Protocol for Insertion of a Second Translationally coupled Beta Globin Gene Into the Di-Alpha/Beta Expression Plasmid

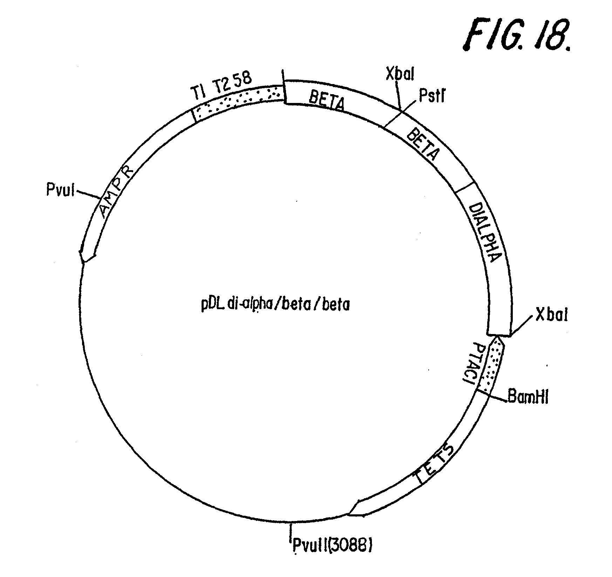

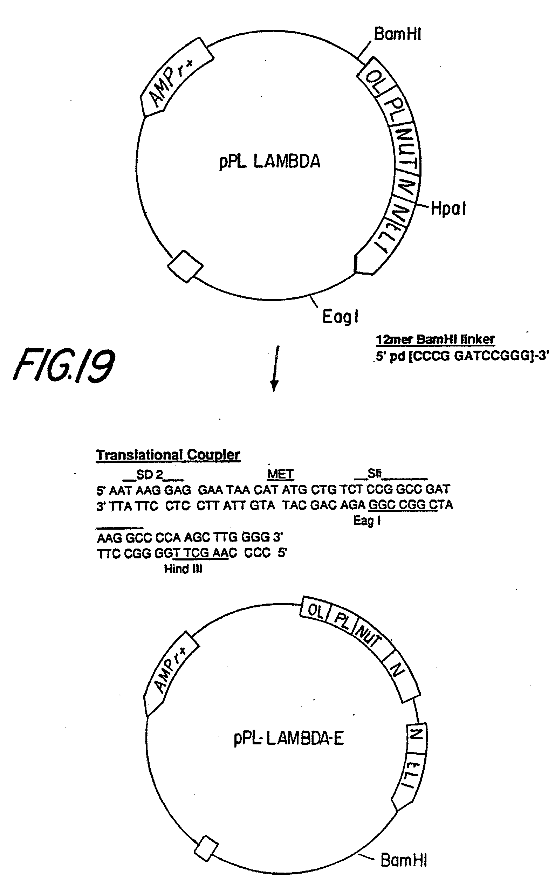

Plasmid pDL III-47a (Figure 13) is digested with HindIII and PstI and the HindIII/PstI fragment gel purified. The restriction site overhangs are then filled by T4 polymerase. The same plasmid is digested with HindIII and filled in with T4 polymerase. The beta globin gene is then blunt end ligated into the plasmid (Figure 18). Following transformation, individual clones can be analyzed by restriction digest to confirm the presence and orientation of the inserted beta globin gene. IPTG induction and SDS-PAGE analysis can be used to evaluate production to di-alpha and beta globins.

### Example 14: Hypothetical Protocol for Expression of Di-Beta Globin

A di-beta globin gene, analogous to the di-alpha globin gene described above can be constructed as follows. Plasmid pDL II-95a is a plasmid containing the des-val beta globin gene in the pGem-1 (Promega, Madison, W1) vector. pDL II-95a would be digested with the restriction enzymes PstI and BspMII and the large linear DNA sequence isolated by gel purification. A separate pDL II-95a plasmid would be restriction digested with PstI and NheI, and the small PstII to NheII fragment gel purified. Oligonucleotides encoding the di-beta linker containing the C-terminal amino acids of beta₁ and the N-terminal amino acids of beta₂, connected by a linker peptide of variable length (See Example 15) would be synthesized, gel purified and annealed as described previously. The generic sequence for such an oligonucleotide connecting the C-terminal his of beta₁ to the N-terminal val of beta₂ would be: 
NheI
BspMII
His Val
5'-CTAGCTCACAAATACCAC(XXX)nGTTCACCTGACT-3'
3'-GAGTGTTTATGGTG(XXX)nCAAGTGGACTGAGGCC-5'
 where the "XXX" indicates the codon(s) for the amino acid(s) linking the two beta globin peptides. Since the two beta globin termini are separated by approximately 18 angstroms in the deoxy state, it is anticipated that "n" would be in the range of 5 to 9. In the above example the oligonucleotide sequence spans the Nhe 1 restriction site, through the carboxyl terminal histidine of beta₁, a variable length coding sequence containing the amino acid linker to create di-beta, the amino terminal valine of beta₂ and the BspM II restriction site in the beta₂ gene.

To construct the plasmid encoding the di-beta polypeptide, the unphosphorylated di-beta linker DNA with NheI and BspMII restriction sites at its termini would be ligated to the PstI /NHeI fragment. Following ligation the dimeric forms resulting from the ligation of the PstI sites would be digested with PstI to produce fragments containing only PstI and BspMII termini. These fragments would then be subcloned into the PstI/BspMII cut plasmid. Following transformation, clones containing the di-beta construct would be confirmed by PstI and HindIII restriction fragment analysis. The linker region would be sequenced and the appropriate constructs subcloned into the expression plasmid containing either the di-alpha globin gene or a single alpha globin gene. E. coli would be transformed in the previously described fashion and assayed for production of hemoglobin as described before.

### Example 15: Hypothetical Protocol for Development of Linkers by Mutation and Selection

In this hypothetical example, the linker is obtained by mutagenesis of a linker-encoding DNA sequence and selection for functional linkers. Oligonucleotides spanning the BstBI site of alpha₁ to the EaqI site alpha₂ will be synthesized such that the six nucleotides comprising the preferred glycine-glycine linker are randomized. By randomizing these nucleotides, codons for all combinations of amino acids will be present in the oligonucleotide mixture. Following purification and annealing of the oligonucleotides they will be used to construct the di-alpha/beta co-expression genes as described above.

Clones containing the various di-alpha/beta plasmids will then be screened for production of increased levels of recombinant hemoglobin using a protocol developed at the Company. E. coli clones will be arrayed on nitrocellulose filters overlayed on 2xYT-ampicillin plates containing 1mM IPTG. Following overnight incubation of 37°C, the plates will be sealed in a plastic bag in which the air has been displaced by carbon monoxide (CO). CO binding to intracellular recombinant hemoglobin produces a distinctive red color in the E. coli colonies. Colonies producing the most intense red color will be further analyzed.

In this experiment the assumption is made that certain combinations of amino acids in the di-alpha linker will permit more stable folding of the individual, linked alpha globin chains and, therefore, result in greater levels of production of intracellular recombinant hemoglobin. This increase level of production will result in a more intense red color in the appropriate E.coli clones.

After selection of several clones producing higher levels of recombinant hemoglobin, more detailed analyses will be done on individual clones to determine the optimal di-amino acid linker. The analyses will include determination of quantities of recombinant hemoglobin produced, oxygen affinity, and protein stability. Finally, clones found to be producing the best quality recombinant hemoglobin will be DNA sequenced to determine the amino acids comprising the linker.

### Example 16: Hypothetical protocol for the synthesis of plasmids containing alpha and beta globin genes under the regulation of two separate promoters on the same plasmid.

It is anticipated that recombinant hemoglobin can be expressed from constructs where the different globin genes are under the control of separate promotors. This situation would yield two separate mRNA's; one with a dicistronic sequence encoding an alpha globin gene and another with a dicistronic sequence encoding a beta globin gene. For construction of an expression system in which both the alpha and beta globin genes are under the regulation of separate promoters, on the same plasmid, the following protocol would initially be used. Plasmid pDL III-1a containing the des-val alpha globin gene would be digested with the restriction enzyme BamHI, reacted with bacterial alkaline phosphatase, phenol extracted, ethanol precipitated, and resuspended in TE buffer. The plasmid pJR IIII 50-a which is the pKK expression plasmid containing the des-val beta construct would then be digested with the restriction enzymes BamHI and PvuI, to excise a fragment from the plasmid containing the P_{tac} promoter, the des-val beta sequence, the transcriptional terminator sequence and a portion of the ampicillin resistance gene. Following gel purification of this fragment, a PvuI-BamHI linker would be synthesized and ligated onto the insert. The insert would then be back-cut with BamHI to generate BamHI compatible sites on both the 5'- and 3'- ends of the insert. This insert would then be cloned into BamHI linearized plasmid pDL III-1a, resulting in a plasmid in which a translationally coupled des-val beta globin gene under regulation of one P_{tac} promoter is positioned on the 3' side of a translationally coupled des-alpha globin gene under regulation of a separate P_{tac} promoter. Restriction enzyme mapping would be used to confirm the orientation of the beta globin containing insert. E. coli JM-109 would be transformed with the plasmid containing the separate P_{tac} - globin constructs and grown in media containing ampicillin to isolate clones containing the plasmid. The clones containing the plasmid would then be induced with IPTG and expression of des-val alpha globin, des-val beta globin and des-val hemoglobin would be assayed by SDS-PAGE analysis, Western blotting with anti-hemoglobin antibodies and isolation of des-val hemoglobin by standard chromatographic methods.

Alternatively, coexpression of both globin genes could be achieved from DNA sequences on separate vectors under the control of separate promotors.

### Example 17: Hypothetical Protocol for the Construction of Vectors Containing alpha and beta globin genes under the regulation of separate promoters and on different vectors.

E. coli clones containing plasmid pDL III-1a which is the pKK223-3 plasmid containing a dicistronic loader gene/desval alpha construct under the regulation of the P_{tac} promoter would be transformed with a plasmid containing the dicistronic loader gene/des-val beta construct under control of the same promoter, but with a gene conferring additional antibiotic resistance to tetracycline. This could be constructed in the following manner: Plasmid pJR IV-50a contains the des-val beta globin gene under control of the P_{tac} promoter. This plasmid would be cut with PvuII to generate a linear plasmid with blunt ends. This would be ligated with a NotI phosphorylated linker (New England BioLabs). The ligation mixture will be used to transform E. coli. Plasmid DNA would be prepared and plasmids containing a NotI site identified by digestion with NotI agarose gel electrophoresis. This plasmid will contain the P_{tac}:des-val beta globin sequence. The gene for resistance to the antibiotic kanamycin is commercially available (Pharmacia) and contains EcoRI restriction sites on both ends. The ends will be converted to blunt ends by treatment with T4 DNA polymerase by the method of Maniatis, et al.. The resulting fragment will be ligated with a 50 fold excess of phosphorylated NotI linker (25°C, 60 min). The ligation reaction would be made 0.01 M in EDTA, heated to 70° for 20 min and ethanol precipitated. The precipitated DNA will be taken up in 100 ul of NotI buffer and treated with 100 units of NotI (37°) for 2 hr. The fragment would be purified by agarose gel electrophoresis. The NotI adapted kanamycin resistance gene would then be ligated into the NotI linearized pJR IV-50a to yield a plasmid with the gene for des-val beta globin under control of P_{tac} with kanamycin resistance. E.coli JM-109 clones containing plasmid pDL III-la, the plasmid containing des-val alpha globin under control of P_{tac} with resistance to ampicillin, will then be transformed with kanamycin resistant plasmid containing the gene for des-val beta globin and clones will be selected for resistance to both ampicillin and kanamycin. Other antibiotic resistance genes could be used as well. Expression of alpha and beta globin polypeptides under the regulation of separate promoters will then be analyzed by IPTG induction, SDS page and western blotting.

Should we encounter problems with plasmid exclusion, we could use the same strategy with the pIN plasmids that have been used to express polypeptides from separate plasmids in E. coli (McNally, et al., PNAS 85, 7270, 1988).

One potential problem that we may face with creating plasmids in which the alpha and beta globin genes are under separate but identical promoters is the possibility of homologous recombination within the identical sequences on the plasmids, eg. the promotor region. This could result in deletion of a segment of important DNA sequence. It is therefore preferable to use different, non-homologous promotors for each different globin gene, eg. P_{tac} and _{Ptrc}, or the lambda P_{L} promotor in appropriate host (containing cI857).

### Example 18: Synthesis and Assembly of the Di-AlPha Beta Globin Construct in aP_{L} Regulated Vector System

In prior examples, the globin genes were under Tac promoter control, and the alpha (of di-alpha) and beta globin genes were each translationally coupled to the ribosomal loader cistron taught by Schoner, et al. In this example, the lambda P_{L} promoter and a different translational coupler (see below) are used.

Translational Coupler

SD2 MET Sfi
5'AAT AAG GAG GAA TAA CAT ATG CTG TCT CCG GCC GAT (Cont'd)
3'TTA TTC CTC CTT ATT GTA TAC GAC AGA GGC CGG CTA (Cont'd)
EAGI

AAG GCC CCA AGC TTG GGG3'
TTC CGG GGT TCG AAC CCC5'
Hind III

The pL expression system has a different translational coupler as compared to the pTAC system. Sequences coding for the two SD's and the translational stop were added onto the 3' end of the N protein coding sequences to act as a translational coupler. Subsequent to that the globin coding sequences are identical to that used in the pTAC system.

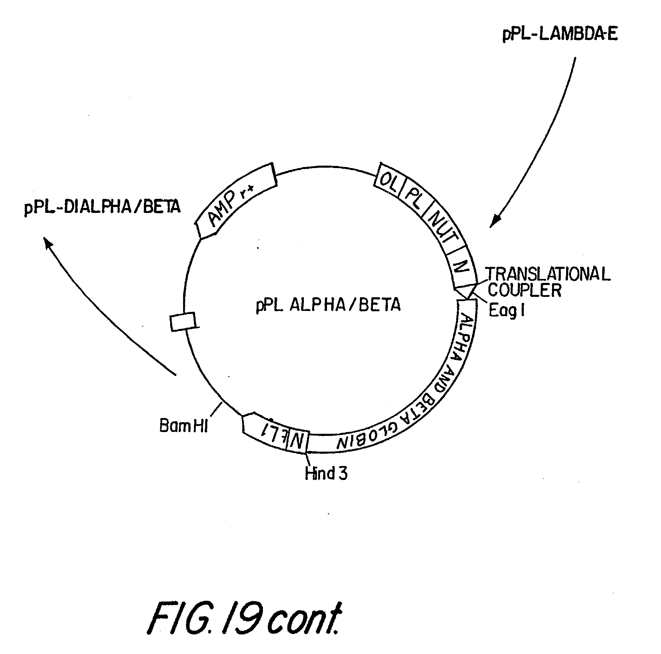

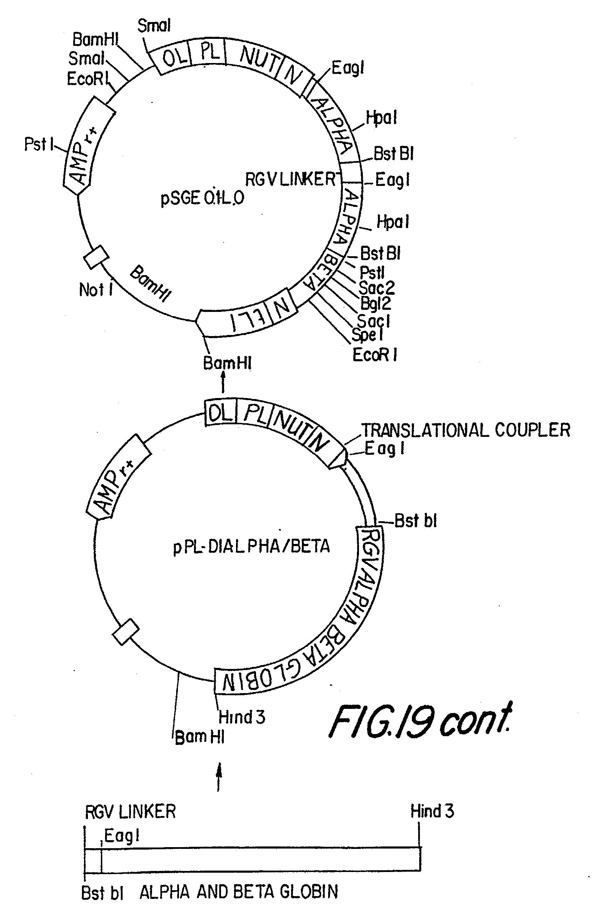

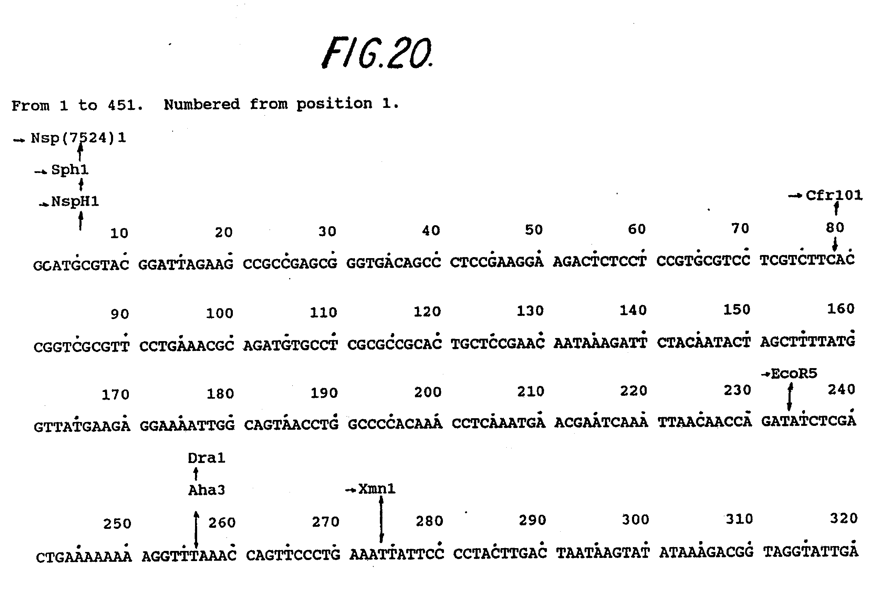

Using the pPL-lambda vector available from Pharmacia, a plasmid construct was assembled to generate a genetically crossed-linked tetrameric human hemoglobin (wild type) in E. coli strains N99Ci+ and N4830-1 (cI857). These bacterial strains were obtained from Pharmacia and are inducable by addition of naladixic acid (40µg/ml) or mitomycin C (10µg/ml) in the presence -of the wild type Ci+ repressor or heat treatment of the strain containing the cI857 repressor gene (Mott, et al., pNAS 82, 88, 1985 and Gottesmann, M.E., et al., J.Mol. Biol. 140, 57, 1980). A diagramatic representation of the cloning strategy is depicted in figure 19.

### Removal of the EagI Site from the pPL-Lambda Vector

Removal of the EagI site from pPL-lambda was necessary to enable cloning of the di-alpha gene sequence, because both alpha structural genes contains a EagI site located 6 bp into the coding sequence. The pPL-lambda vector was digested with EagI, and the ends were filled using T4 DNA polymerase. The Bam HI linker (5'-CCCGGATCCGGG-3')(Pharmacia), was blunt-end ligated to the EagI digested pPL-lambda plasmid by standard methods. This eliminated the EagI site in the desired construct. The resulting mixture was digested with EagI to eliminate any plasmids still containing the EagI site. E. coli N99Ci+ cells were transformed with resulting plasmid, pPL-lambda-E. Clones containing the desired plasmid were identified by restriction digest analysis.

### Incorporation of the Synthetic Co-Translational Coupler into pPL-hambda-E

Prior to inserting the globin genes into the vector it was necessary to incorporate the synthetic translational coupler sequence into the HpaI site of pPL-lambda-E. This was done by digestion of pPL-lambda-E with HpaI followed by blunt-end ligation of the co-translational coupler into the HpaI site of the vector. Ligation of the coupler to the blunt end resulted in destruction of the HpaI site. The ligation mixture was treated with HpaI to digest any plasmid remaining containing the HpaI site. E. coli N99Ci+ cells were transformed with the resulting reaction mixture. Clones were screened with EcoRI and Hind III restriction digests to identify clones containing the co-translational coupler in the proper orientation. DNA fragments of 522 bp and 4762 bp were observed for plasmid containing the desired orientation. To confirm the orientation of the coupler, the resulting plasmid was sequenced using a primer (5'CAATGGAAAGCAGCAAATCC-3') complementary to the sequence 30 base pairs upstream from the translational coupler sequence. The desired plasmid was denoted as pPL-lambda-E+TC.

### Construction of an Expression Plasmid Containing Des-Val Alpha and Beta Genes Under Control of pPL-Lambda

The Des-Val alpha and beta globin genes were obtained from pDL III-14c by digestion with EagI and Hind III, followed by agarose gel purification of the desired 942 bp segment. The purified alpha and beta globin gene fragment was cloned into EatI and Hind III digested pPL-lambda-E+TC. The ligation mixture was used to transform E. coli N99Ci+, and clones wre screened for the presence of the desired plasmid, pPL-alpha/beta with EcoRI (4758 and 1468 bp), and with PstI and Hind III (4005, 1775, 520 bp) confirmed presence of the desired restriction sites. Further confirmation was obtained through sequencing with the 20 bp primer, above, to confirm the sequence between the co-translational coupler and the Des-Val alpha globin gene, and a second primer (5'ACCCGGAAAACTTCCGTC-3') to confirm the sequence between Des-Val beta and the pPL vector.

### Construction of an Expression Plasmid Containing Di-Alpha and Beta Globin Genes Under Control of pPL-Lambda

An RGV crosslinker which encodes for the carboxy terminal portion of alpha globin, linked via a single glycine residue to the native sequence of the amino portion of a second alpha globin chain, was prepared by separately phosphorylating the 5' ends of 5'CGAAATAACGTGGTGTTCTGTCTGC-3' and 3'TTTATGGCACCACAAGACAGACGCCGG-5' with T4 kinase, followed by annealing. This double stranded oligonucleotide was cloned onto the EagI end of a purified fragment of Des-Val alpha and beta globin prepared from pDL III-14c digested with EagI and HindIII, as described above. The linear DNA sequence generated from this ligation, now containing sticky ends coding for BstBI and HindIII restrcition site sequences, was purified by agarose gel electrophoresis and cloned into BstBI and HindIII digested pPL-alpha/beta. The new plasmid designated pPL-dialpha/beta contained a sequence with a co-translational coupler upstream to a sequence containing di-alpha globin linked via a glycine residue, followed by a cotranslational coupler adjacent to a beta globin gene sequence, all under conrol of a sinle P_{L} promoter. These clones were identified through screening minipreps with EagI restriction digestions. Clones without the second alpha globin gene merely linearised upon digestion, whereas clones containing the second gene released 431 bp and 6222 bp DNA fragments.

### Construction of the Expression Plasmid pSGE0.1-L0 containing a ROP- Origin of Replication Mutation

pPL-dialpha/beta was digested with PvuII then treated with T4 DNA polymerase to fill in the sticky ends. The linearized plasmid was then blunt end ligated witha NotI linker (Promega Corp., Madison, WI) (5'TTGCGGCCGCAA-3'). The ligation mixture was then treated with PvuII to remove any remaining plasmid containing the PvuII site. E. coli were transformed with pSGE0.1-L0 and positive clones were identified by the presence of the unique NotI restriction site.

### Expression of Hemoglobin SGE0.1 in E. Coli Under the Control of the Lambda P_{L} Promoter

E. coli N99Ci+ and E. coli N4830-1 were transformed with pSGE0.1-L0 and grown on agar plates containing ampicillin, as described previously. These E. coli strains contain the cI+ repressor gene and the cI857 heat sensitive repressor gene, respectively.

Inocula of N99Ci+ were grown at 37°C in TB media to an OD₆₀₀ of ^{~} 1.0 and induced with naladixic acid (40µg/ml). Cultures were incubated for 4-6 hrs. at 37°C before the cells were harvested. Hemoglobin production was estimated by SDS-PAGE analysis of total cell protein and by western blot analysis. By these techniques, SGE0.1 was estimated to be produced at ^{~}0.02% of the total cell protein in this cell line. Hemoglobin (57µg) was isolated by Mono Q chromatography and shown to have an optical spectrum representative of that for normal hemoglobin.

Inocula of N4830-1 were incubated at 30°C in TB media to an OD₆₀₀ of 1.0 and induced by addition of sufficient preheated TB media (65°C) to raise the temperature of the inocula TO 42°C. The culture was then incubated at 42° for 4-6 hrs. Total cell protein analysis with SDS-PAGE revealted that SGE0.1 was being synthesized at 0.4% of the total cell protein, corresponding to 0.18 mg protein per gram of wet cell paste. SGE0.1 prepared from a 2L preparation was purified as described elsewhere, and resulted in isolation of 7.6 mg of purified material, SGE0.1 (7.6mg) was isolated and had a P₅₀ of 7.08 (Hemox Analyzer, pH 7.4, 0.1 M NaCl, 37°C), and an optical spectrum representative of native hemoglobin.

### Example 19: Production of Hemoglobin in Yeast

All restriction enzymes and DNA-modifying enzymes were purchased from BRL, New England Biolabs, IBI, Pharmacia or Boerhringer-Mannheim. The concentrations of enzymes used were those suggested by the supplier to produce a complete reaction in 30 minutes. The buffers and conditions for the use of these enzymes were those provided with the enzymes, unless otherwise stated. Plasmid DNA was purified from E.coli DH5α as described by Birnboim and Doly (Nucleic Acids Research 1979, 7:1513-1520). Electrophoretic analysis of DNA was carried out in agarose gels using tris-acetate electrophoresis buffer (Maniatis et al. Molecular Cloning, Cold Spring Harbor, NY, 1982). DNA was visualized by staining the gels with 0.5µg/ml ethidium bromide and exposing the gel to ultraviolet light. DNA fragments were purified from agarose gels using a kit purchased from BIO-101. DNA fragments were purified from acrylamide gels by crushing the excised gel fragment, containing the DNA, of interest in 3.25M ammonium acetate and incubating overnight at 37°C. Gel fragments are removed by centrifugation (12,000 x g, 15 min) and the DNA precipitated with 2 volumes of 95% ethanol, 5% isopropanol. The precipitate is dried in vacuo and dissolved in 0.1XTE (1XTE is 10mM Tris. HCl pH7.8, 1mM Na₃ EDTA), Acrylamide gel electrophoresis of DNA was done as described by Maniatis, et al. (Molecular Cloning, Cold Spring Harbor, NY, 1982.) in tris-acetate electrophoresis buffer. Bacteriological growth media and DNA transformation methods are described by R.W. Davis et al. (Advanced Bacterial Genetics, Cold Spring Harbor Laboratory, New York, 1980, p140-141). Transformation of S. cerevisiae with linear or circular DNA was carried out as described by H. Ito et al. (J. Bacteriology 153:163-168 (1983)). Transformants were selected on SD medium lacking uracil or tryptophane (SD-ura, SD-trp) depending on the selectable marker on the plasmid (F. Sherman et al., Methods in Yeast Genetics: A Laboratory Manual, Cold Spring Harbor Laboratory, 1979). All other yeast media used are described by Sherman et al. (ibid.)

### SYNTHESIS AND ASSEMBLY OF A GALACTOSE REGULATED PROMOTER

This synthetic promoter consists of two functional parts, a regulatory sequence and sequence that allows efficient initiation of mENA synthesis. One of the regulatory regions we chose includes the nucleotide sequence that confers positive regulation of transcription in the presence of galactose (M. Johnston and R. Davis, 1984. Molecular and Cellular Biology 4:1440-1448; L. Guarente et al., 1982, Proc Nat Acad Sci (USA) 79:7410-7414.). The transcriptional initiation site is derived from the consensus sequence for the S.cerevisiae glyceraldehyde-3-phosphate dehydrogenase gene (GAP491) (L. McAlister and M.J. Holland, J. Biol Chem 260:15019-15027, 1983; J.P. Holland et al., J. Biol Chem 258:5291-5299, 1983).

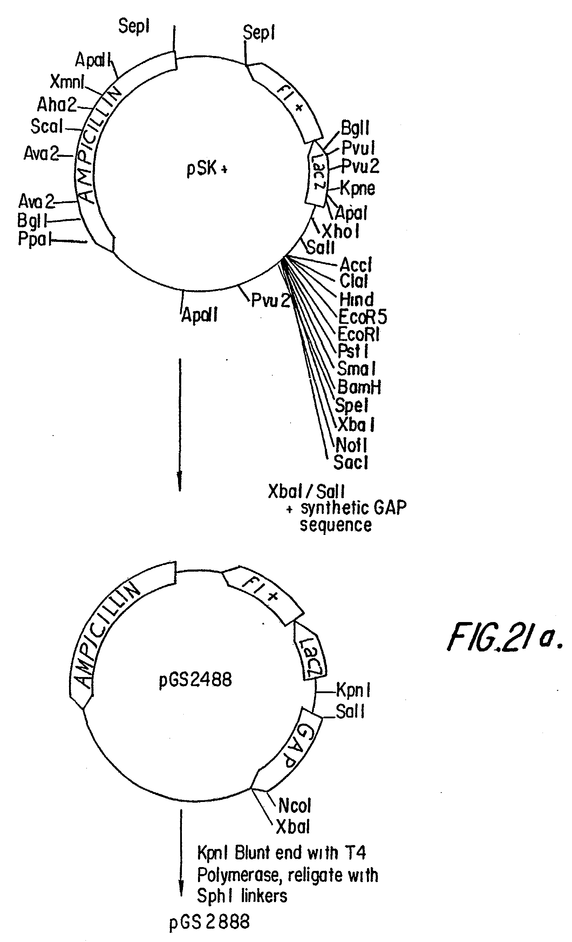

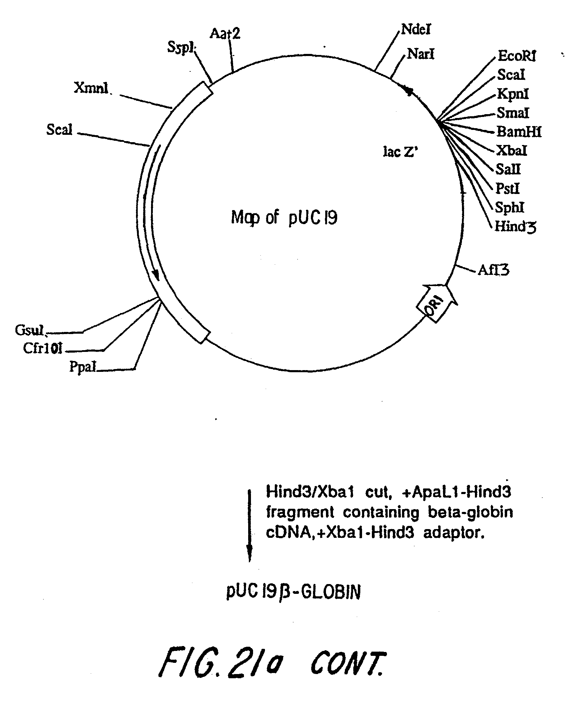

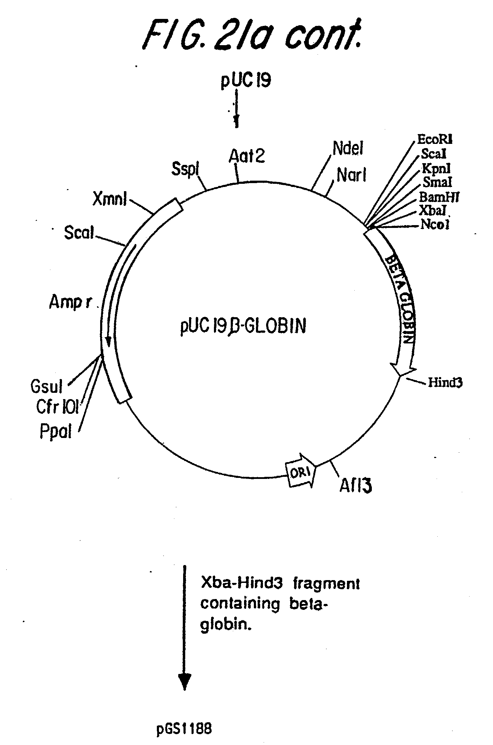

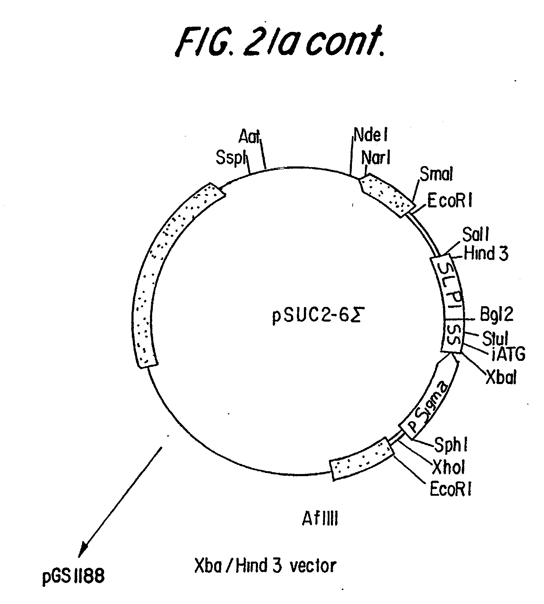

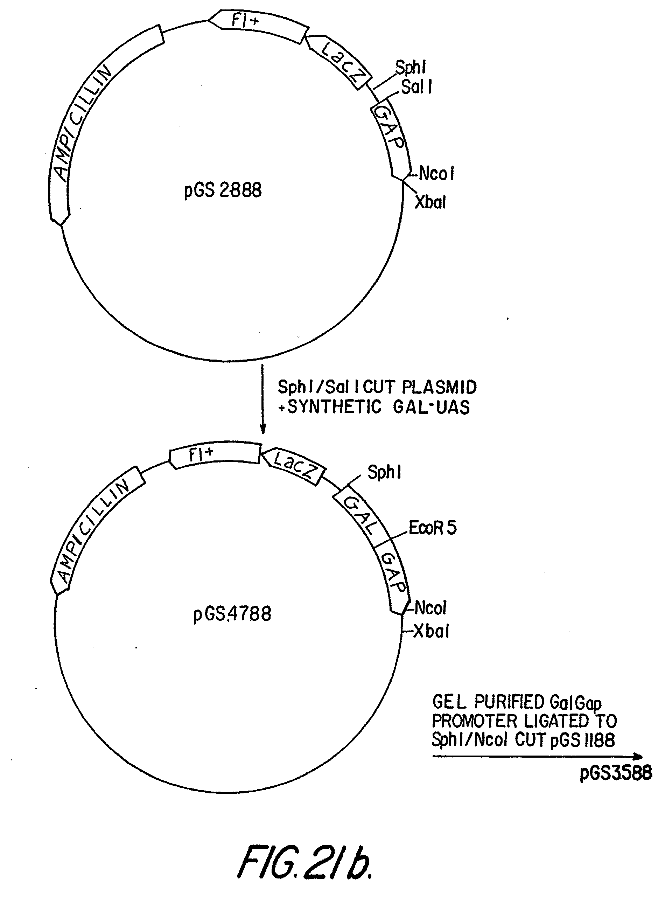

The synthetic oligonucleotides shown in figure 1 were synthesized on a Biosearch 8600 DNA synthesizer. Each oligonucleotide was cleaved from its support column with 28% NH₄OH. The blocking groups were removed by incubating the cleaved oligonucleotide in 28% NH₄OH at 65°C for ≥ 16hr. All oligonucleotides were purified by preparative polyacrylamide gel electrophoresis in slabs of 10% acrylamide (19:1 acrylamide:bis-acrylamide) containing 7M urea. Oligonucleotides were eluted from acrylamide slices by incubation (16hr) in 50mM ammonium acetate (pH7.4), 2.5mM magnesium acetate, 0.25mM EDTA and 0.25% SDS at 37°C. Acrylamide fragments were removed by centrifugation (14,000xg, 10min) and the oligonucleotide precipitated from the aqueous phase by the addition of NaCl to 0.25M and 3 volumes of 100% ethanol. The precipitated oligonucleotides were collected by centrifugation (14,000xg for 30min), washed twice with 80% ethanol, once with 100% ethanol and the pellets dried. Pellets were dissolved in 0.1XTE. 2 x 10⁻¹⁰ moles (each) of oligonucleotides 1-5 (Table 5) were phosphorylated in 0.02 ml of 0.066M Tris HCl (pH 7.6), 0.01M MgCl₂, 0.002M dithiothreitol (DTT), 0.001M spermidine and 10 units of T4 polynucleotide kinase. Phosphorylation reactions were carried out at 37°C for 30min and terminated by heating to 96°C for 5 min. 2 x 10⁻¹⁰ moles of oligonucleotides 1 and 6 (Table 5) were added to the phosphorylated oligonucleotides in a final volume of 0.04ml of 0.07M Tris HCl (pH7.6) 0.01M MgCl₂. The mixture of oligonucleotides 1-6 (Table 5) was heated to 96°C for 5 min, 75°C for 20 min, 55°C for 30 min, 37°C for 60 min and 25°C for 15 min. T4 DNA ligase (10 units), DTT (0.002M final concentration), and ATP (0.001M) were added and the mixture incubated at 4°C for 16hr. The resulting 210 bp oligonucleotide contains a 5' end compatible with a SalI restriction endonuclease site and a 3' end compatible with an XbaI site. Because the oligonucleotides comprising the two ends of the intact oligonucleotide were not phosphorylated, they cannot ligate to each other. This oligonucleotide was cloned into the vector pSK+ (Stratagene, Inc.) (Figure 21(a)) that had been digested with XbaI and Sall. The ligation mixture contained 50ng of XbaI, Sail digested pSK(+), and 5pMoles of the ligated oligonucleotide in a volume of 0.01ml. E.coli DH5α was transformed with a portion of the ligation reaction and clones that contain inserts were identified by screening for white colonies on LB-ampicillin (0.15mg/ml) agar plates supplemented with XGAL (4µg/ml). Positive identification was made by preparing plasmid DNA from these isolates and digesting with XbaI and Sall. The restriction digests were analyzed by agarose gel electrophoresis and three clones containing a fragment of the expected size (^{~} 250bp) were identified. The DNA sequence of all three clones was determined and one was chosen for further use and designated pGS2488 (Figure 21(a)).

### ASSEMBLY OF THE SYNTHETIC GALACTOSE UPSTREAM ACTIVATOR (GAL_{UAS}) SEQUENCE

The oligonucleotides shown in Table 6 were synthesized and purified as described above. Oligonucleotides 2-5 were phosphorylated, annealed with oligonucleotides 1 and 6 and ligated as described for the assembly of GAP. The full length oligonucleotide generated by this protocol has non-phosphorylated ends compatible with the restriction endonuclease sites generated by SphI and SalI. However, when the oligonucleotide is ligated to a Sail site the resulting junction formed between the two fragments will no longer contain a cleavable SalI site.

The GAL_{UAS} is contained on an SPHI - Sail fragment. To clone this fragment into pGS2488 required that we change the KpnI site of this plasmid to an SphI site. The plasmid pGS2488 was modified by cleaving with KpnI. The KpnI digested plasmid was incubated with 2 units of T4 polymerase in 0.05ml buffer containing 50µM of each deoxyribonucleotide triphosphate (A,G,C,T), 0.033M Tris-acetate (pH 7.9), 0.066M potassium acetate, 0.01M magnesium acetate, 0.5mM DTT and 100µg/ml bovine serum albumin (BSA). Na₃EDTA was added to 0.015M and the mixture extracted 1X with phenol-chloroform. DNA was precipitated with ethanol. The dry pellet was dissolved in 0.008ml of T4 DNA ligase buffer, 50ng phosphorylated SphI linkers (New England Biolabs) and 10 units of T4 DNA ligase. The mixture was incubated for 1 hour at 25°C and used to transform E.coli DH5α. Plasmid DNA was prepared from 12 transformants and tested by restriction enzyme digestion and agarose gel electrophoresis, for the presence of an SphI site and the absence of a KpnI site. A clone containing a plasmid with these characteristics was identified and the plasmid was designated pGS2888 (Figure 21(b)).

The next step in the assembly of this hybrid promoter was to clone the SphI - SalI fragment containing the GAL_{UAS} into pGS2888. pGS2888 was digested with SphI and Sall, phenol-chloroform extracted and ethanol precipitated. Fifty nanograms of SphI, Sail digested pGS2888 was incubated with 25ng of the annealed, ligated GAL_{UAS} mixture in 0.005ml 1X ligase buffer containing 10 units of T4 DNA ligase. The ligation mixture was incubated overnight at 4°C and a portion used to transform E.coli DH5α. Ampicillin resistant clones were isolated and plasmid DNA prepared. The plasmid DNA (digested with XbaI and SpI) was analyzed by agarose gel electrophoresis. A plasmid containing a fragment of the expected size (⁻ 500bp) was identified. The sequence of the putative GAL_{UAS} portion of this plasmid was determined and the plasmid was designated pGS4788 (Figure 21(b)). The complete sequence of the synthetic GALGAP promotor (pGGAP) is shown in figure 20.

### CONSTRUCTION OF A pGGAP-β-GLOBIN EXPRESSION CASSETTE

The plasmid pLCIIFX-β-globin (K. Nagai, M. Perutz and C. Payart, Proc Nat Acad Sci (USA) 82:7252-7255) was used as the source of human β-globin cDNA. The coding region of the β-globin cDNA can be excised as an ApaL1 to Hind3 fragment that is missing only the first four nucleotides of the β-globin coding (translated) sequence. pLcFX β-globin (5µg) was digested with ApaL1 and Hind3 and a 550 bp fragment containing the cDNA was purified by acrylamide gel electrophoresis. The ApaL1 - Hind3 fragment containing the β-globin cDNA was cloned into Xba1, Hind3 digested pUC19 using the following adaptor (synthesized and purified as described above):

XbaI NcoI ApaL1
YH1a/b5'-CTAGAACCATGG
TTGGTACCACGT-5'

The two oligonucleotides were mixed (12.6µg of each), NaCl was added to 0.25M and three volumes of ethanol (anhydrous) was added. The precipitated oligonucleotides were collected by centrifugation (14,000 x g, 15 min) and the pellet washed twice with 80% ethanol, once with anhydrous ethanol and dried in vacuo. The adaptors were dissolved in 1ml of 0.1XTE. Fifty nanograms of Xba1-Hind3 fragments containing the β-globin cDNA and 12.5ng of the ethanol precipitated (non-phosphorylated) YH1a, b oligonucleotide in 0.010 ml of T4 DNA ligase buffer containing 10 units of T4 DNA ligase. The ligation mixture was incubated at 4°C for 16 hr. and a portion used to transform E.coli DH5α. Transformants were selected on LB-ampicillin plates containing 4µg/ml XGAL. Plasmid DNA was prepared from 12 white colonies. The DNA was digested with Nco1 or ApaL1 and analyzed by agarose gel electrophoresis. Four of these colonies contained plasmids with the expected restriction fragments and one was designated pUC19β-globin (Figure 21(a)). The plasmid pSUC2-6∑ (G. Stetler et al. Biotechnology 7:55-60 (1989)) (Figure 21(a)) was digested with Hind3 and Xba1 and the large fragment was purified by agarose gel electrophoresis. The Xbal to Hind3 fragment containing p-globin cDNA was also purified (agarose gel electrophoresis) from pUC19β-globin. Ten nanograms of gel-purified Xbal, Hind3 digested pSUC2-6Σ was mixed with 16 nanograms of the Xba1, Hind3 fragment from pUC19β-globin in 0.01ml of ligase buffer containing 10 units of T4 DNA ligase. The ligation mixture was incubated at 25°C for 1 hr. and a portion used to transform E.coli DH5α. Transformants were selected on LB-ampicillin medium and three were used to prepare plasmid DNA. These were analyzed by digestion with EcoR1 and analyzed by agarose gel electrophoresis. Two of these contained plasmids with the expected restriction fragments and one was designated pGS1188 (Figure 21(b)). This plasmid contains β-globin under the transcriptional control of the sigma promoter and contains the 3' transcriptional termination signals and polyadenylation signals of the MFα1 gene.

### REPLACEMENT OF THE SIGMA PROMOTER WITH pGGAP

The plasmid pGS1188 was digested with SphI and NcoI and the vector plus β-globin cDNA was separated from the sigma promoter by agarose gel electrophoresis and purified as described previously. Plasmid pGS4788 (10ug) was also digested with SphI and NcoI and the ^{~} 500bp fragment (containing pGGAP) produced by this digest was purified by agarose gel electrophoresis. Fifty nanograms of the gel-purified β-globin containing vector was incubated with 50ng of NcoI-SphI fragment containing the pGGAP promotor in 0.01ml of 1X ligase buffer with 10 units of T4 DNA ligase. The mixture was incubated for 1 hour at 25°C and a portion of the ligation mixture used to transform E.coli DH5α. Ampicillin resistant clones were selected. Plasmid DNA isolated from 12 of these clones was analyzed by digestion with SphI and NcoI to identify plasmids containing the ^{~} 500 bp GGAP promoter. The presence of the β-globin cDNA was confirmed by digestion with XbaI and Sall, followed by agarose gel electrophoresis analysis. A plasmid containing the expected fragments was identified and designated pGS3588 (Figure 21(b)). To aid the subcloning of this fragment into a yeast vector, the SmaI site of pGS3588 was converted to a XhoI site as follows: 1 µg of pGS3588 was digested with SmaI, the digest was extracted once with phenol-chloroform and ethanol precipitated. The precipitated DNA was dissolved in T4 DNA ligase buffer, with 100ng of phosphorylated XhoI linker and 10 units of T4 DNA ligase (final volume of 0.01ml). The ligation was incubated at room temperature for 2 hr and a portion of the ligation mixture was used to transform E.coli DH5α (excess linkers were not removed prior to transformation). Ampicillin resistant clones were isolated and plasmid DNA prepared. Plasmids containing the additional XhoI site were identified by digestion with XhoI and agarose gel electrophoresis analysis. A plasmid containing the pGGAP-β-globin expression cassette was identified and has been designated pGS3888 (Figure 21(b)).

### CONSTRUCTION OF AN α-GLOBIN EXPRESSION CASSETTE

We obtained a partial length cDKA (pα-MRC) clone from K. Nagai (MRC, Cambridge). To adapt the cDNA encoding α-globin for expression from the pGGAP promotor, two oligonucleotide primers were synthesized (synthesis and purification of oligonucleotides was as described above).

α-1: 5'-GAATTCCATGGTGCTGTCTCCTGCCGACAAGACC-3'.

α-2: 5'-CTGCAGTCGACTTAACGGTATTTGGAGGTCAGCACGGTGCT-3'.

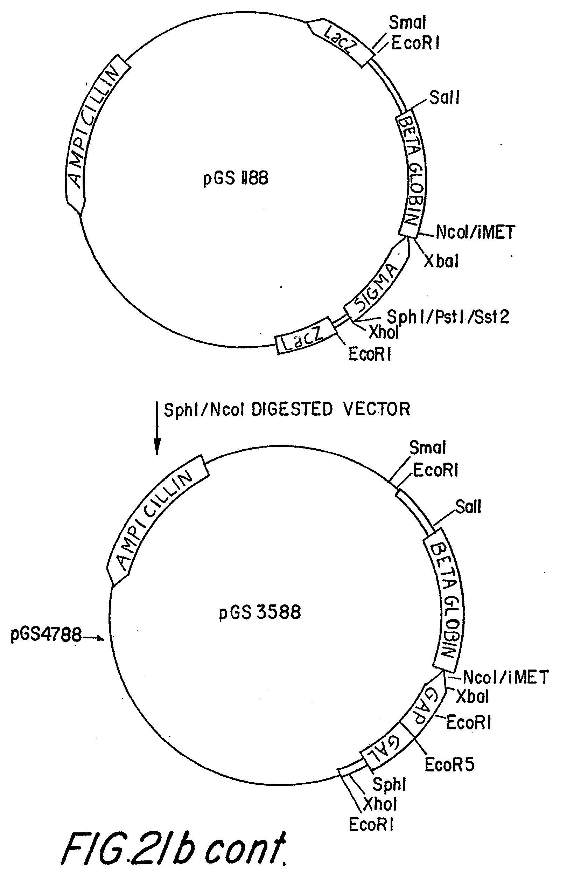

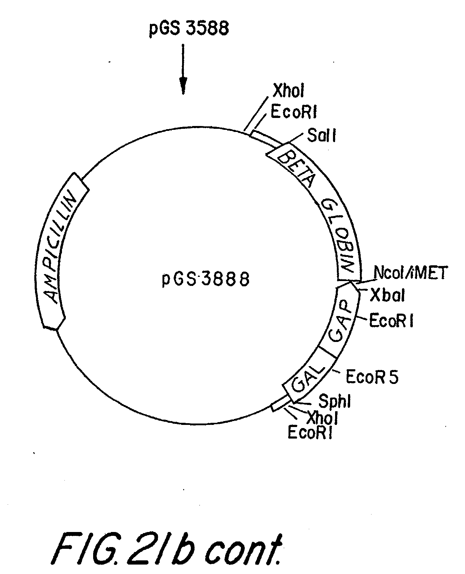

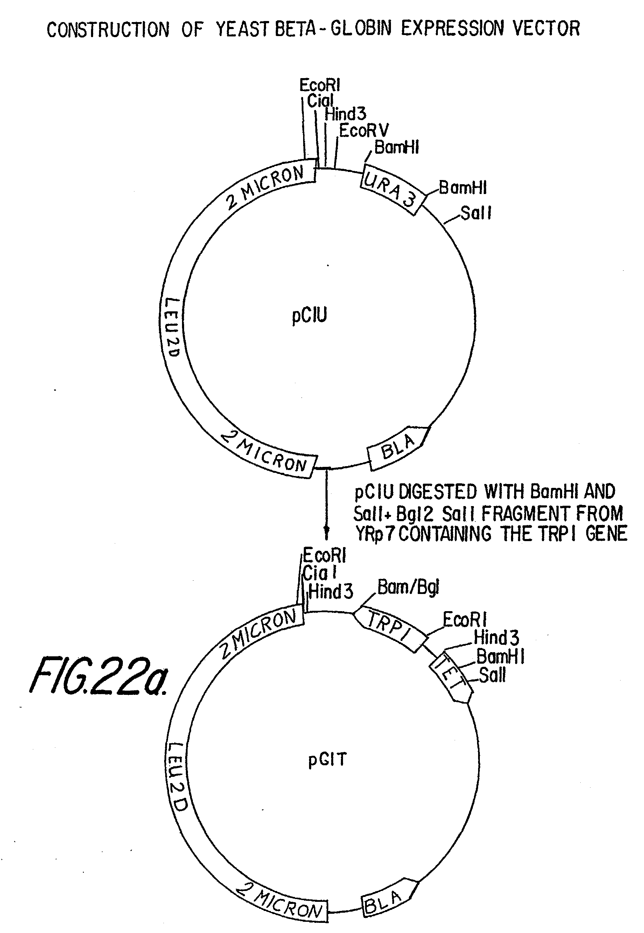

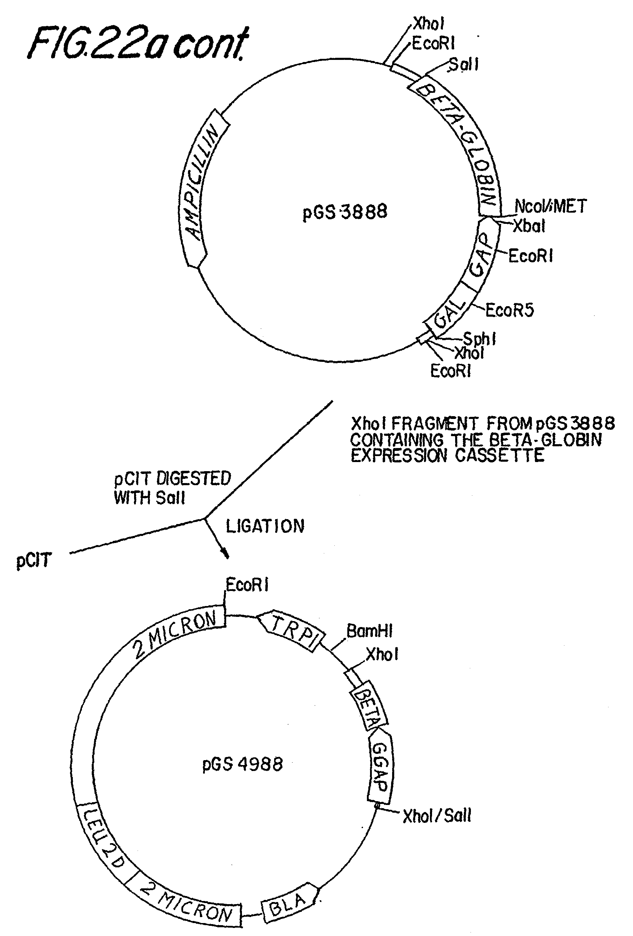

These two oligonucleotides were used as primers for a polymerase chain reaction (PCR) (R.K. Sakai et al., 1985. Science 230:1350-1354) using a Perkin Elmer-Cetus PCR kit and pα-MRC as template. α-globin cDNA (8.3ug/ml) in 0.018 ml H₂O was denatured by the addition of 0.005ml 10M NaOH. The mixture was incubated at 25°C for 5 min. Denatured DNA was precipitated by the addition of 0.003ml 3M sodium acetate (pH 5.2) and 0.075ml anhydrous ethanol. The precipitated DNA was washed twice with 80% ethanol, once with 100% ethanol and dried. The dried pellet was dissolved in: 0.005ml 20µM α-1, 0.005ml 20µM α-2, 0.010ml 10X Taq polymerase buffer (Perkin Elmer-Cetus), 0.0005ml TaqI polymerase (Perkin Elmer-Cetus), 0.663ml H₂O, 0.016ml of all four deoxyribonucleotide-triphosphates (1.25mM each). TaqI polymerase was added after heating the solution to 94°C for 1 min. After addition of the enzyme the aqueous solution was overlayed with 0.100ml paraffin oil. The reaction-mixture was cycled, by hand, 25 times at the following temperatures: 37°C for 2 min, 68.5°C for 3min and 94°C for 1 min. After the twenty-fifth cycle, the reaction mix was incubated at 37°C for 2 min and 68.5°C for 10 min. A portion (0.005ml) of the reaction mixture was analyzed by agarose gel electrophoresis and revealed a band of the expected size (^{~} 430bp). The PCR-amplified DNA fragment should contain a 5' extension, that includes an ATG codon embedded in an optimal sequence context for initiation of translation (M. Kozack 1986, Cell 44:283-292). The 3' end should contain an extension that includes a translational terminator and a SalI restriction endonuclease site. The PCR-amplification reaction was phenol-chloroform extracted and ethanol precipitated. The dry pellet was dissolved in 0.05ml 1mM Tris-HCl (pH 7.8). A portion of this material (0.025ml, 1.25ug) was digested with NcoI and SalI and purified by acrylamide gel (5%) electrophoresis. A gel slice containing the fragment was eluted by crushing the gel slice in 0.3ml 2.5 ammonium acetate (pH 7.4) and incubating at 37°C for 16hr. Acrylamide fragments were removed by centrifugation and the DNA precipitated by the addition of 0.75ml of ethanol. The pellet was collected and dissolved in 0.020ml 1mM Tris HCl (pH 7.8) 0.1mM EDTA. This fragment was cloned into NcoI, SI digested and agarose gel purified pGS3888. Fifty nanograms of NcoI, SalI digested pGS3888 was incubated (2hr, 25°C) with 50ng of gel purified, PCR-amplified NcoI-SalI fragment containing the α-globin cDNA in 0.01ml ligase buffer with 10 units of T4 DNA ligase. A portion of this reaction mixture was used to transform E.coli DH5α- and ampicillin resistant clones were selected on LB-ampicillin medium. Plasmid DNA was prepared from 12 independent isolates and digested with Nco1 and SalI. The restriction digests were analyzed by acrylamide gel electrophoresis (5%), all twelve contained a fragment of the expected size. One of these was designated pGS4088 (Figure 22). The α-globin insert in pGS4088 was completely sequenced to assure that no mutations had been introduced by PCR-amplification.

### CONSTRUCTION OF A YEAST EXPRESSION PLASMID THAT CO-EXPRESSES α-AND β-GLOBIN GENES FROM A SINGLE PLASMID

The α-globin and β-globin expression cassettes from the plasmids pGS3888 and pGS4088 were cloned into a single plasmid in a way that allows them to be excised on a single NotI fragment. This NotI fragment was then cloned into the high copy yeast plasmid pC1N to generate a plasmid carrying and expressing both α-and β-globin chains under the control of separate (though identical) promoters. The details are presented below.

### INTRODUCTION OF NOTI SITE INTO pSK(+)

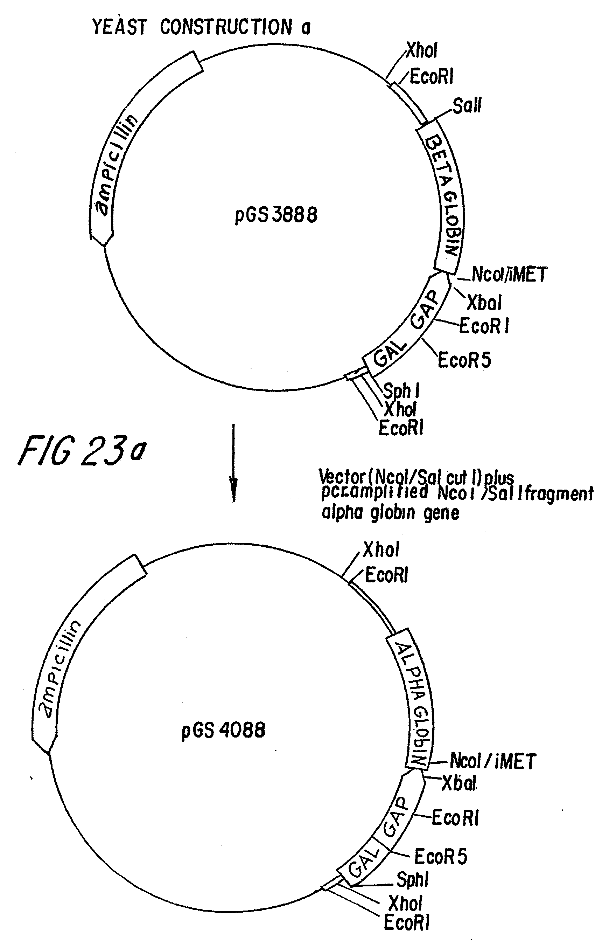

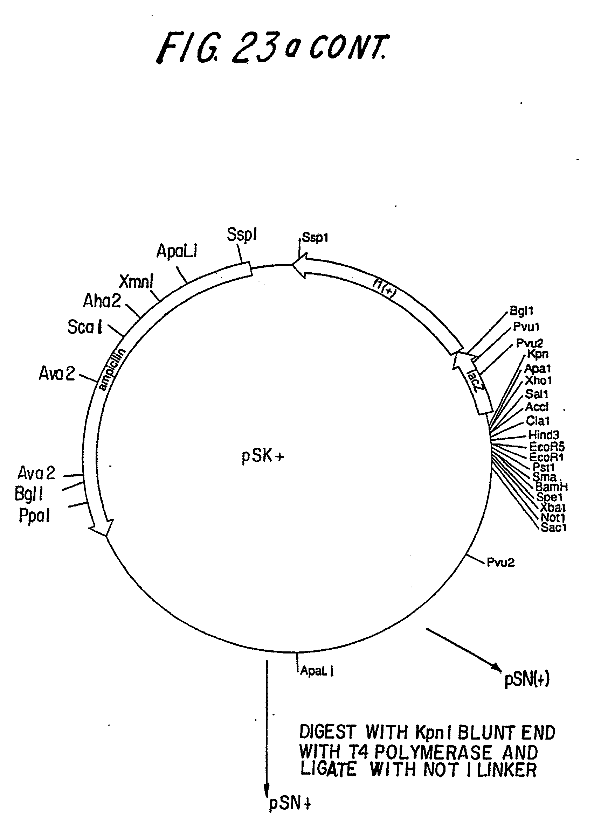

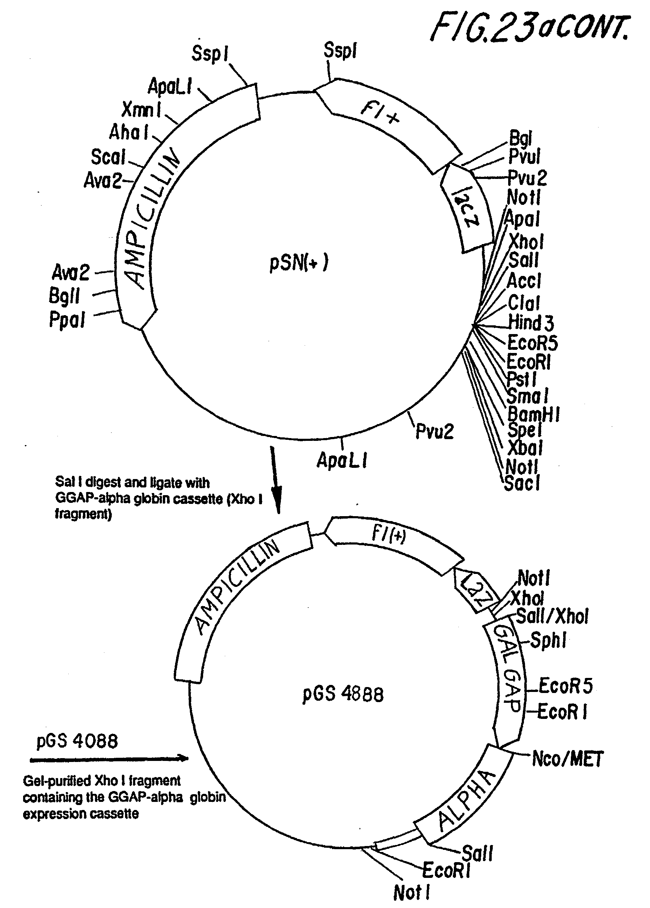

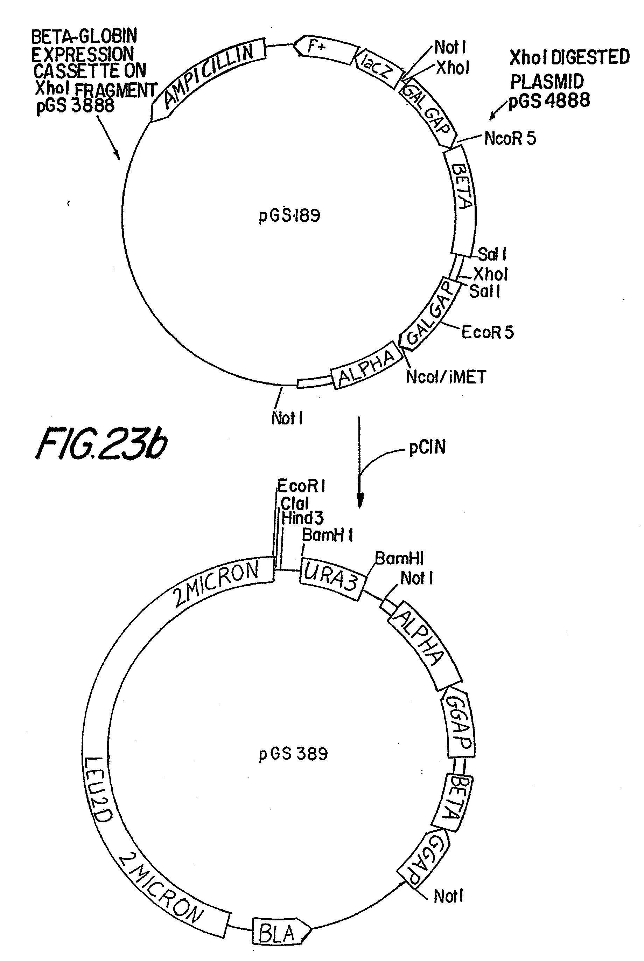

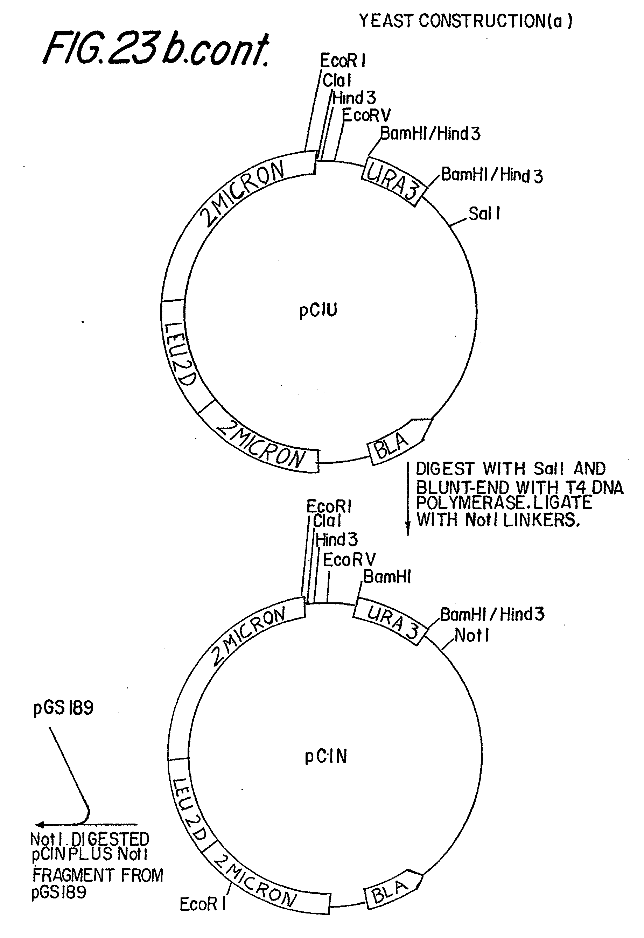

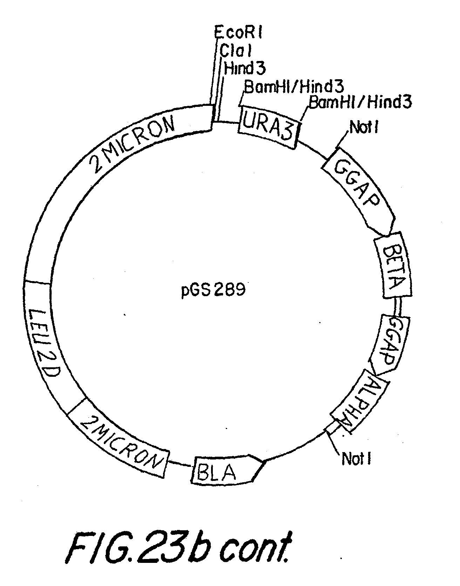

The plasmid pSK(+) (Stratagene, Inc.) (Figure 23(a)) was modified by digesting 100ng of purified plasmid DNA with KpnI. After digestion was complete, the DNA was ethanol precipitated and the dry pellet dissolved in 0.05ml T4 DNA polymerase buffer containing 10 units of T4 DNA polymerase. The reaction mixture was incubated at 37°C for 20 min, Na₃EDTA (10mM) was added and the sample heated to 70°C for 10 min. The digested DNA was precipitated with ethanol and the dry pellet dissolved in 0.01 ml of 10mM Tris HCl (7.8) 1mM EDTA. A portion of this material (20ug) was dissolved in 0.005ml of ligase buffer containing 10 units of T4 DNA ligase and 50ng of phosphorylated NotI linkers. The ligation mixture was incubated at 25°C for 2 hr and a portion used to transform E.coli DH5α. Ampicillin resistant colonies were selected on LB-ampicillin medium. Plasmid DNA was isolated, digested with Not1 and analyzed by acrylamide gel (5%) electrophoresis. A plasmid containing the additional NotI site is expected to generate a new, 90bp fragment. Such a plasmid was identified and designated pSN(+) (Figure 23(a)).

### CLONING THE pGGAP-α-GLOBIN EXPRESSION CASSETTE INTO PSN(+)

pSN(+) was digested with SalI, phenol-chloroform extracted and ethanol precipitated. The precipitated DNA was dissolved (50ug/ml) in 1mM Tris-HCl (pH 7.8), 0.1mM EDTA. pGS4088 was digested with XhoI and the ^{~} 1100bp fragment containing the α-globin expression cassette was isolated from an agarose gel. The purified fragment was dissolved (20ug/ml) in 0.1XTE. Twenty-five nanograms of SalI digested pSN(+) was mixed with 50ng of the gel-purified α-globin fragment in 0.01ml ligase buffer containing 10 units of T4 DNA ligase. The ligation mixture was incubated for 1.5 hr at 25°C and a portion used to transform E.coli DH5α. Ampicillin resistant clones were isolated. One hundred transformants were transferred, in a grid pattern, to fresh plates. Replicas of the grid were made on a nitrocellulose filter and prepared for colony hybridization (R.W. Davis et al. Adv Bacterial Genetics, Cold Springs Harbor, NY, 1980.). The hybridization probe was oligonucleotide 4 (Table 6). This oligonucleotide (20pM) was labelled with ³²P-ATP in a 0.02ml reaction mixture containing 0.050M Tris-HCl (pH 7.6), 0.01M MgCl₂, 0.005M DTT, 0.0001M spermidine, 0.0001M EDTA, 50pM of ³²P-ATP and 10 units of T4 polynucleotide kinase. The reaction mixture was incubated for 2hr at 37°C, unincorporated ATP was removed by spin-column chromatography (BIORAD) used according to the manufactures instructions. The filters were incubated (37°C) in hybridization solution (6XSSC, 50% formamide, 2% SDS, 20pMoles of ³²P labelled probe) for 16 hr. The filters were washed 4 times (15 min each) with 1X SSC, 0.1% SDS at 55°C; twice (5 min each) with 0.2XSSC, 0.1% SDS at 50°C and twice (5 min each) with 0.2XSSC. Autoradiographs were made of the filters. Colonies that produced a hybridization signal were used to prepare plasmid DNA. This DNA was digested with EcoR1 and the fragments produced by this digest were analyzed by agarose gel electrophoresis. A plasmid containing a fragment of the correct size was identified and designated pGS4888 (Figure 23(a)).

### CLONING THE PGGAP-β-GLOBIN EXPRESSION CASSETTE INTO pGS4888

The construction of pGS4888 required cloning an XhoI fragment (pGGAP-α-globin) into a SalI site. The combination of a XhoI site with a Sail site destroys the recognition sites for both XhoI and SalI restriction endonucleases, leaving pGS4888 with a single unique XhoI site. An XhoI fragment from pGS3888 was purified by agarose gel electrophoresis and cloned into XhoI digested pGS4888 essentially as described for the construction of pGS4888. Colony filter hybridizations were performed using the NcoI-SalI fragment (containing β-globin cDNA) from pGS3888 as a hybridization probe. This fragment was purified by agarose gel electrophoresis and radioactively labelled with α³²P-dCTP using an Amersham random oligonucleotide labelling kit. Filters were hybridized, washed and autoradiographs made as described for the construction of pGS4888. Plasmid DNA was prepared from colonies producing a hybridization signal and digested with either NcoI or NcoI and SphI. These digests allow identification of plasmids that contain both the α- and β-globin expression cassettes and reveals the relative orientation of the α- and β-globin transcriptional units. A plasmid containing both transcriptional units was identified and given the designation pGS189 (Figure 23(b)).

### CONSTRUCTION OF PLASMID pC1N

The plasmid pC1U was modified to introduce a NotI site. pC1U DNA (100ug) was digested with SalI, phenol-chloroform extracted and ethanol precipitated. The SalI ends were modified with T4 DNA polymerase to produce "blunt" ends and phosphorylated NotI linkers were added. The procedures used for these modifications are essentially the same as those used to produce pSN(+) (described above). The plasmid resulting from these manipulations is called pCIN (Figure 23(b)). This pC1N was digested with Notl, phenol-chloroform extracted and ethanol precipitated. A ^{~} 2.4kb NotI fragment (carrying α- and β-globin expression cassettes) was purified from NotI digested pGS189 DNA by agarose gel electrophoresis. Fifty nanograms of NotI digested pC1N was mixed with 50ng of the gel purified, 2.4 kb fragment isolated from pGS189 in 0.01 ml of ligase buffer containing 10 units of T4 DNA ligase. The reaction mixture was incubated at 25°C for 2hr and a portion used to transform E.coli DH5α. Ampicillin resistant clones were selected on LB-ampicillin plates and plasmid DNA prepared. The purified DNA was digested with EcoRl and analyzed by agarose gel electrophoresis to identify plasmids carrying the α- and β-globin genes and to determine the orientation of the insert with respect to the vector. Two plasmids were identified, representing the two possible orientations and have been designated pGS289 (Figure 23(b)) and pGS389 (Figure 23(b)).

### EXPRESSION OF RECOMBINANT HUMAN HEMOGLOBIN IN SACCHAROMYCES CEREVISIAE

S. cerevisiae strains GSy112 (MATαpep4::H1S3prb1 1.6R his3 200 ura3-52 leu2::hisG can1 cir°) and RSY334 (MATα- reg1-501 pep4-3 prb1-1122 ura3-52 leu2-3, leu2-112) were transformed with plasmids pGS289 or pGS389 by the method of Ito et al. (J. Bacteriology 153:163-168 (1983)). Transformants were selected on yeast SD-ura. Single colony isolates were picked and streaked to SD medium lacking uracil and leucine. Colonies from this selective medium (SD-ura, -leu) were used to inoculate 2ml of SD-ura,-leu, the cultures were incubated at 30°C for 24hr and used to inoculate 20ml cultures of YP + 3% ethanol, these were incubated for an additional 24hr and galactose was added to a concentration of 2%. Samples were removed at 4, 8, 24, 28, 32 and 48 hours post induction. Cells were collected by centrifugation and washed with 1 ml 10mM Tris HCl (pH 7.8), 1mM EDTA and resuspended in SDS-PAGE sample buffer (2 x 10⁸ cells/ml). Samples were boiled for 10 min and the insoluble material removed by centrifugation, 0.005ml samples were analyzed by SDS-PAGE (12.5% gel) followed by transfer to nitrocellulose. α- and β-globin chains- were stained using commercially available rabbit anti-human hemoglobin and an immunoblotting kit purchased from Promega. The protocols used were supplied by Promega. The immunoblot indicated that both chains are synthesized and that pGS289 produces slightly less material than pGS389. An apparent lack of stoichiometry between the α-chain (lower band) and β-chain is due to a difference in immunoreactivity of the antibody to the two chains. This was demonstrated by comparing Commassie brilliant blue stained gels of purified human and recombinant hemoglobin with immunoblotted samples.

### Example 20: Characterization Of Human Recombinant Hemoglobin Synthesized in Yeast

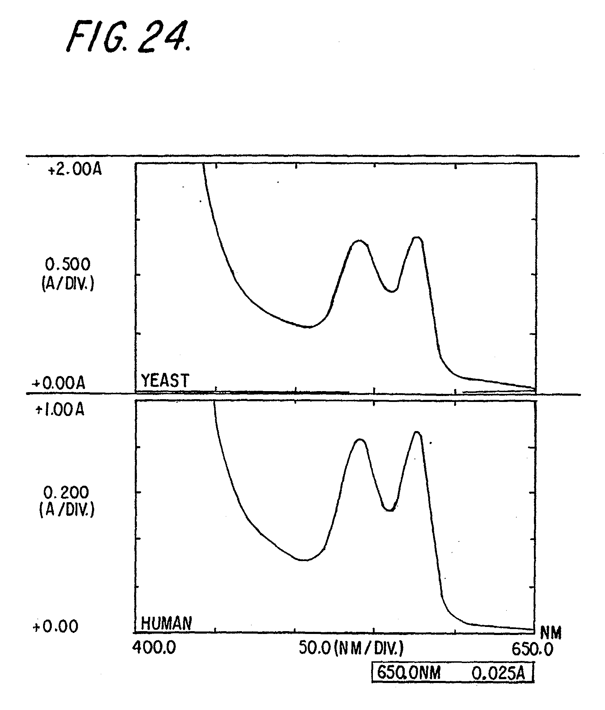

RSY334[pGS389] was grown in 100ml SD-leucine to an OD₆₀₀ of 2.4. This was used to inoculate 1L of YPE. This culture was shaken at 30°C, for 24hr at which time 50ml of 40% galactose was added. The incubation was continued and the cells harvested 24 hr later (OD₆₀₀ of 9). The cell pellet was resuspended in 100ml of 0.01M Tris-HCl (pH 7.18), 0.001M EDTA and carbon monoxide bubbled through the cell suspension for 3 min. The cells were collected by centrifugation and after the CO treatment were distinctly red. The cell pellet (19 gm) was resuspended in 19ml of lysis buffer (0.01M NaPO₄ pH 6.0, 0.020M DTT, 1% Triton X 100, 0.001M PMSF, 0.005M benzamidine and 0.06mM leupeptin) and bubbled with carbon monoxide for 2 min. The cell suspension was sonicated with Branson 250 sonicator equipped with a 0.5 inch disrupter horn. The sonication time was 2 min (0.5 sec pulses) at full power. This was repeated 4 times with 2 min cool-down periods between sonication intervals. The cell debris was removed by centrifugation (27,000 x g for 15 min) and the supernatant saved (0°C). The pellet was resuspended in 5ml of lysis buffer. The resuspended pellet was sonicated with the microtip as described above (70% of maximum output). The cell debris was removed by centrifugation as described above and this supernatant combined with the first. The combined solutions were clarified by centrifugation (38,000 x g, 20 min), producing a clear, red solution. This was loaded (after adjusting the pH to 6.0, with 10 mM phosphoric acid) onto a 5ml S-Sepharose fast flow column equilibrated with 0.01H sodium phosphate (pH 6.0). All of the red material bound to the column and the column was washed with 20ml of 0.01M sodium phosphate (pH 6.0). The column was eluted with 0.05M sodium phosphate (pH 7.5), 0.1M NaCl and 1ml fractions were collected. The red color eluted in two fractions. The purity of this material was analyzed by SDS-PAGE and appears to be ≥ 50% pure after the first chromatography step. This material was dialyzed against 10mM sodium phosphate (pH 6.0), 0.001M EDTA for 16 hr at 0°C and rechromatographed on a mono-S FPLC column (ph 6.8 - pH 9.0, 0.02M sodium phosphate gradient). The peak fraction from this is ≥ 85% pure. An absorbtion spectrum was obtained by scanning the mono-S purified material from 400mM to 650mM with a Shimadzu spectrophotometer (Figure 24, top). The spectrum obtained was identical to that of human hemoglobin (Figure 24, bottom), indicating that the protein had folded and incorporated heme. The amount of hemoglobin recovered in the two peak fractions was determined from the extinction coefficient (1.23 x 10⁴ A450/M) to be ^{~} 20mg.

### Example 21: Construction of Vectors for the Expression of a-and β-Globin from Separate Yeast Plasmids

In addition to the development of a single yeast vector that carries both α- and β-globin expression cassettes we also developed a system that uses separate plasmids for each of the two globin cDNA's. The two plasmids each carry two yeast genes that are used to maintain the plasmid in yeast. Both have the LEU2 gene in common and one (pGS4688) has the URA3 gene, the other (pGS4988) has the TRP1 gene. By using a host that carries mutations in URA3, LEU2 and TRP1 both plasmids (one with α-globin and the other with the β-globin expression cassette) can be maintained. The constructions of these vectors is described below.

### CONSTRUCTION OF pC1T

The plasmid pC1U (5ug) was digested with BamHI and SalI and the largest fragment was purified by agarose gel electrophoresis. The plasmid YRp7 (10ug) (J. Strathern, E. Jones, and J. Broach, The Molecular Biology of the Yeast Saccharomyces, Cold Spring Harbor, NY, 1981) was digested with BglII and SalI and the fragment containing the TRPI gene was purified by agarose gel electrophoresis. Twenty-five ng of gel-purified, BamHI and Sail digested pC1U was mixed with 50ng of the BglII-SalI fragment in ligase buffer containing 10 units of T4 DNA ligase. The reaction mixture was incubated at 25°C for 1.5 hr and a portion of the ligation reaction mixture used to transform E.coli DH5α. Tetracycline resistant colonies were selected on LB-tetracycline medium. Plasmid DNA was prepared from 15 transformants, digested with EcoR1 and analyzed by agarose gel electrophoresis. One isolate with the expected EcoR1 restriction fragments was chosen and was designated pC1T (Figure 22(a)).

### CLONING THE β-GLOBIN EXPRESSION CASSETTE IN pC1T

One hundred nanograms of pC1T was digested with Sall, phenol extracted, ethanol precipitated and dissolved in 0.01ml of 10mM Tris-HCl (pH 7.8), 1mM EDTA. The plasmid pGS3888 was digested with XhoI and the 1.2 kb XhoI fragment containing the β-globin expression cassette was purified by agarose gel electrophoresis. Ten nanograms of Sail digested pC1T was mixed with 60ng of the XhoI fragment containing the β-globin expression cassette in 0.01ml of ligase buffer containing 10 units of T4 DNA ligase. The reaction mixture was incubated at 25°C for 30 min. A portion of this material was used to transform E.coli DH5α. One hundred ampicillin resistant transformants were picked and patched to LB-ampicillin agar. They were incubated for 5 hr at 37°C and overlayed with a nitrocellulose filter. Plasmids containing the XhoI fragment were identified by colony hybridization, using the XhoI fragment as a hybridization probe, as described above. Colonies producing an autoradiographic signal were used to prepare plasmid DNA. The purified DNA was digested with EcoR1 and analyzed by agarose gel electrophoresis for the presence of the expected restriction fragments. Two clones containing the desired XhoI insert were identified and the orientation of the insert was determined by agarose gel analysis of EcoR1 digests or SphI digest of the plasmid DNA. Both isolates contained the desired insert in the same orientation and one was designated pGS4988 (Figure 22(a)).

### CLONING α-GLOBIN EXPRESSION CASSETTE IN pC1U

The Xho1 fragment from pGS4088 containing the α-globin expression cassette was purified by agarose gel electrophoresis and the recovered fragment dissolved (100ng/0.01ml) in 1mM Tris-HCl ph 7.8, 0.1mM EDTA. Twenty-five nanograms of the gel purified fragment was mixed with 10ng of Sail digested (phenol-chloroform extracted, ethanol precipitated) pC1U in 0.01 ml ligase buffer containing 10 units of T4 DNA ligase. The reaction mixture was incubated at 25°C for 1. 5 hr and a portion used to transform E.coli DH5α. Ampicillin resistant colonies were selected on LB-ampicillin plates. Plasmid DNA was prepared from 12 transformants, digested with HindIII and analyzed by agarose gel electrophoresis. The two possible orientations were isolated and given the designation pGS4488 (Figure 22(b)) and pGS4688 (Figure 22(b)).

### CONSTRUCTION OF DIPLOID STRAINS EXPRESSING α- AND β-GLOBIN CHAINS FROM SEPARATE PLASMIDS

S. cerevisiae RSY330 (MAα pep4-3 prb1-112 hist7 ura3-52 trp1-289 can1 gal1) was transformed (Ito et al.) with pGS4488 or pGS4688. Trp+ transformants were selected on SD-trp medium and streaked for single colonies. S. cerevisiae BJY1991 (MATα prb1-112 pep4-3 leu2-3,112 trp1- 101 ura3-52 gal2 can1) was transformed with pGS4988. URA⁺ transformants were selected on SD-ura medium and streaked for single colonies. These strains were each tested for the production of α- and β-globin as follows: (1) Single colonies were picked from SD-selective medium and used to inoculate 2ml of SD-selective (liquid) medium. The cultures were incubated for 24 hr at 30°C and diluted into 25ml of fresh SD-selective medium and incubated for an additional 24 hr. The cells were collected by centrifugation and resuspended in 25ml of YP-galactose (2%) and the incubation continued for an additional 24 hr. The cells were harvested (8,000 x g, 10 min) and the pellet washed with 50ml 0.010M TrisHCl (pH 7.8), 0.001M EDTA. The pellets were dissolved by heating to 96°C for 10 min in SDS-PAGE sample buffer and the debris removed by centrifugation (15,000 x g, 10 min). The cleared supernates from 1x10⁶ cells each were analyzed by SDS-polyacrylamide gel electrophoresis and Western immunoblotting as described above. β-globin was readily detectable in extracts from BJY3505[pGS4988]. We were also able to detect α-globin cross-reacting material, although the signal strength was considerably weaker. The production of tetromeric hemoglobin requires the presence of both α and β chains, ideally these would be expressed in the same cell. Because strains BJY3505, BJY1991 and RSY330 are haploids they each can be mated with a yeast strain of the opposite mating type. Strains RSY330 and BJY1991 are both mating type α, whereas BJY3505 is mating type a. BJY3505[pGS4988], RSY330[4688] or BJY191[4688] or RSY330[pGS4688] matings were done and diploids selected by streaking onto SD minimal medium with no additional amino acids or other nutrients. Neither of the plasmid-bearing parental strains are capable of growth on this medium, diploids, however, can grow. Because the diploids are homozygous for the mutations in TRP1 and URA3 both plasmids must be present for the cells to grow in the absence of these nutrients. These diploid strains were analyzed for the synthesis of α and β-globin as described above. A most surprising result was obtained. Although α-globin and β-globin are synthesized at low levels in the haploid strains, co-expression in a diploid strain results in a substantial increase in the levels of both chains. Furthermore, after induction (24 hr) with galactose the cell pellets develop a distinct, pink-red color. These results suggest that: (a) co-expression of α-and β-globin stabilizes the two proteins, perhaps as a consequence of their interaction and (b) the protein is apparently folding and incorporating heme.

### Example 22: Oxygen Binding Properties of Yeast-Derived Hemoglobin

The P₅₀ of the yeast derived hemoglobin is nearly identical to that of Hgb Ao. Depending on how we measure it, the P₅₀ ranges from about 5 to 10 torr in a phosphate free solution. At 25°C it is about 4-6 torr in 50mM BisTris, pH 7.4, NaCl 0.1M. In the same solution, at 37°C, the P₅₀ is from 8.5 to 11.

### Example 23: Expression of Di-Alpha Hemoglobin in S. Cerevisiae

### Methods

Unless stated otherwise, all enzymes (restriction endonucleases, T4 DNA ligase, T4 DNA polymerase, T4 polynucleotide kinase) were purchased from New England Biolabs, Pharmacia, BRL, Stratagene or Boerhinger Mannheim. Restriction enzymes and T4 DNA ligase were used with the buffers supplied by the manufacturers.

Ethanol precipitation of nucleic acids was carried out by the addition of 0.5 volumes of 7.5M ammonium acetate and 2 volumes of 20:1 ethanol-isopropanol. The pellet was collected by centrifugation at 14,000 x g for 15 minutes, washed twice with 80% ethanol, once with 95% ethanol and dried in vacuo. Phenol extractions were done by the addition of 50:49:1 mixture of phenol:chloroform:isoamyl alcohol. Phases were separated by centrifugation at 14,000 x g and the aqueous phase collected. Plasmid DNA was purified from E.coli DH5α as described by Birnborn and Doly (Nucleic Acids Research 1979, 7:1513-1520). Electrophoretic analysis of DNA was carried out in agarose gels using tris-acetate electrophoresis buffer (Maniatis, et al. Molecular Cloning, Cold Springs Harbor, NY, 1982). DNA was visualized by staining the gels with 0.5µg/ml ethidium bromide and exposing the gels to ultraviolet light. DNA fragments were purified from agarose gels using a kit purchased from BIO-101. DNA fragments were purified from acrylamide gels by crushing the excised gel fragment in 3.25M ammonium acetate and incubating overnight at 37°C. Gel fragments are removed by centrifugation (14,000 x g, 15min) and the DNA precipitated with ethanol. The precipitate is dissolved in TE (10mM Tris HCl, pH7.8, 1mM Na₃ EDTA). Acrylamide gel electrophoresis of DNA was done as described by Maniatis, et al. (Molecular Cloning, Cold Spring Harbor Laboratory, NY, 1982). Bacteriological growth media and DNA transformation methods are as described by R.W. Davis, et al. (Advanced Bacterial Genetics, Cold Spring Harbor Laboratory, NY, 1980). Media for the growth of S.cerevisiae has been described by F. Sherman et al. (Methods in Yeast Genetics: A Laboratory Manual, Cold Spring Harbor Laboratory, NY, 1979). Transformation of S. cerevisiae with linear or circular DNA was carried out as described by H. Ito, et al. (J. Bacteriology, 153:163-168(1983)).

Removal of the PstI and SpeI sites from pGS4888. The design of the synthetic linker for joining two α-globin chains allows the inclusion of PstI and SpeI sites flanking a 30bp sequence that includes the junction of the two α-globin coding sequences. Because we anticipate testing several different linker sequences, these sites will allow directional cloning of relatively short synthetic oligonucleotides encoding different linker sequences. Removal of the PstI and SpeI sites from the vector sequence is, therefore, necessary so that the sites in the coding region are usable. One µg of the plasmid pGS4888 was digested with PstI and ethanol precipitated. The dry pellet was resuspended in 50µl of 33mM Tris-acetate, pH7.9, 66mM potassium acetate, 10mM magnesium acetate, 0.5mM DTT and 50 µM of each dNTP (T4 polymerase buffer). Two units of T4 DNA polymerase were added and the reaction mixture incubated for 15min at 37°C. Na₃ EDTA was added to 12.5mM and the reaction mixture heated to 65°C for 15min, phenol extracted and ethanol precipitated. The dry pellet was dissolved in 14 µl of T4 DNA ligase buffer (BRL) and 1µl (10 units) of DNA ligase added. The ligation mixture was incubated a 4°C for 16hr. A portion of the ligation reaction was used to transform E.coli DH5α and transformants were selected on LB-ampicillin plates. Plasmid DNA was prepared from 12 transformants. The DNA was analyzed by agarose gel electrophoresis of PstI digests. Five transformants had lost the PstI site and one of these was designated pGS1889. The SpeI site of this plasmid was removed as described above after digestion of pGS1889 with SpeI. A plasmid was identified that had lost both the PstI and the SpeI site and was designated pGS1989.

Strategy for Joining Two Copies of the α-globin cDNA. A fragment containing the 5'-363bp of the α-globin coding region can be excised from pGS4888 as an NcoI to ApaL1 fragment. A second fragment containing nucleotides 109 through the 3' untranslated region can be removed as a FokI to Sa1I fragment. These two fragments can then be joined together to create a single translation unit encoding two tandem copies the of α-chain cDNA by using a synthetic oligonucleotide adaptor (Table 8). The purification and assembly of these fragments is described below.

The plasmid pGS4888 was sequentially digested with ApaL1 and NcoI and a ^{~}365bp fragment (α-chain 1) purified by acrylamide gel electrophoresis. A second fragment of ^{~}351bp (α-chain 2) was prepared by sequential digest with FokI and Sail followed by acrylamide gel electrophoresis. Four oligonucleotides were synthesized (Table 8) and assembled into a ^{~} 173bp linker (RGGV) containing an ApaL1 and FokI ends. Synthesis, purification and assembly of oligonucleotides was as described previously. This adaptor encodes an amino acid bridge linking the carboxy-terminus of α-chain1 with the amino-terminus of α-chain2 as well as portions of the 3' end of α-chain1 and the 5' end of α-chain2. The carboxy terminal arginine residue of α-chain1 is separated from the amino terminal valine of α-chain2 in this construct by 2 glycine residues. The "diglycine bridge" portion is flanked by HpaI, SpeI and PstI sites. These sites allow the substitution of a variety of bridges by the use of ^{~} 30bp adaptors to connect the two α-chains into an expression cassette was carried out in a four part ligation as described below.

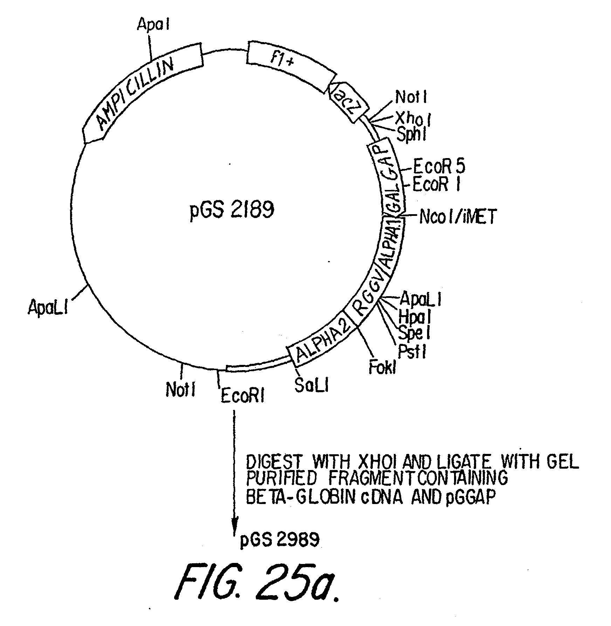

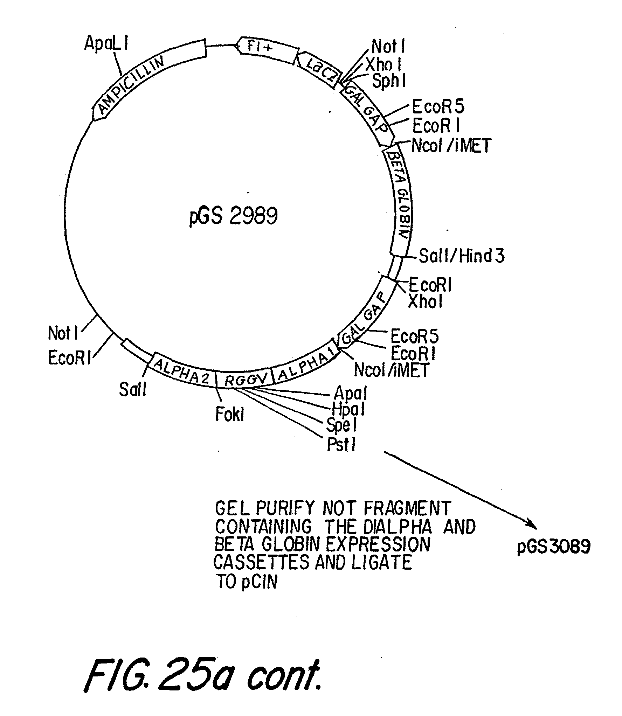

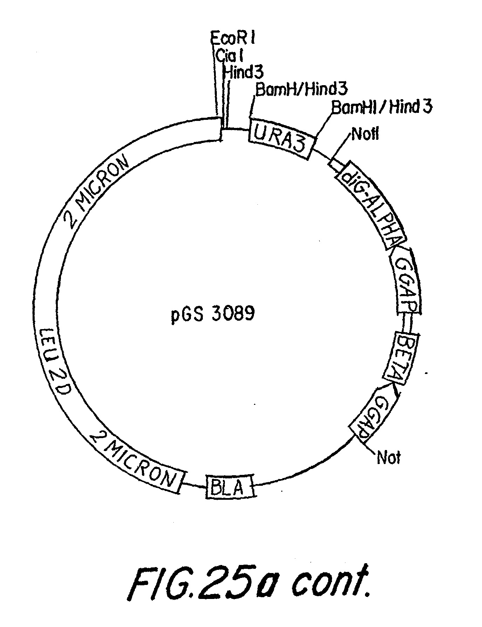

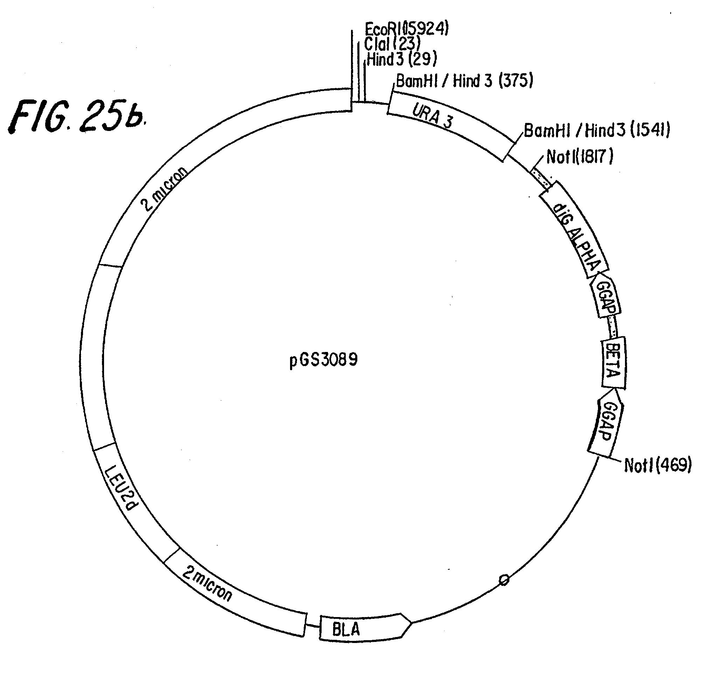

Plasmid pGS1989 was sequentially digested with NcoI and Sail and the large fragment containing the plasmid vector and pGALGAP was purified by agarose gel electrophoresis (gp1989). Fifty nanograms of gp1989 were mixed with 200ng each of the gel purified ApaL1-NcoI α-chain1 fragment and the FokI-Sa1I α-chain2 fragment. A twenty-fold molar excess of the synthetic ApaL1-FokI adaptor was added (the 5'-ends of the adaptor segment are not phosphorylated). The ligation reaction was carried out in a volume of 20µl for 4hr at 23°C. A portion of this reaction mixture was used to transform E. coli DH5α and ampicillin resistant colonies were selected. Transformants were patched to nitrocellulose filters and screened by hybridization with the ³²P-labelled oligonucleotide AL2as (Table 8). Five pMoles of oligo AL2-as were incubated in 20µl of a solution containing 2 units of T4 polynucleotide kinase, 50mM Tris-HCl (pH7.6), 10mM MgCl₂, 6mM DTT, 0.1mM spermidine, 0.1mM EDTA, and 10pMoles of -γ³²P-ATP (7000 Ci/mMole) for 2hr at 37°C. Filters were processed as described by Maniatis, et al. and the hybridization was done at 37°C in 5XSSC, 50% formamide, 100µg/ml yeast tRNA and 2% SDS for 16hr. The filters were washed, sequentially, in 2XSSC and 1XSSC at 55°C for 15min (twice in each, all wash solutions contained 1% SDS). Dried filters were exposed to X-ray film and colonies giving a hybridization signal were used to prepare plasmid DNA. Plasmid DNA was analyzed by restriction enzyme digestion to identify plasmids that contained inserts of a size consistent with two α-chains (NcoI-Sa1I digest) and that contained unique PstI, SpeI and HpaI sites. A plasmid identified in this manner was designated pGS2189 (Figure 25).

An XhoI fragment from pGS3888 containing the β-globin expression cassette was purified by agarose gel electrophoresis. XhoI digested pGS2189 (50ng) was combined with 150ng of the gel purified insert from pGS3888 in 1µl of ligation buffer containing 10 units of T4 DNA ligase. A portion of this mixture was used to transform E.coli DH5α and ampicillin resistant colonies were selected. Plasmid DNA was isolated and analyzed by digestion with XhoI, BamHI or NcoI. Several plasmids were identified that produced restriction fragments of the expected sizes, all contained inserts in the orientation shown in Figure 25. One of these was designated pGS2989. Although this plasmid contains the linked α-globin genes and a β-globin gene under the control of separate promoters, it is not capable of replication in S.cerevisiae. The entire expression cassette, containing the two genes (diα and β-globin), can be purified as a NotI fragment. Ten µg of pGS2989 was digested with PvuI and NotI and the NotI fragment gel purified. The digestion with PvuI was done to reduce the size of the vector sequences which otherwise would congrate with the desired NotI fragment. Two hundred ng of the gel purified NotI fragment was combined with 50ng of NotI digested pC1N in 10µL of ligation buffer containing 10 units of T4 DNA ligase. The reaction mixture was incubated at 4°C overnight and a portion was used to transform E.coli DH5α. Ampicillin resistant transformants were selected and plasmid DNA prepared. DNA was digested with NcoI-SalI, PstI or NotI to identify plasmids with the diα, β-globin expression cassette and to determine the orientation of the inserted fragment. Several plasmids were identified that contained the correct insert, all of which have the inserted fragment in the same orientation. One of these was designated pGS3089 (Figure 25). This plasmid was used to transform strains GSY112 and RSY334.

### Expression and Purification

S.cerevisiae strains GSY112[pGS3089] and RSY334 [pGS3089] were grown to saturation in SD-uracil medium and diluted into 2L of YPD medium (all cultures were incubated at 30°C). Twenty-four hours after inoculation (of the YPD culture), galactose was added to 1% and the cultures incubated for another 24hr. Carbon monoxide was bubbled through the culture and the cells collected by centrifugation. A 1:1 mixture (weight:vol) of cells and breakage buffer (10mM sodium phosphate (pH7.2), 1mM EDTA, 1mM EGTA and 5mM DTT) were disrupted in a "Bead-Beater" (Biospec Products, Bartlesville, OK). Debris was removed by centrifugation (10,000 x g, 30min). The soluble fraction was adjusted to pH6.0 with phosphoric acid and chromatographed on a column of S-sepharose (fast flow) equilibrated in 10mM sodium phosphate pH6.0. The loaded column was washed with 10mM Tris-HCl, pH6.7 and hemoglobin eluted by washing with 20mM Tris-HCl, pH7.5. A bright red band was collected and the pH adjusted to 8.0, by the addition of NaOH. This material was then chromatographed on Q-sepharose (fast flow) equilibrated in 20mM Tris-HCl, pH8.0. Hemoglobin was eluted with a NaCl gradient (0-0.4M). A final chromatography step was carried out on Sephacryl S-200 equilibrated in 5mM NaPO₄, pH7.4, 0.1M NaCl. Each step of the purification protocol was analyzed by SDS-polyacrylamide gel electrophoresis (Laemmli, U.K., 1970, Nature 227:680-685) and staining with Commassie brilliant blue. The purified protein contains a band that comigrates with monomer β-globin and a band in the expected position for α-globin dimer. This material comigrates with human tetrameric hemoglobin when analyzed by size exclusion chromatography (Progel TSK G3000 SWXL HPLC column). This protein is red and binds O₂ with a 50% binding affinity (P₅₀) of 8-10 Torr, indicating that it has incorporated heme and is capable of reversible O₂ binding.

The purified protein was separated into α- and β-chains by reverse phase HPLC and the sequence of the 10 amino-terminal residues of each chain were determined. The sequence matched that of bona fide human hemoglobin, indicating that the initiating methionine had been efficiently removed from both the α-globin dimer and the β-globin dimer.

### Example 24: Construction of Low Affinity, Genetically Cross-Linked, Hemoglobin Mutants and Expression in Yeast

### Construction of vector for site directed mutagenesis.

The XhoI fragment containing the β-globin gene and its GALGAP promoter waas isolated from pGS2989 by preparative agarose gelelectrophoresis and ligated with XhoI digested Phagescript (RF-form, obtained from Stratagene, Inc.). E. coli XL1-Blue was transformed with the DNA ligation mixture and phage containinginserts were identified (white plaques on medium containing XGAL). Single plaques were isolated and DNA was prepared and analyzed by digestion with XhoI and agarose gel electrophoresis. This construct was designated Mpβ-globin.

Preparation of single stranded template. A saturated culture of E. coli XL1-Blue (available commercially from Stratagene, 11099 North Torrey Pines Road, LaJolla, CA 92037) (200 µl) was used to inoculate 4 ml of 2X YT broth. This culture was incubated for 2 hr. at 37°C and then infected with a single M13β-globin phage plaque and the incubation continued for 6-8 hrs. Cells were removed by centrifugation and discarded. Phage were precipitated from 1.6ml of clarified medium by the addition of 320 µl of cold 30% PEG 8000 in 3.5M ammonium acetate followed by incubation on ice for 30 min. Phage were collected by centrifugation and resuspended in 0.1 ml of 10mM Tris-HCl pH 8.0, 1mM EDTA (TE). DNA was isolated by extracting twice with phenol/cholorform. DNA (contained in the aqueous phase) was precipitated by the addition of NaCl to 0.5M and two volumes of 95% ethanol. The DNA pellet is dissolved in 20µl of water.

In vitro mutagenesis reactions. Two hundred ng of template DNA are mixed with a twenty-fold molar excess of the appropriate, phosphorylated mutagenic oligonucleotide in 10 µl of 20mM Tris-HCl, pH 7.4, 2mM MgCl₂, 50mM NaCl and heated to 70 °C for 5 minutes. The annealing reaction is allowed to slowly cool (40 min) to 40°C and then to 30°C (15 min). After the last annealing step the mixture is transferred to ice for 5 min. To this mixture 1µl of synthesis buffer (4mM each dNTP, 7.5mM ATP, 175mM Tris-HCl pH 7.4, 37.5mM MgCl₂ 215mM DTT), 0.5 µl of T4 gene32 protein (2 µg/µl), 1µl of T4 DNA ligase (3 units), and 1 ml of T4 DNA polymerase (1 unit) are added. The mixture is incubated at 37°C for 90 min (following 5 min at room temperature). The reaction is stopped by the addition of 90ml of 0.1M Tris-HCl (pH 8.0) and 0.1M EDTA. Approximately 0.1µl of the reaction mix was used to transfect E. coli XL1-Blue cells (Cells were prepared for transformation by the method of D. Hanahan, 1983. J. Mol. Biol. 166:557).

Screening for phage containing the mutant gene. Approximately 50-100 plaques were picked to fresh plates seeded with the appropriate host strain (XL1-Blue) in ordered arrays. After incubation for 6-8 hrs. at 37°C the plates were overlaid with nitrocellulose filters and prepared for hybridization essentially as described in Davis, R.W, et al. (Advanced Bacterial Genetics: A Manual of Genetic Engineering. Cold Spring Harbor Laboratory, New York 1980). The choice of hybridization temperature with the mutagenic oligonucleotide was determined on the basis of the nucleotide composition of the oligonucleotide. The oligonucleotide was labelled with γ³²P-ATP and polynucleotide kinase (T. Maniatis et al. Molecular Cloning: A Laboratory Manual. Cold Spring Harbor Laboratory, 1982). Hybridizations were done at 2-5°C below the calculated Tm for the correct match in 6XSSC, 2%SDS, 100/µ/ml yeast tRNA using 10⁵ cpm/ml of the labelled oligonucleotide for >6 hr. The Tm was calculated using the formula: 4°C for every GC pair + 2°C for each AT pair assuming an Na+ concentration of 1M. Filters were washed at the same temperature as the hybridization in 5XSSC, 2% SDS and exposed to XRay film. Plaques giving positive hybridization signals were used to prepare single stranded DNA, as described above. The single-stranded DNA was used as template for sequencing reactions (Sanger, F. and Coulson, A.R. 1975, J. Mol. Biol. 94:441) to confirm that the mutant sequence was indeed present.

Oligonucleotides used for mutagenesis.

βN108K-"Presbyterian" 5'-AGGCTCCTGGGCAAGGTGCTGGTCTGT-3'

βE90K-"Agenogi" 5'-GCCACACTGAGTAAGCTGCACTGTGAC-3'

βV67I-(No Alias) 5'-CATGGCAAGAAAATCCTCGGTGCCTTT-3'

βN102T-"Kansas" 5'-GTGGATCCTGAGACTTTCAGGCTCCTG-3'

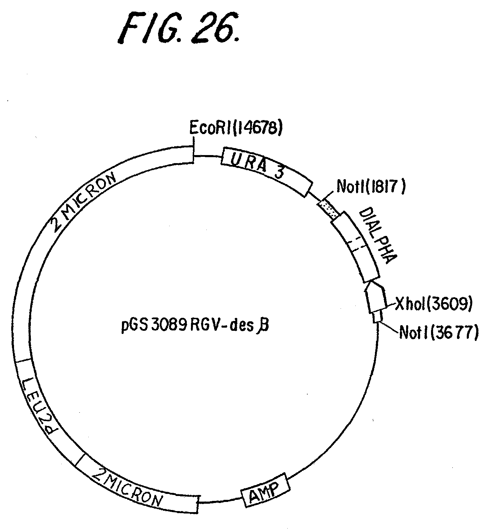

Cloning into yeast expression vectors. Phage RF DNA was prepared from phage-infected cells that had been confirmed to have the mutant sequence and the XhoI fragment containing the altered β-globin gene was purified by agarose gel electrophoresis. This fragment was cloned into a derivative of pGS3089 that had been altered to change the di-α-crosslink from the diGly configuration to a single glycine bridge, and from which the β-globin gene had been deleted, and designated pGS3889desβ (Figure 26). This created a unique XhoI site into which altered β-globin expression cassettes could be inserted, allowing coexpression with the α-globin dimer. The expression plasmids generated in this way (pGS3889 for "Presbyterian", pGS5189 for "Agenogi", pGS5689 for "Kansas" and pGS4989 for "βV67I", all identical to pGS3089 except for mutation in specified codon) were used to transform S. cerevisiae strains GSY112 and RSY334.

Characteristics of a genetically crosslinked low affinity mutant protein expressed in yeast. Cells bearing the plasmid pGS3889 (single gly bridge, βN108K alias "Presbyterian") were grown and hemoglobin purified as previously described. This material when analyzed for functionality was substantially "right-shifted" compared to the crosslinked protein with a wild type β -chain (P₅₀=23-25 for the mutant, with N=2.5).

### Example 25: Effect of Choice of Strain and Induction Temperature on Expression of Di-Alpha Hemoglobin in E. coli

Table 100 contains comparisons of di-alpha/beta fermentations. It also provides comparison of temperatures of induction. The column labeled "mg di-A+B" is total mg di-alpha and beta polypeptides per fermentation. The adjacent column "mg/OD-L" simply expresses the first column number on a cell density basis. The two columns labeled RHGB present total and cell density-corrected output of functional recombinant hemoglobin. The last column shows that in terms of final functional hemoglobin recovery, strain JM109 is preferable. Without binding ourselves to any theory, we believe that this difference has to do with the proteases expressed in different strains. It is interesting to note that of the two best JM109 runs, one induction was at 30° and one at 37° with roughly equivalent amounts of final functional Hb produced.

### Example 26: Construction of Genetically Cross-Linked α-Globin Dimers Connected By a Single Glycine or Proline Residue

The following synthetic adaptors for altering the diα-globin bridge were synthesized and purified as described in previous sections.

T S K Y R P V L S P A
5'-A ACT AGT AAG TAC AGA CCT GTT TTG TCT CCT GCA-3 RPV
3'-T TGA TCA TTC ATG TCT GGA CAA AAC AGA GG-5'
HpaI PstI

T S K Y R G V L S P A
5'-A ACT AGT AAG TAC AGA GGT GTT TTG TCT CCT GCA-3' RGV
3'-T TGA TCA TTC ATG TCT CCA CAA AAC AGA GG-5'
HpaI PstI

Complementary pairs of RPV and RGV oligonucleotides were combined (2.4µg of each oligonucleotide) in 0.05 ml of water. The two pairs of adaptors were precipitated (separately) by the addition of 2.µl of 4M NaCl and 0.158mL of 100% ethanol followed by centrifugation. The pellets were washed with 80% and 100% ethanol and dried. The adaptors were dissolved in 24µl of TE.

Cloning of the RBGV and RPV adaptors. The plasmid of pGS2989 was sequentially digested with the enzymes HpaI and PstI and the vector was purified after agarose gel electrophoresis. The digested plasmid was then ligated with a 10-fold molar excess of either the RGV or RPV adaptor (these adaptors were not phosphorylated) as previously described. A portion of the ligation mixture was used to transform E. coli DH5. Transformants were selected on LB plates containing ampicillin. Clones containing the new adaptor were identified by colony filter hybridization. The hybridization probes were either the RPV or RGV upper strands (as shown above). These proves were labelled with γ32P-ATP and T4 polynucleotide kinase. Filters were hybridized with the appropriate probes (10⁵ cpm/ml) at 37°C in a solution containing 6XSSC. 50% formamide, 2% SDS and 150µg/ml yeast tRNA. Filters were incubated for >12 hrs. and washed 4 times with 2XSSC, 2%SDS (250ml, 20min each) and exposed to X-ray film. Colonies that produced autoradiographic signals were used to prepare plasmid DNA which was sequenced using the following primer: 5'-AAGCTTCAGCACCGTATTCA-3' (α² seql). This primer was specifically designed to minimize homology with the corresponding sequence in the α1 subunit of the dimer and to maximize homology with sequences near the 5' end of the α2 subunit of the diα dimer. This allows sequence to be determined reading from the α region through the sequence that bridges the two α-domains without a background sequence reading from a similar sequence near the 5'-end of the α1-domain. The plasmids constructed in this way were designated pGS2989RPV(single proline crosslink) or pGS2989RGV (single glycine crosslink).

Cloning into yeast expression plasmids. The NotI fragments from either pGS2989RPV or pGS2989RGV that contain the hemoglobin expression cassette were purified by agarose gel electrophoresis and subcloned into pC1N that had been digested with NotI. Plasmid DNA from ampicillin resistant transformants was isolated and analyzed by digestion with NotI and agarose gel electrophoresis. These plasmids were designated pGS3089RPV or pGS3089RGV. A second set of plasmids was generated from these to facilitate the substitution of different β-chain mutants. These plasmids were generated by digestion with XhoI and religation under dilute conditions <1µg/ml). This favors deletion of the β-chain expression cassette. Plasmid DNA was isolated from ampicillin resistant transformants and the structures confirmed by digestion with NotI and XhoI (these plasmids have been designated pGS3089RGV-desβ, see Figure 26, and pGS3089RPV-desβ).

### Reference Example: Reconstitution of Recombinant Alpha-Globin and Recombinant Beta-Globin with Heme and chemical Reduction to Yield Artificial Hemoglobin

Conventional methods of preparing artificial hemoglobin are exemplified by the following procedure.

The lyophilized recombinant alpha and beta-globins (100 mg each) were individually dissolved in 8M urea/50mM Tris-Cl, pH 8.01/1mM EDTA/ 1mM DTT, diluted to a concentration of and incubated at room temperature for 3-4 hours. The alpha-globin was then diluted to 0.3mg/ml with chilled 20mM K₂HPO₄, pH 5.7/1mM EDTA/1mM DTT. Hemin (25 mg) was dissolved in 2.4mg 0.1M KOH, diluted with an equal volume of 1M KCN; this solution was then made 0.1mg/ml in hemin and 20mM K₂HPO₄, pH 6.7 with stock phosphate buffer. Hemin from this solution was added to a 2.8 molar excess to the chilled alpha-globin; and equal molar amount of beta-globin was added and the solution was dialyzed at 4°C overnight against 0.1M K₂HPO₄, pH 7.6/1mM EDTA/ 1mM KCN. The artificial Hb solution was concentrated by ultra-filtration using a PM-10 membrane (Amicon) and transferred into a 200ml screw-top test tube with a rubber septum. The hemoglobin solution was deoxygenated by evacuation and flushing with N₂, and then the solution was saturated with co. 100mM sodium dithionite solution was prepared anaerobically in a 20ml screw-top test tube with rubber septum. 4.5 equivalents of dithionite were added to the Hb solution with a syringe, and the mixture incubated on ice for 15 min. The Hb solution was gel-filtered against 10mM Na phosphate buffer pH 6.0 on a 4x40 cm Sephadex G-25 (fine) column. The colored solution was then applied to a 2x10 cm-52 (Whatman) column equilibrated with the same buffer and the chromatography was developed with a linear gradient of 500ml 10mM Na phosphate buffer pH 6.0 and 500ml of 70mM sodium phosphate buffer pH 6.9. CO was removed from Hb by photolysis under a stream of oxygen. Artificial Hgb prepared this way is isolated in only about 25% yield from the fusion peptides but shows native oxygen binding properties.

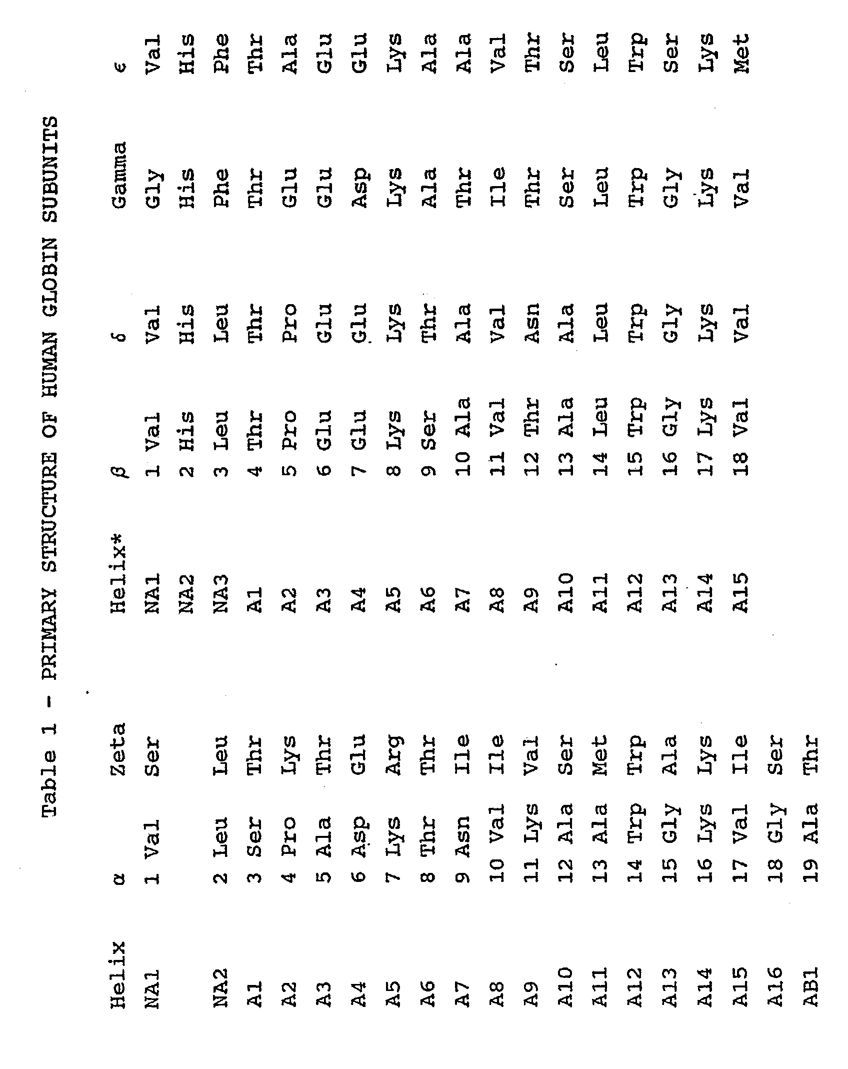

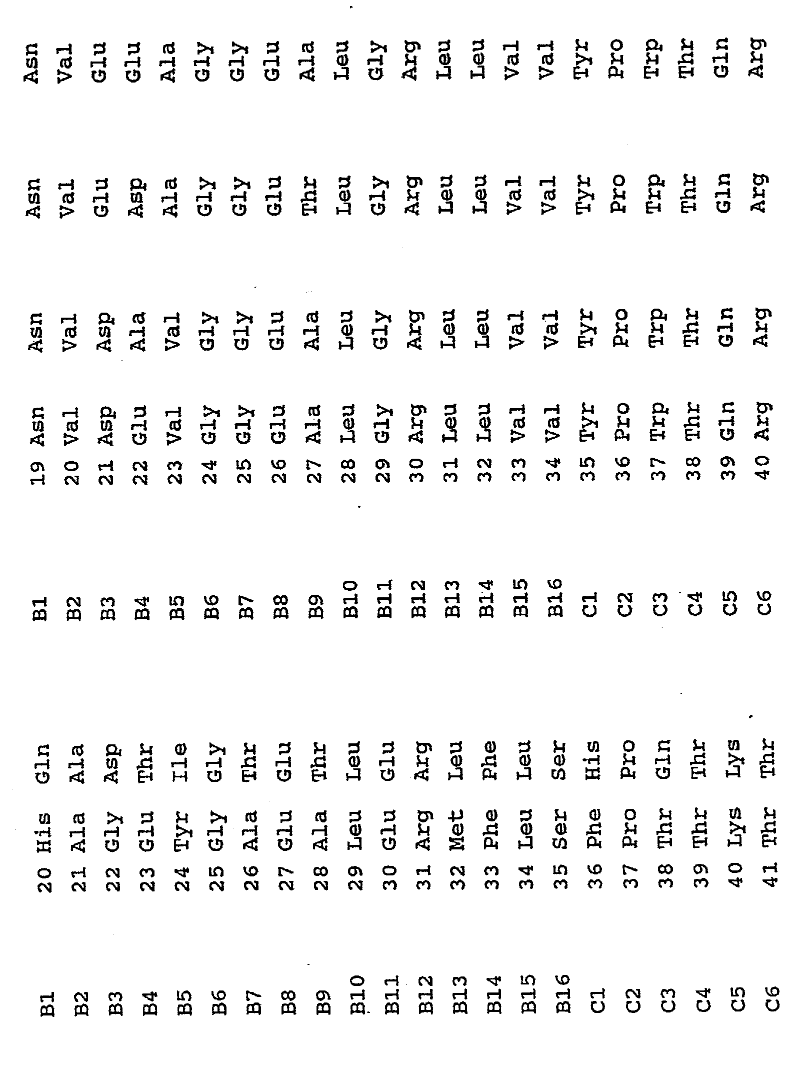

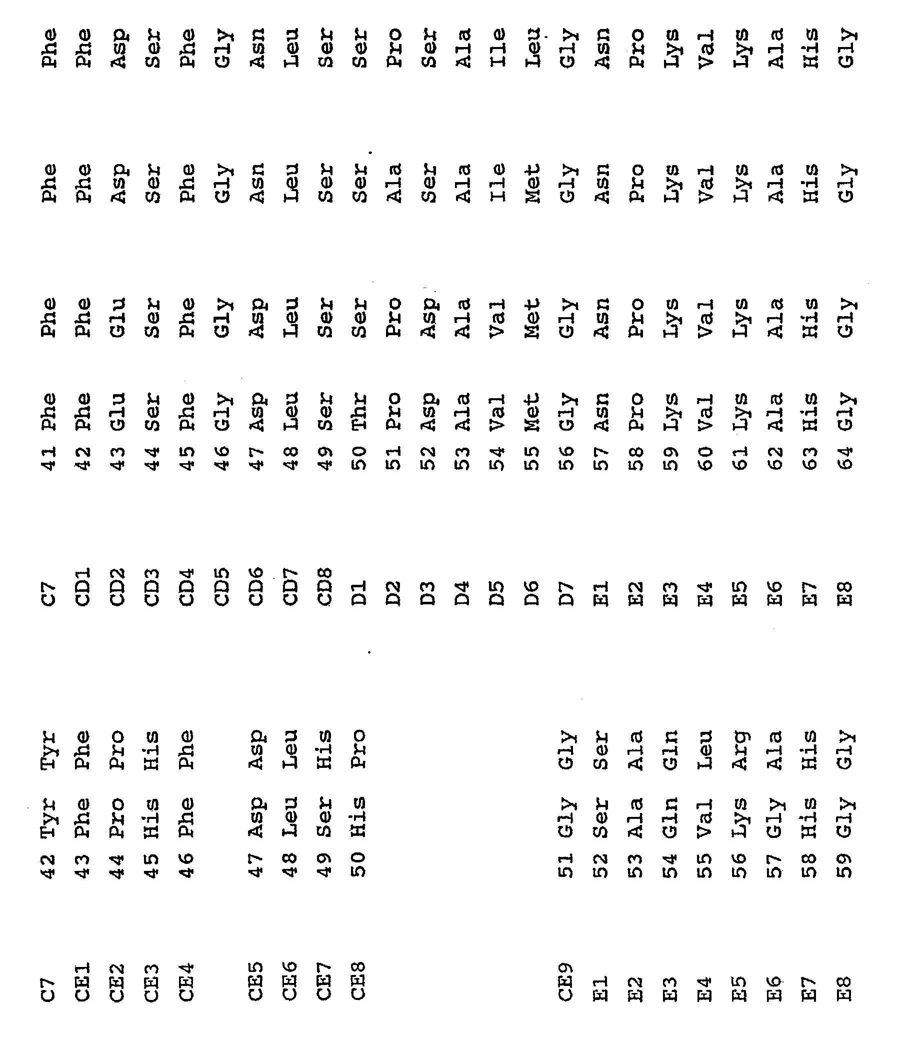

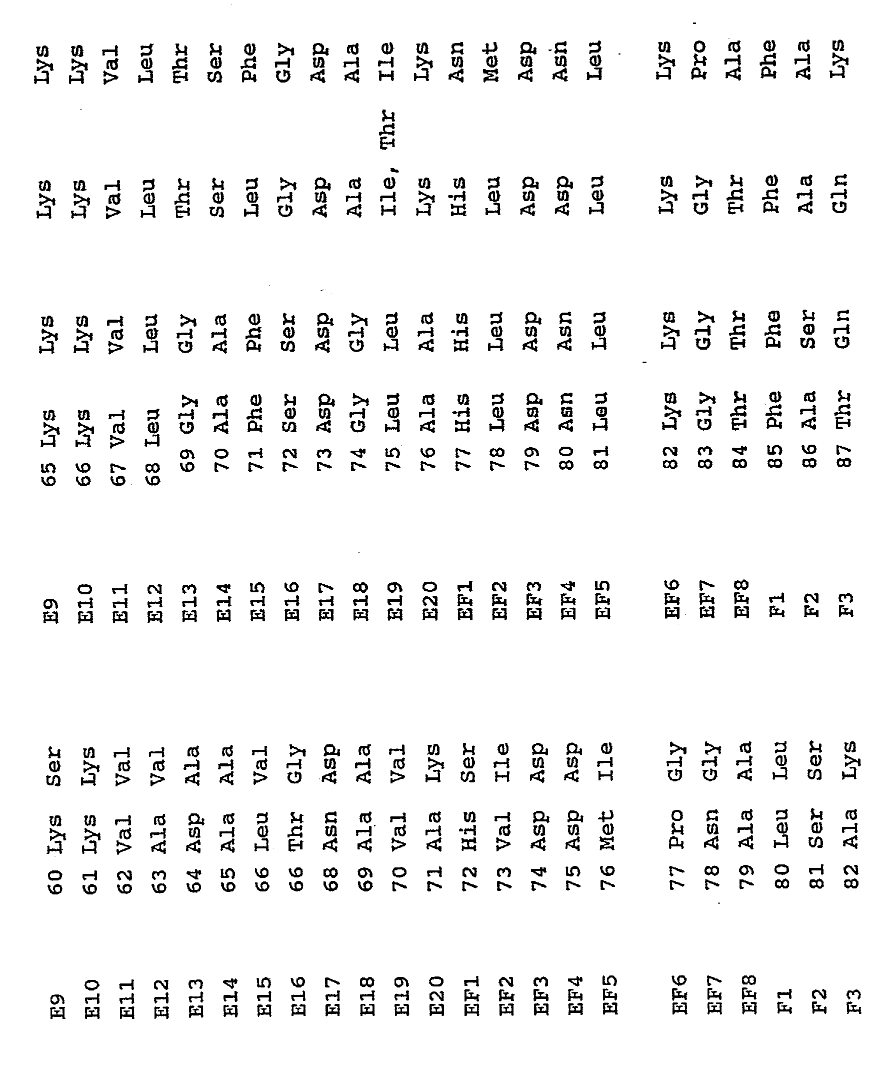

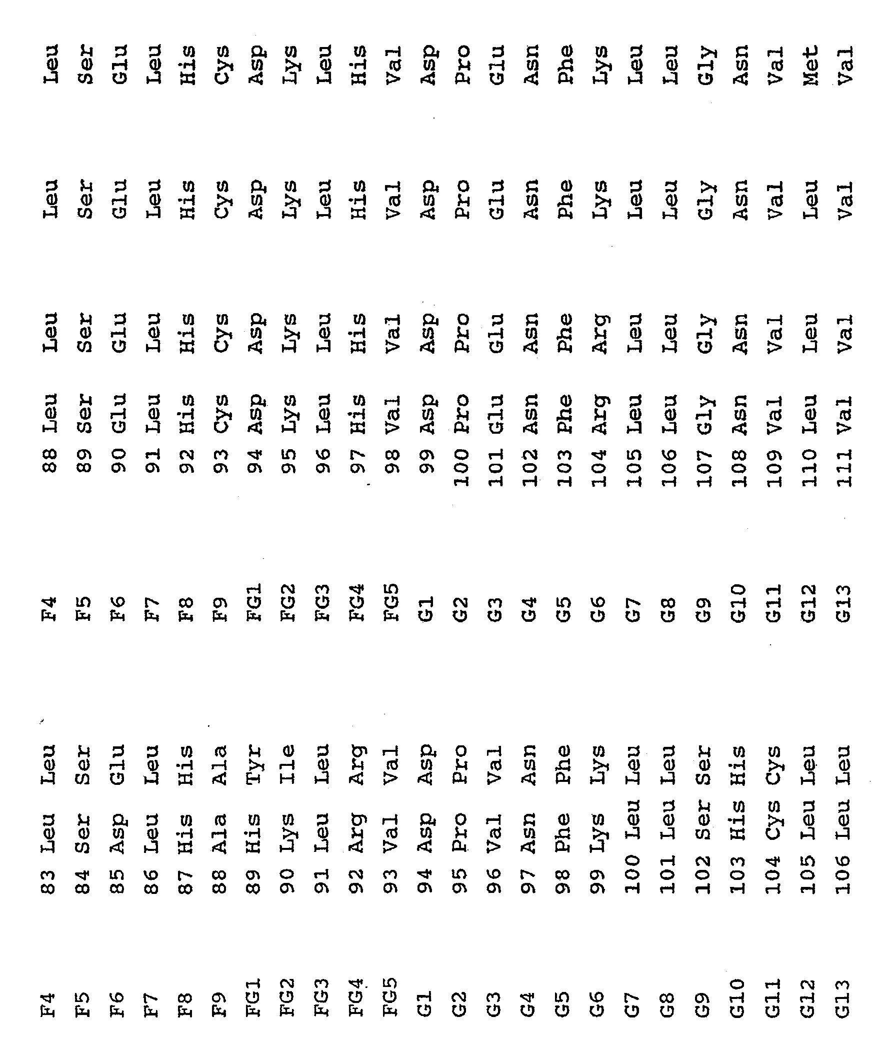

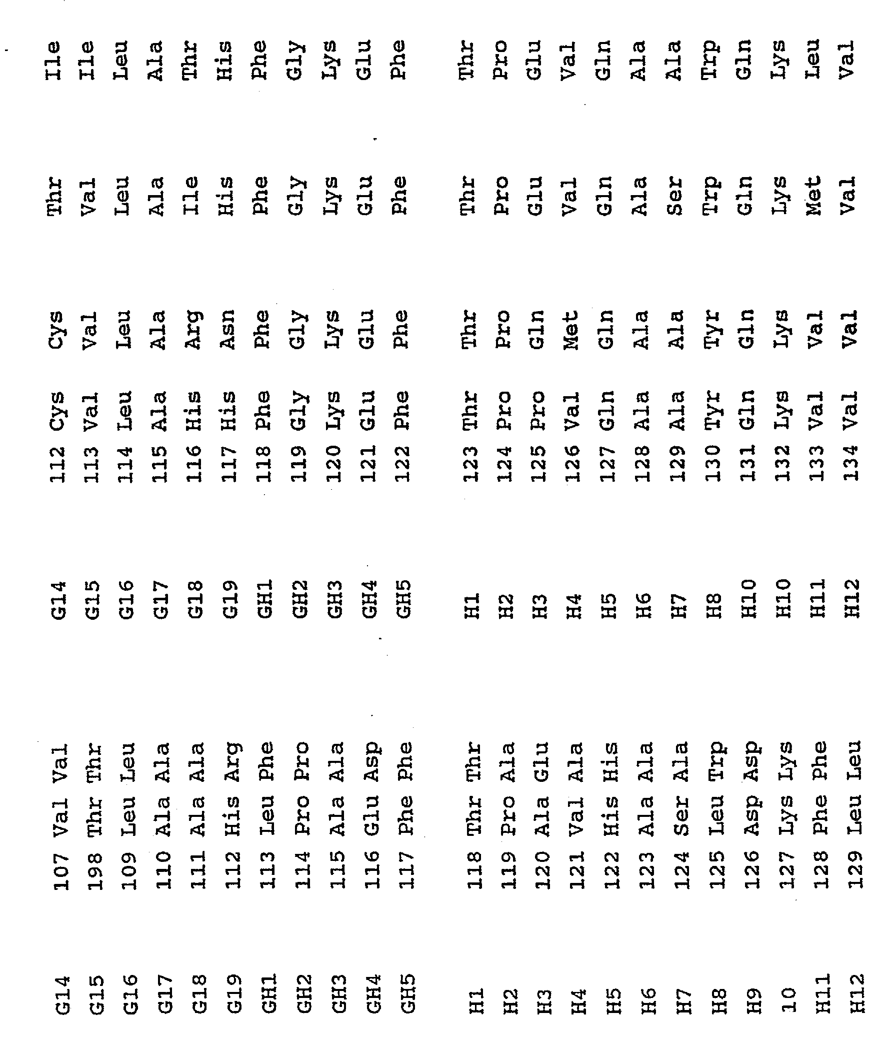

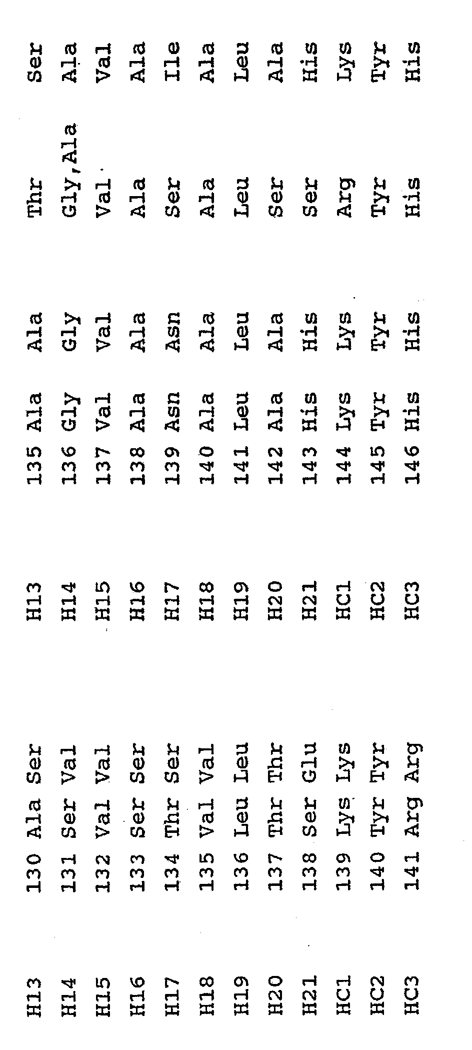

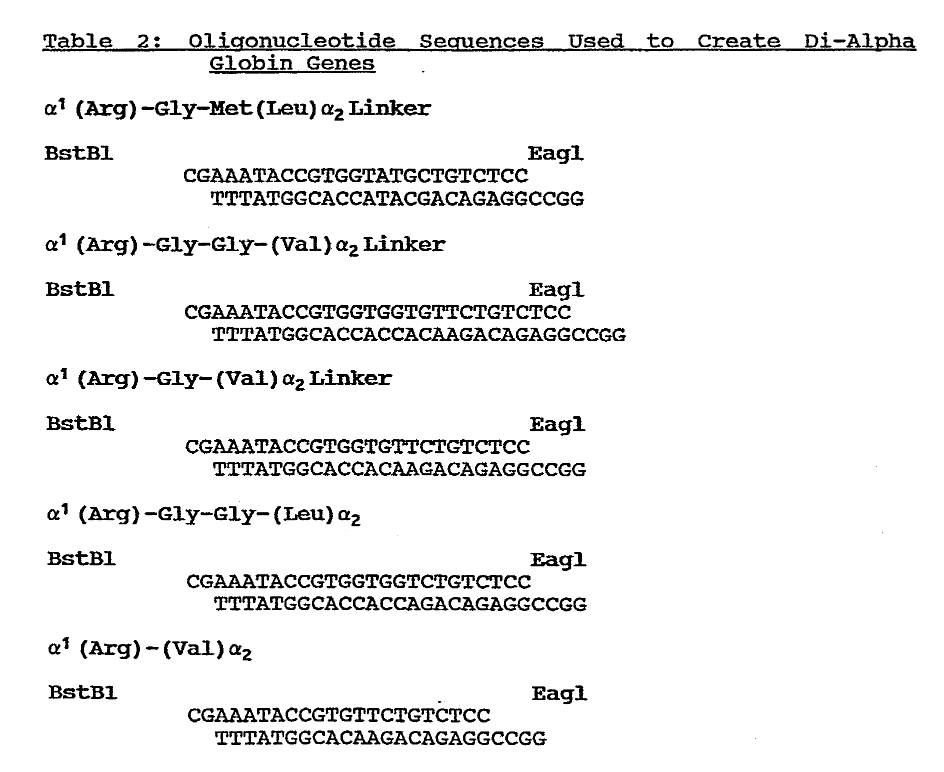

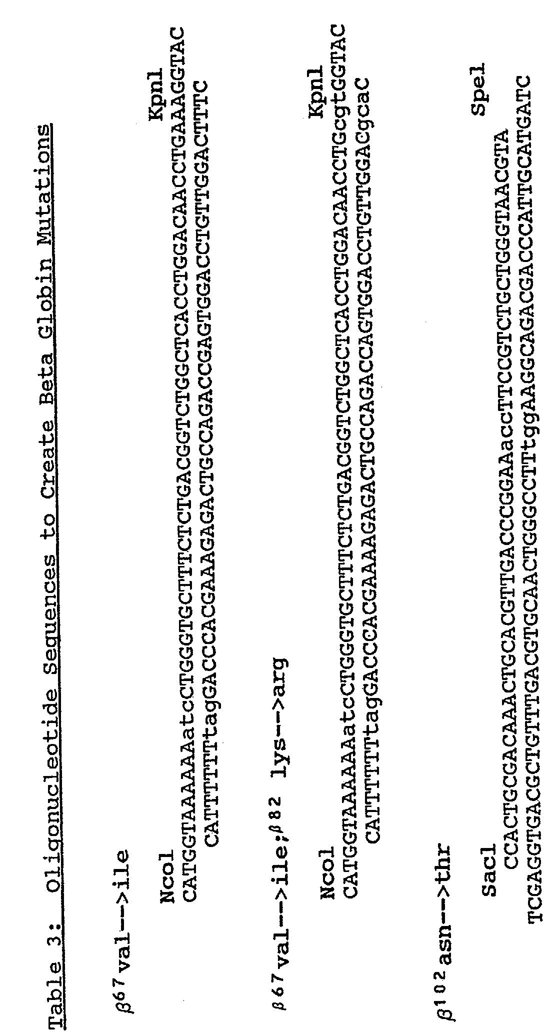

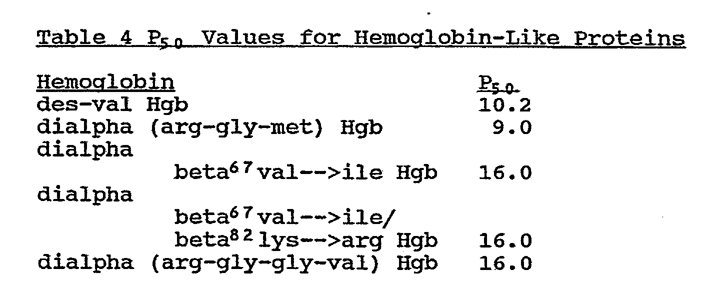

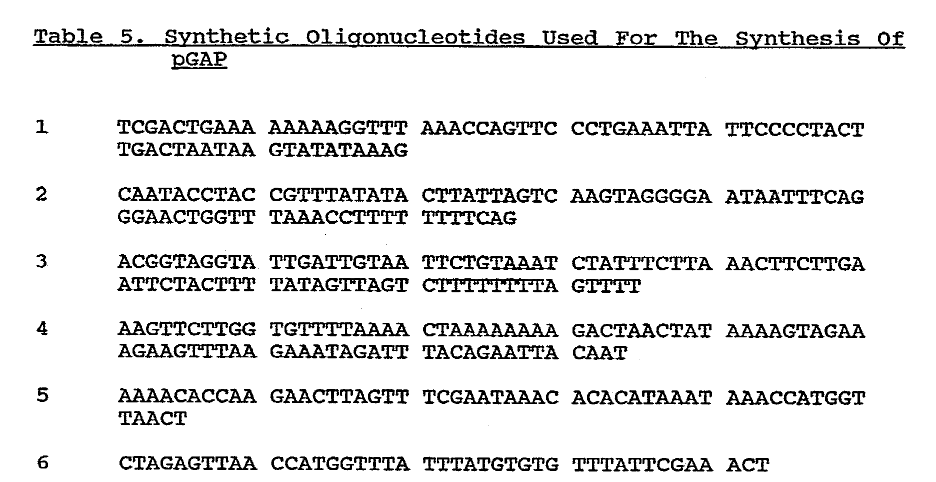

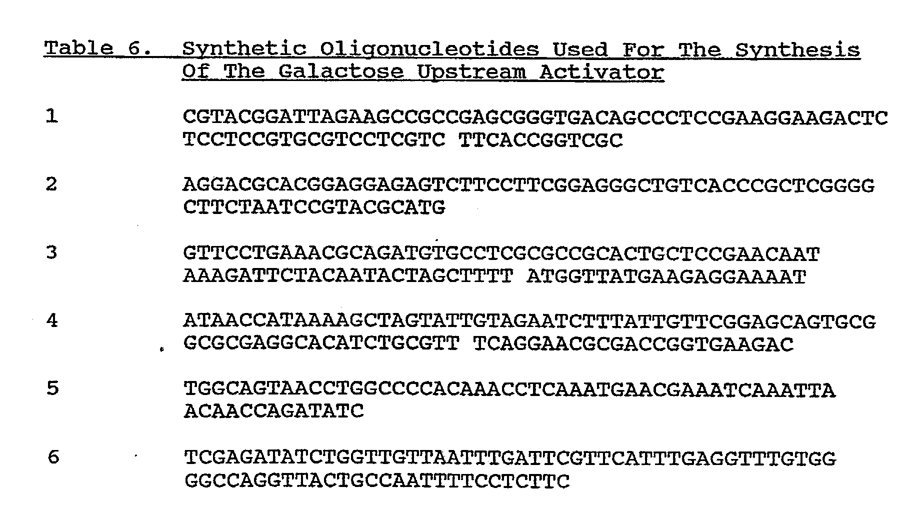

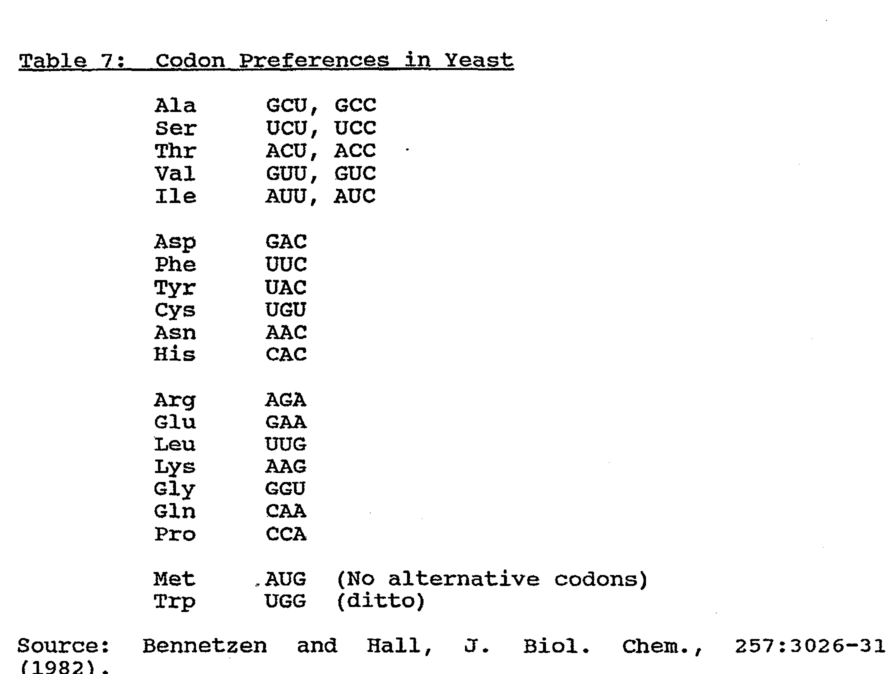

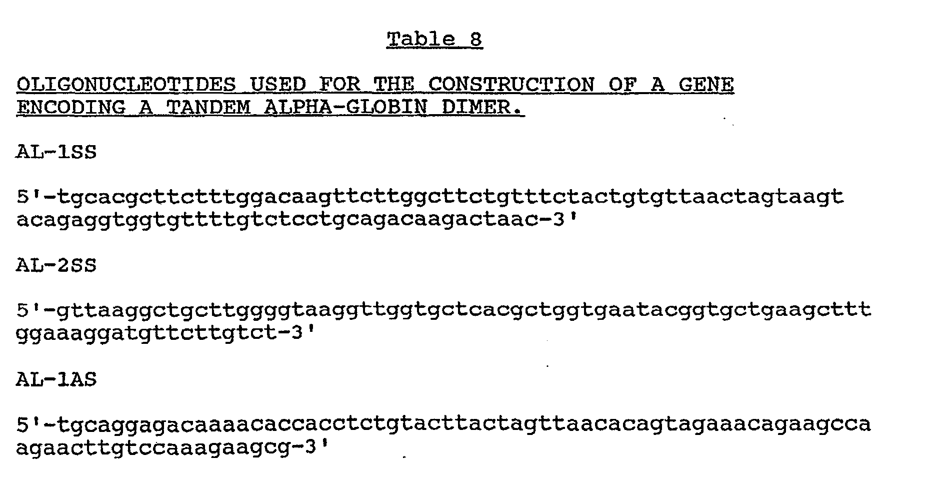

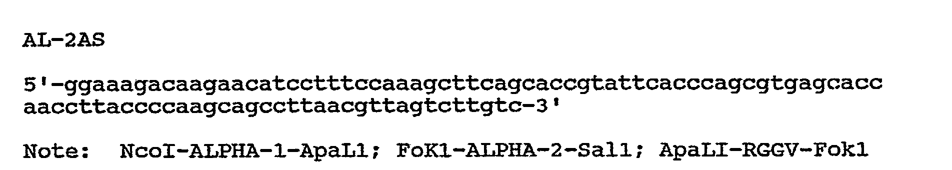

**Table 9:**

| **Oxygen Affinity of Recombinant Mutant Hemoglobin Generated From FX-Hqb and Tryptic Digestion** | |
|---|---|
| Oxygen affinity measured at 37°C in 50 mM Bis-Tris, pH 7.4, 0.1 M NaCl on a Hemox-Analyzer. Solutions were 60 uM in heme and measured between 130 and 1.2 torr oxygen tension. | |

| | P₅₀ |
|---|---|
| HgB Aₒ | 9.5 |
| rHgB Aₒ | 9.2 |
| HgB Providence | 10.2 |
| Hgb Kansas | 11.3 |
| HgB (beta 67 val-->ile) | 22.4 |

The value for Hgb Aₒ is of course for free hemoglobin in solution. The P₅₀ of whole blood is much higher.

**Table 10:**

| Effects of NaCl and Inositol Hexaphosphate on Oxygen Binding to Hemoglobin Aₒ and Recombinant des-Fx Hgb | | | | |
|---|---|---|---|---|
| | P₅₀ | | P₅₀ | |
| | 0.1 M NaCl | 0 M NaCl | 2.2 mM IHP | 0 M 1HP |
| Hgb Aₒ | 6.6 | 2.8 | 51.1 | 6.6 |
| des-Fx Hgb | 4.9 | 3.9 | 5.5 | 4.9 |

**Table 11:**

| Distribution of FX-Alpha, FX-Beta and FX-Hgb in E. coli. | | |
|---|---|---|
| | milligrams of protein per OD-liter of E. coli | |
| | Soluble | Insoluble |
| FX-Alpha | 36 | 0 |
| FX-Beta | 21 | 21 |
| FX-Hgb | 188 | 0 |

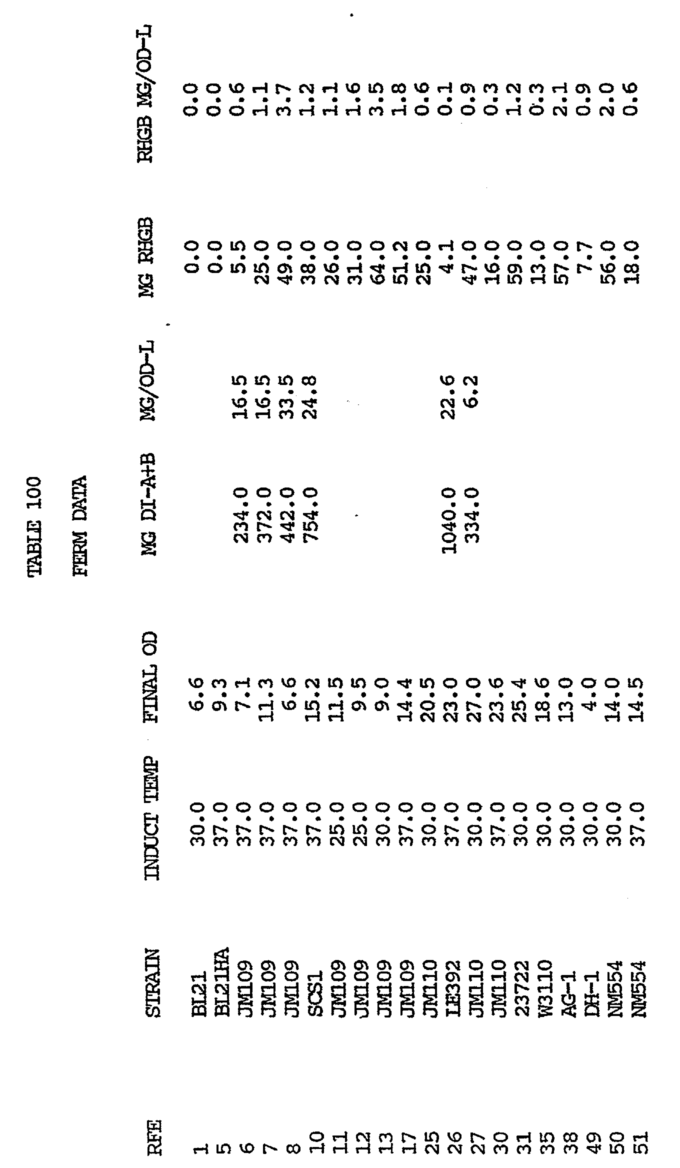

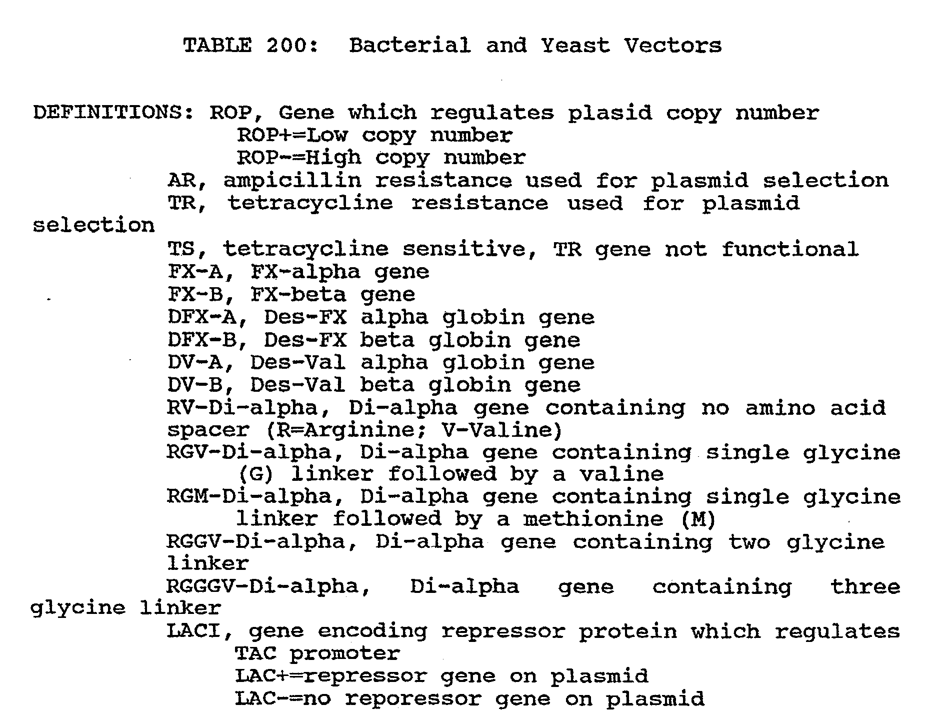

All bacterial plasmids listed below which contain alpha, di-alpha and/or beta genes also have translational couplers. The pPL expression system translationally couples the lambda N protein gene to a globin gene.

### pKK223-3

Parental plasmid obtained commercially from Pharmacia LKB, 800 Centennial Ave., P.O. Box 1327 Piscataway, N.J. 08855-1327; all remaining plasmids derived from this parental plasmid. Has TAC promoter followed by a poly-restriction site region to facilitate gene insertion.
AR, TS, ROP+, LAC-
1. pDL II-62m
   pKK223-3 containing FX-A
   AR, TS, ROP+, LAC-
2. pDL II-10a
   pKK223-2 containing FX-B
   AR, TS, ROP+, LAC-
3. PDL II-66A
   Parental plasmids are 1 and 2; contains both FX-A and FX-B in single operon
   AR, TS, ROP+, LAC-
4. pGEM FX-A
   Parental plasmids are 1 and pGem1 which is commerically available from Promega Corporation,
   2800 Woods Hollow Rd., Madison, WI 53711.
   pGem1 containing FX-A
   AR
5. pGEM FX-B
   Parentals are 2 and pGem1
   pGem1 containing FX-B
   AR
6. pDL II-83a
   Parental is 4; contains DFX-A
7. pDL III-6f
   Parental is 5; contains DFX-B
   AR
8. pDL II-86c
   Parentals are pKK223-3 and 6
   contains DFX-A
   AR, TS, ROP+, LAC-
9. pDL III-13e
   Parentals are 7 and 8
   pKK223-3 containing both DFX-A and DFX-B
   AR, TS, ROP+, LAC-
10. pDL II-91f
   Parental is 4 contains DV-A
   AR
11. pDL II-95a
   Parental is 7 contains DV-B
   AR
12. pDL III-la
   Parentals are pKK223-3 and 10
   contains DV-A
   AR, TS, ROP+, LAC-
13. pDL III-14c
   Parentals are 11 and 12
   contains DV-A and DV-B
   AR, TS, ROP+, LAC-
14. pDL III-47a
   Parental is 13
   contains RGM-DIALPHA and DV-B
   AR, TS, ROP+, LAC-
15. pDL III-82A
   Parental is 13
   contains RGGV-DIALPHA and DV-B
   AR, TS, ROP+, LAC-
16. pDL IV-8a
   Parental is 13
   contains RGV-DIALPHA and DV-B
   AR, TS, ROP+, LAC-
17. pDL IV-47b
   Parental is 13
   contains RV-DIALPHA and DV-B
   AR, TS, ROP+, LAC-
18. pDL IV-66a
   Parental is 13
   contains RGGGV-DIALPHA and DV-B
   AR, TS, ROP+, LAC-
19. pDL IV-3a
   Parental is 15
   ROP gene is inactivated by insertion of a Not I linker into the Pvu II site within the ROP gene
   AR, TS, ROP-, LAC-
20. pDL IV-38a
   Parental is 15
   contains the Nagai mutation in DV-B
   AR, TS, ROP+, LAC-
21. pDL IV-58f
   Parental is 20
   ROP gene inactivated as in 19
   AR, TS, ROP-, LAC-
22. pDL IV-59a
   Parental is 21 and pBR322 which is commerically available from a number of different suppliers.
   contains a functional TR gene constructed in the following manner:
   The Eco R1 site of pBR322 was changed to a Bam H1 site by insertion of a Bam H1 linker. This permitted removal of the 5' end of the TR gene from pBR322 as a Bam H1 fragment. This fragment was then inserted into the Bam H1 site located at the junction of the TAC promoter and the inactive TR gene of pKK223-3. Insertion of this fragment reactivates the TR gene if the fragment inserts in the proper orientation. Selection of colonies on tetracycline plates assures presence of the fragment in the proper orientation.
   AR, TR, ROP-, LAC-
23. pJR V-83a
   Parental is 11
   contains DV-B with the Presbyterian mutation (asn108 to lys).
   AR, TS, ROP+, LAC-
24. pJR VI-29a
   Parentals are 15 and 23
   contains RGGV-DIALPHA and DV-B with Presbyterian mutation
   AR, TS, ROP-, LAC-
25. pJR VI-53b
   Parental is 24
   made TR by insertion of BAM H1 fragment
   AR, TR, ROP+, LAC-
26. pJR VI-61a
   Parental is 25
   made ROP- by insertion of Not I linker into Pvu II site
   AR, TR, ROP-, LAC-
27. pDL V-4a
   Parentals are 16 and 26
   contains RGV-DIALPHA and DV-B with Presbyterian mutation
   AR, TS, ROP-, LAC-
28. pDL V-10a
   Parental is 27
   insertion of Bam H1 fragment to convert to TR
   AR, TR, ROP-, LAC-
29. pDL V-16d
   Parental is 28
   contains LACI gene inserted into Not I site
   LACI gene obtained using following protocol:
   Polymerase chain reaction (PCR) primers containing NOT I sites at their 5' ends were used to amplify the lac 1 gene
   Following gel purification the gene was inserted into the NOT I site of pDLV-1a AR, TR, ROP-, LAC+

Several other plasmids constructs have been designed to facilitate the incorporation of a second beta globin gene under regulation of its own TAC promoter.
30. pDL IV-64a
   Parental is 14
   contains beta globin under regulation of a synthetic TAC promoter
   AR, TS, ROP+, LAC-
31. pDL IV-67a
   Parental plasmids are 14 and 30
   contains DIALPHA under regulation of one pTAC and DV-B under regulation of a second pTAC
   DV-B is adjacent to DIALPHA
   AR, TS, ROP-, LAC-
32. pJR VI-54a
   Parental plasmids are 14 and 30
   contains DIALPHA and DV-B under regulation of one pTAC and a second DV-B under regulation of another pTAC
   The second DV-B is inserted into the Pvu II site of the plasmid
   AR, TS, ROP-, LAC-
33. pPL Lambda,
   Commercially available plasmid from Pharmacia LKB (see above) contains pL promoter and coding region for N protein of lambda which can be used for expression of fusion or translationally coupled recombinant proteins.
34. pPL-alpha/beta
   Parental plasmids are 13 and 33
   contains DV-A and DV-B
   AR, ROP+
35. pPL-dialpha/beta
   Parental plasmid is 34
   contains RGV-DIALPHA and DV-B
   AR, ROP+
36. pSGE0.1-L0
   Parental plasmid is 35
   ROP gene inactivated by insertion of Not I linker into Pvu II site in ROPgene
   AR, ROP-
37. pSK+
   Commercially available from Stratagene, LaJolla, Ca.
38. pGS2488
   Derived from (37) by insertion of synthetic GAP 491 transcriptional initiation site.
39. pGS2888
   Derived from (38) by conversion of KpnI site to SphI site.
40. pGS4788
   Derived from (39) by insertion of synthetic GAL_{UAS} into (39) to form GALGAP hybrid promoter.
41. pLC IIFX-β-globin
   Plasmid available from Kiyoshi Nagai, Medical Research Council, London, England; bears β globin gene
42. pUC19
   Plasmid commercially available from Bethesda Research Laboratories, Gaithersburg, Maryland
43. pSUC2-6Σ
   Plasmid described by Stetler, et al., Biotechnology, 7:55-60 (1989)
44. pUC19β-globin
   Derived from (41) and (42)
45. Plasmid pGS1188
   β-globin gene (from 44) is under control of the sigma promoter and the MFX terminator (both from 43).
46. Plasmid pGS3588
   Derived from (45) and (40); sigma promoter replaced by GALGAP promoter of (40).
47. Plasmid pGS3888
   Derived from (46) by conversion of SmaI site to XhoI site.
47a. Plasmid pα-MRC
   Plasmid available from K. Nagai, MRC; bears alpha-globin gene.
48. Plasmid pGS4088
   Derived from (47) by insertion of α-globin gene, replacing the β-globin gene.
49. Plasmid pSN(+)
   Derived from (37) by changing KpmI site to NotI site.
50. Plasmid pGS4888
   Derived from (49) by insertion of pGGAP-α-globin expression cassette from (48).
51. Plasmid pGS189
   Derived from (50) by insertion of pGGAP-β-globin expression cassette from (47). Plasmid thus bears both GALGAP α globin and GALGAP β globin operons.
52. Plasmid pC1U
   Plasmid described in Stetler, et al.; Biotechnology, 7:55-60 (1989)
53. Plasmid pCLN
   Derived from (52) by addition of NotI site.
54. Plasmid pGS289
   Derived from (53) by insertion of α-globin and β-globin expression cassettes.
55. Plasmid pGS389
   Ditto, but with insert orientation reversed.
56. Plasmid pYRp7
   Plasmid described in Strathern, et al., The Molecular Biology of the Yeast Saccharomyces (Cold Spring Harbon 1981)
   Source of TRP1 gene.
57. pC1T
   Derived from (52) and (56).
   Replacing Ura3 gene (52) with Trp1 gene (56).
58. pGS4988
   Derived from (57) by insertion of β-globin expression cassette from (47).
59. Plasmid pGS4488
   Derived from (52) by insertion of α-globin expression cassette of (48).
60. Plasmid pGS4688
   Ditto, but with insert orientation reversed.
61. Plasmid pGS4888
   Derived from (49) by insertion of GGAP-alpha globin expression cassette from (48).
62. Plasmid pGS1889
   Derived from (61) by removal of PstI site.
63. Plasmid pGS1989
   Derived from (62) by removal of SpeI site.
64. Plasmid pGS2189
   Derived from (61) and (63). Encodes di-alpha globin with RGGV linker.
65. Plasmid pGS2989
   Derived from (64) and (47), contains di-alpha globin gene and beta globin gene.
66. Plasmid pGS3089
   Derived from (53) by insertion of expression cassette of (65).
66a. Phagescript
   Phage commercially available from Stratagene.
67. Phage Mpβ-globin
   Derived from Phagescript by insertion of GALGAP promoter and β-globin gene from (65).
67a. Plasmid pGS3089 RGV desβ
   Derived from (66) by deletion of XhoI fragment containing β globin expression cassette.
68. Plasmid pGS3889
   (66) with "Presbyterian" (βN108K) mutation.
69. Plasid pGS5189
   (66) with "Agenogi" (βE90K) mutation.
70. Plasmid pGS5689
   (66) with "Kansas" (βN102T) mutation.
71. Plasmid pGS4989
   (66) with "βV67I" mutation.
72. Plasmid pGS2989 RPV
   (64) with RPV di-alpha linker
73. Plasmid pGS2989 RGV
   (64) with RGV di-alpha linker
74. Plasmid pGS3089 RGV
   (53) with Hgb expression cassette from (73)
75. Plasmid pGS3089 RPV
   (53) with Hgb expression cassette from (72)

**TABLE 300**

| TABLE OF BACTERIAL STRAINS | | | |
|---|---|---|---|
| | Strain | Availability | Expression |
| 1 | BL21 | Brookhaven | TAC |
| 2 | JM109 | Commercial | TAC |
| 3 | SCS1 | Commercial | TAC |
| 4 | JM110 | ATCC | TAC |
| 5 | LE392 | ATCC | TAC |
| 6 | 23722 | ATCC | TAC |
| 7 | W3110 | ATCC | TAC |
| 8 | AG-1 | Commercial | TAC |
| 9 | DH1 | ATCC | TAC |
| 10 | NM554 | Commercial | TAC |
| 11 | N99cl+ | Commercial | PL |
| 12 | N4830-1 | Commercial | PL |

## Claims

1. A DNA molecule encoding a human hemoglobin-like protein comprising a first gene encoding a di-alpha globin-like polypeptide and a second gene encoding a beta globin-like polypeptide, wherein the alpha globin-like domains of the di-alpha polypeptide have at least 75% sequence identity with human alpha globin and the beta globin-like polypeptide has at least 75% sequence identity with human beta globin.

2. The DNA molecule of claim 1, wherein the alpha globin-like domains are essentially identical in sequence to human alpha globin and the beta globin-like polypeptide is essentially identical in sequence to human beta globin.

3. The DNA molecule of claim 2, wherein the beta globin-like polypeptide comprises a Presbyterian mutation.

4. A DNA encoding a di-beta globin-like polypeptide, wherein said polypeptide renders a hemoglobin-like protein a low affinity hemoglobin mutant.

5. A method for the production of a hemoglobin-like protein wherein an alpha globin-like polypeptide and a beta globin-like polypeptide are each expressed in transformed, non-erythrocyte cells, the method further comprising expressing the alpha globin and beta globin-like polypeptides in the same cell in such manner that the alpha and beta globin-like polypeptides are assembled and combined with heme so as to intracellularly produce a biologically functional hemoglobin-like protein and isolating said protein.

6. A hemoglobin-like protein having reversible oxygen-binding activity, said protein comprising at least one polypeptide which is a non-naturally occurring, genetically fused globin-like polypeptide comprising two globin-like domains connected by one or more peptide bonds into a single polypeptide chain, said chain being capable of associating with alpha and/or beta globin and incorporating heme to form a hemoglobin-like protein with reversible oxygen binding activity.

7. A non-naturally occurring, genetically fused globin-like polypeptide comprising two globin-like domains connected by one or more peptide bonds into a single polypeptide chain, said chain being capable of associating with alpha and/or beta globin and incorporating heme to form a hemoglobin-like protein with reversible oxygen activity.

8. The polypeptide of claim 38 wherein the linker is -Gly- or -Gly-Gly-.

9. A human hemoglobin-like protein having reversible oxygen-binding activity, said protein selected from the group consisting of multimeric proteins composed of:
(a) a di-alpha globin-like polypeptide and two beta globin-like polypeptides,
(b) a di-beta globin-like polypeptide and two alpha globin-like polypeptides, and
(c) a di-alpha globin-like polypeptide and a di-beta globin-like polypeptide, said protein bearing prosthetic heme groups.

10. A recombinant DNA molecule comprising expressible first and second DNA sequences encoding first and second alpha globin-like polypeptide sequences and optionally a linker DNA sequence encoding a linker amino acid sequence, said first and second polypeptide sequences and if included said linker amino acid sequence being expressed as a single polypeptide chain, said chain being capable of associating with beta globin and incorporating heme to form a human hemoglobin-like protein with reversible oxygen-binding activity.

11. A recombinant DNA molecule comprising expressible first and second DNA sequences encoding first and second beta-globin-like polypeptide sequences, and a linker DNA sequence encoding a linker amino acid sequence, said first and second polypeptide sequences and said linker amino acid sequences being expressed as a single polypeptide chain, said chain being capable of associating with alpha globin and incorporating heme to form a human hemoglobin-like protein with reversible oxygen-binding activity.

12. The recombinant DNA molecule of claim 10, further comprising a third DNA sequence encoding a beta globin-like polypeptide.

13. The molecule of claim 12, wherein said first DNA sequence, linker DNA sequence, and second DNA sequence define a single cistron of a polycistronic operon and said third DNA sequence defines another cistron thereof, said cistrons being transcribed from a common promoter.

14. The molecule of claim 13 wherein the common promoter is the Tac promoter.

15. A method of producing a human hemoglobin-like protein with reversible oxygen binding activity wherein the two alpha subunits of native hemoglobin are replaced by a single di-alpha globin-like polypeptide, which comprises providing a host transformed with a recombinant DNA molecule according to claim 10, cultivating said host under conditions whereunder it expresses said di-alpha globin-like polypeptide, and combining said polypeptide with beta globin and heme to obtain a human hemoglobin like protein.

16. The method of claim 15 wherein the di-alpha globin and the beta-globin are co-expressed from said recombinant DNA molecule and heme is provided by said host and incorporated to form said hemoglobin-like protein.

17. A method of producing a human hemoglobin-like protein with reversible oxygen binding activity wherein the two beta subunits of native hemoglobin are replaced by a single di-beta globin-like polypeptide, which comprises providing a host transformed with a recombinant DNA molecule according to claim 11, cultivating said host under conditions whereunder it expresses said di-beta globin-like polypeptide, and combining said polypeptide with alpha globin and heme to obtain a human hemoglobin like protein.

18. A non-chemically crosslinked hemoglobin-like protein composed of fewer than four polypeptide chains and having a half-life in the circulatory system substantially in excess of that for des-Val hemoglobin.

19. The hemoglobin protein of claim 18 which has a half-life of at least 3 hours in the circulatory system of a rat.

20. A method of determining a functional linker for a di-alpha globin-like or di-beta globin-like polypeptide, suitable for use in the production of a hemoglobin-like protein, which comprises (a) providing a family of recombinant DNA vectors, each vector encoding a di-alpha globin-like polypeptide or a di-beta globin-like polypeptide **characterized by** a polypeptide linker of one of more amino acids, said family collectively encoding a plurality of different polypeptides differing in the linker amino acid sequence, (b) transforming cells with said family of vectors, (c) producing a di-alpha or di-beta hemoglobin-like protein in said cells, (d) screening said cells for the production of a hemoglobin-like protein by determining which cells react with carbon monoxide in a manner indicating the presence of a hemoglobin-like protein, and (e) determining the amino acid sequence of the linker of the di-alpha hemoglobin or di-beta hemoglobin produced by the cells which screened positively in step (d) above.

21. In a method for the production of a hemoglobin-like protein wherein an alpha globin-like polypeptide and a beta globin-like polypeptide are each expressed in transformed, non-erythrocyte cells, the improvement comprising expressing the alpha globin and beta globin-like polypeptides in the same cell in such manner that the alpha and beta globin-like polypeptides are assembled and combined with heme so as to intracellularly produce a biologically functional hemoglobin-like protein.

22. The method of claim 21 which comprises (a) providing a yeast cell bearing (i) a gene encoding a human alpha globin-like polypeptide, said gene being linked to a promoter functional in said cell, and (ii) a gene encoding a human beta globin-like polypeptide, said gene being operably linked to a promoter functional in said cell, and (b) coexpressing said alpha globin and beta globin-like genes to obtain alpha globin and beta globin-like polypeptides, said cell folding together such polypeptides and incorporating heme to produce a human hemoglobin-like protein in soluble, recoverable form.

23. The method of claim 22 wherein at least one of said genes is expressed under the control of a GALGAP hybrid promoter.

24. The method of claim 22 wherein both genes are carried by the same plasmid.

25. The method of claim 22 wherein each gene is carried by a different plasmid.

26. The method of claim 22 wherein the yeast cell is of the species Saccharomyces cerevisiae.

27. The method of claim 22 wherein the yeast cell is of a protease-deficient strain.

28. The method of claim 22 wherein the hemoglobin-like protein is a non-naturally occurring, mutant hemoglobin.

29. The method of claim 15 wherein the host is a yeast cell.

30. The method of claim 15 wherein the host is a bacterial cell.

31. The method of claim 21 which comprises (a) providing a transformed nonhuman cell bearing a polycistronic genetic unit comprising a first cistron coding for an alpha globin-like subunit protein and a second cistron coding for a beta-globin-like subunit protein which may precede or follow said first cistron, and a single promoter controlling transcription of said polycistronic genetic unit, said cell being capable of synthesizing heme and incorporating the heme into said subunit proteins so as to form a human hemoglobin-like protein, and (b) cultivating the cell under conditions suitable for expression of said human hemoglobin-like protein.

32. The method of claim 31 in which the cell is a bacterial cell.

33. The method of claim 32 in which the bacterial cell is Escherichia coli.

34. The method of claim 33 in which the promoter is the Tac promoter.

35. The method of claim 31 wherein said first and second cistrons are preceded, respectively by first and second introductory cistrons, wherein the second cistron may precede or follow the first cistron, and the first and secondary introductory cistrons each contain a Shine- Delgarno sequence operably positioned to facilitate translation of the following cistron.

36. The method of claim 32 wherein a substantial portion of the functional human hemoglobin-like protein is found in the soluble fraction of the cell.

37. The method of claim 35 wherein the human hemoglobin like protein has a lower oxygen affinity than native human hemoglobin.

38. A di-alpha globin-like polypeptide consisting essentially of first and second alpha globin-like polypeptide sequences connected by one or more peptide bonds into a single polypeptide chain, said chain being capable of associating with beta globin and incorporating heme to form a human hemoglobin-like protein with reversible oxygen-binding activity.

39. A di-beta globin-like polypeptide consisting essentially of first and second beta-globin-like polypeptide sequences connected by one or more peptide bonds into a single polypeptide chain, said chain being capable of associating with alpha-globin and incorporating heme to form a human hemoglobin-like protein with reversible oxygen-binding activity.

40. The polypeptide of claims 38 or 39 wherein the first and second alpha or beta globin-like polypeptide sequences are connected directly by a single peptide bond between the normal C-terminal of the first polypeptide and the normal N-terminal of the second polypeptide.

41. The polypeptide of claims 38 or 39 wherein the first and second alpha or beta globin-like polypeptides are connected by a peptide linker of one or more amino acids.

42. The polypeptide of claim 41 wherein the linker consists of 1 to 3 amino acids.

43. The polypeptide of claim 42 wherein the amino acids of the linker are disposed in a random coil.

44. The polypeptide of claim 43 wherein the amino acids of the linker are selected from the group consisting of lysine, aspartic acid, arginine, serine, asparagine, proline and glycine.
